# EUROPEAN PATENT SPECIFICATION

(11) **EP 2 858 489 B1**
(45) Date of publication and mention of the grant of the patent: **09.11.2016**
(21) Application number: 12750333.2
(22) Date of filing: 12.06.2012
(51) Int. Cl.: A01K 67/033, C12N 15/85, C12N 15/866

(54) **RECOMBINANT DNA ELEMENTS FOR THE EXPRESSION OF RECOMBINANT PROTEINS IN A HOST CELL**
REKOMBINANTE DNS-ELEMENTE ZUR EXPRESSION REKOMBINANTER PROTEINE IN EINER WIRTSZELLE
ÉLÉMENTS ADN RECOMBINANTS POUR L'EXPRESSION DE PROTÉINES RECOMBINANTES DANS UNE CELLULE HÔTE

(43) Date of publication of application: 15.04.2015
(73) Proprietor: Alternative Gene Expression, S.L., 28223 Pozuelo de Alarcón, Madrid (ES)
(72) Inventor: GOMEZ SEBASTIAN, Silvia, E-28040 Madrid (ES); LÓPEZ VIDAL, Javier, E-28040 Madrid (ES); MARTINEZ ESCRIBANO, José Angel, E-28040 Madrid (ES)
(74) Representative: Hoffmann Eitle
(86) International application number: PCT/EP2012/061081
(87) International publication number: WO 2012/168492

(56) References cited:
- WO-A1-2010/025764
- US-A- 5 965 393
- US-A1- 2009 068 703
- NAGAI S ET AL: "Comparative transient expression assay analysis of hycu-hr6- and IE1-dependent regulation of baculovirus gp64 early promoters in three insect cell lines", VIRUS RESEARCH, AMSTERDAM, NL, vol. 155, no. 1, 1 January 2011 (2011-01-01), pages 83-90, XP027574274, ISSN: 0168-1702 [retrieved on 2010-12-24]
- CROUCH E A ET AL: "Effects of baculovirus transactivators IE-1 and IE-2 on the Drosophila heat shock 70 promoter in two insect cell lines", ARCHIVES OF VIROLOGY ; OFFICIAL JOURNAL OF THE VIROLOGY DIVISIONOF THE INTERNATIONAL UNION OF MICROBIOLOGICAL SOCIETIES, SPRINGER-VERLAG, VI, vol. 150, no. 8, 1 August 2005 (2005-08-01), pages 1563-1578, XP019378571, ISSN: 1432-8798, DOI: 10.1007/S00705-005-0527-8
- NAGAMINE T ET AL: "Induction of a subnuclear structure by the simultaneous expression of baculovirus proteins, IE1, LEF3, and P143 in the presence of hr", VIROLOGY, ACADEMIC PRESS,ORLANDO, US, vol. 352, no. 2, 1 September 2006 (2006-09-01), pages 400-407, XP024896408, ISSN: 0042-6822, DOI: 10.1016/J.VIROL.2006.04.034 [retrieved on 2006-09-01]
- W. KANG: "IE1 and hr facilitate the localization of Bombyx mori nucleopolyhedrovirus ORF8 to specific nuclear sites", JOURNAL OF GENERAL VIROLOGY, vol. 86, no. 11, 1 November 2005 (2005-11-01), pages 3031-3038, XP055016117, ISSN: 0022-1317, DOI: 10.1099/vir.0.81270-0
- LIN X; CHEN Y; YI Y; ZHANG Z: "Baculovirus immediately early 1, a mediator for homologous regions enhancer function in trans", VIROL J, vol. 7, no. 32, 2010, XP002685888, cited in the application
- PASSARELLI ET AL: "Three baculovirus genes involved in late and very late gene expression: ie-1, ie-n, and lef-2.", JOURNAL OF VIROLOGY, vol. 67, no. 4, 1 April 1993 (1993-04-01), pages 2149-2158, XP055016120, ISSN: 0022-538X cited in the application
- NETTLESHIP J E ET AL: "Recent advances in the production of proteins in insect and mammalian cells for structural biology", JOURNAL OF STRUCTURAL BIOLOGY, ACADEMIC PRESS, UNITED STATES, vol. 172, no. 1, 1 October 2010 (2010-10-01), pages 55-65, XP027249557, ISSN: 1047-8477 [retrieved on 2010-02-11] cited in the application
- S. KANGINAKUDRU ET AL: "Targeting ie-1 gene by RNAi induces baculoviral resistance in lepidopteran cell lines and in transgenic silkworms", INSECT MOLECULAR BIOLOGY, vol. 16, no. 5, 1 October 2007 (2007-10-01), pages 635-644, XP055029022, ISSN: 0962-1075, DOI: 10.1111/j.1365-2583.2007.00753.x
- V. J. VALDES ET AL: "Using Double-stranded RNA to Prevent in Vitro and in Vivo Viral Infections by Recombinant Baculovirus", JOURNAL OF BIOLOGICAL CHEMISTRY, vol. 278, no. 21, 16 May 2003 (2003-05-16) , pages 19317-19324, XP055029633, ISSN: 0021-9258, DOI: 10.1074/jbc.M212039200
- OLSON V A ET AL: "The highly conserved basic domain I of baculovirus IE1 is required for hr enhancer DNA binding and hr-dependent transactivation", JOURNAL OF VIROLOGY, THE AMERICAN SOCIETY FOR MICROBIOLOGY, US, vol. 77, no. 10, 1 May 2003 (2003-05-01), pages 5668-5677, XP002998478, ISSN: 0022-538X cited in the application
- H.-R. LO ET AL: "Novel Baculovirus DNA Elements Strongly Stimulate Activities of Exogenous and Endogenous Promoters", JOURNAL OF BIOLOGICAL CHEMISTRY, vol. 277, no. 7, 8 February 2002 (2002-02-08), pages 5256-5264, XP055011180, ISSN: 0021-9258, DOI: 10.1074/jbc.M108895200
- CRISTIANO A FELIPE ALVES ET AL: "hycu-hr6, A large homologous region of the Hyphantria cunea nucleopolyhedrovirus genome, as a powerful and versatile enhancer in insect expression systems", VIRUS GENES, KLUWER ACADEMIC PUBLISHERS, BO, vol. 39, no. 3, 7 October 2009 (2009-10-07), pages 403-408, XP019754329, ISSN: 1572-994X, DOI: 10.1007/S11262-009-0406-6
- GOMEZ-CASADO E; GOMEZ-SEBASTIAN S; NÚÑEZ MC; LASA-COVARRUBIAS R; MARTÍNEZ-PULGARÍN S; ESCRIBANO JM: "Insect larvae biofactories as a platform for influenza vaccine production", PROTEIN EXPR PURIF., vol. 79, 2011, pages 35-43, XP002685889,

### SEQUENCE LISTING

<110> ALTERNATIVE GENE EXPRESSION S.L.
<120> RECOMBINANT DNA ELEMENTS FOR THE EXPRESSION OF RECOMBINANT PROTEINS IN A HOST CELL
<130> 156 164
<150> EP11169634.0
   <151> 2011-06-10
<150> PCT/EP2011/068381
   <151> 2011-10-20
<150> PCT/EP2011/072406
   <151> 2011-12-12
<160> 40
<170> PatentIn version 3.5
<210> 1
   <211> 1911
   <212> DNA
   <213> Autographa californica nucleopolyhedrovirus
<400> 1
   atgatccgta catccagcca cgtcctgaac gtccaagaaa acatcatgac ttccaactgt      60
   gcttccagcc cctactcctg tgaggccact tcagcctgcg ctgaggccca gcaactgcag     120
   gtggacacag gtggcgataa gatcgtgaac aaccaggtca ccatgactca aatcaacttc     180
   aacgcttcct acacctctgc cagcactccc tctcgtgcta gcttcgacaa ctcatactcg     240
   gagttctgcg acaagcaacc taacgattac ttgtcttact acaaccaccc aaccccggac     300
   ggagctgata ctgtcatctc cgactctgaa accgctgccg ctagcaactt cctcgcctca     360
   gttaactcgc tcactgacaa cgatttggtg gagtgtctgc tcaagaccac tgacaacctg     420
   gaggaagctg tgtcctctgc ctactacagc gagtcactcg aacagccagt ggtcgaacaa     480
   ccctctccta gctcagctta ccacgccgag tccttcgaac actctgctgg tgtcaaccag     540
   ccgtcggcca caggcaccaa gaggaagttg gacgagtacc tggataactc ccagggagtt     600
   gtgggtcaat tcaacaagat caagttgaga cctaagtaca agaagagcac catccagtca     660
   tgcgctacac tggaacaaac catcaaccac aacactaaca tctgtacagt ggcttccacc     720
   caggagatca ctcactactt cacaaacgac ttcgccccct acctgatgag gttcgacgat     780
   aacgactaca actcgaacag attctccgat cacatgtctg aaaccggtta ctacatgttc     840
   gtcgttaaga agtccgaggt gaagcctttc gaaatcatct tcgccaagta cgtctctaac     900
   gtggtctacg agtacacaaa caactactac atggttgaca accgtgtgtt cgttgtgacc     960
   ttcgataaga tccgcttcat gatcagctac aacctggtta aggagactgg catcgaaatc    1020
   ccacactcac aggacgtctg caacgatgag accgccgctc aaaactgcaa gaagtgtcac    1080
   ttcgtggacg tccaccacac attcaaggcc gctctgacct cctacttcaa cctcgatatg 1140
   tactacgctc agacaacctt cgtgaccttg ctgcaatcac tcggcgagcg taagtgtgga 1200
   ttcctcttgt cgaagttgta cgagatgtac caggacaaga acctcttcac tttgcccatc 1260
   atgctgagcc gcaaggaatc aaacgagatc gaaaccgcct ctaacaactt cttcgtctcg 1320
   ccatacgttt cccagatcct caagtactcg gagtccgtcc aattcccgga caaccctccc 1380
   aacaagtacg tcgttgataa cctgaacctc atcgtgaaca agaagagcac tctgacatac 1440
   aagtactcgt ccgtcgctaa cctgctcttc aacaactaca agtaccacga caacatcgct 1500
   tctaacaaca acgccgagaa cctcaagaag gtcaagaagg aagacggaag catgcacatc 1560
   gttgagcagt acttgactca aaacgtcgat aacgttaagg gtcacaactt catcgtgttg 1620
   tccttcaaga acgaggaaag gctgaccatc gctaagaaga acaaggagtt ctactggatc 1680
   tctggcgaaa tcaaggacgt tgatgtgagc caggtcatcc aaaagtacaa cagattcaag 1740
   caccacatgt tcgtgatcgg caaggtcaac cgtcgcgagt caactacact gcacaacaac 1800
   ttgctgaagc tcttggcctt gatcctgcag ggactggtgc cactctccga cgccatcaca 1860
   ttcgccgagc aaaagctcaa ctgcaagtac aagaagttcg agttcaacta a 1911
<210> 2
   <211> 1749
   <212> DNA
   <213> Autographa californica nucleopolyhedrovirus
<400> 2
   atgactcaaa tcaacttcaa cgcttcctac acctctgcca gcactccctc tcgtgctagc     60
   ttcgacaact catactcgga gttctgcgac aagcaaccta acgattactt gtcttactac    120
   aaccacccaa ccccggacgg agctgatact gtcatctccg actctgaaac cgctgccgct    180
   agcaacttcc tcgcctcagt taactcgctc actgacaacg atttggtgga gtgtctgctc    240
   aagaccactg acaacctgga ggaagctgtg tcctctgcct actacagcga gtcactcgaa    300
   cagccagtgg tcgaacaacc ctctcctagc tcagcttacc acgccgagtc cttcgaacac    360
   tctgctggtg tcaaccagcc gtcggccaca ggcaccaaga ggaagttgga cgagtacctg    420
   gataactccc agggagttgt gggtcaattc aacaagatca agttgagacc taagtacaag    480
   aagagcacca tccagtcatg cgctacactg gaacaaacca tcaaccacaa cactaacatc    540
   tgtacagtgg cttccaccca ggagatcact cactacttca caaacgactt cgccccctac    600
   ctgatgaggt tcgacgataa cgactacaac tcgaacagat tctccgatca catgtctgaa    660
   accggttact acatgttcgt cgttaagaag tccgaggtga agcctttcga aatcatcttc    720
   gccaagtacg tctctaacgt ggtctacgag tacacaaaca actactacat ggttgacaac    780
   cgtgtgttcg ttgtgacctt cgataagatc cgcttcatga tcagctacaa cctggttaag    840
   gagactggca tcgaaatccc acactcacag gacgtctgca acgatgagac cgccgctcaa    900
   aactgcaaga agtgtcactt cgtggacgtc caccacacat tcaaggccgc tctgacctcc    960
   tacttcaacc tcgatatgta ctacgctcag acaaccttcg tgaccttgct gcaatcactc   1020
   ggcgagcgta agtgtggatt cctcttgtcg aagttgtacg agatgtacca ggacaagaac   1080
   ctcttcactt tgcccatcat gctgagccgc aaggaatcaa acgagatcga aaccgcctct   1140
   aacaacttct tcgtctcgcc atacgtttcc cagatcctca agtactcgga gtccgtccaa   1200
   ttcccggaca accctcccaa caagtacgtc gttgataacc tgaacctcat cgtgaacaag   1260
   aagagcactc tgacatacaa gtactcgtcc gtcgctaacc tgctcttcaa caactacaag   1320
   taccacgaca acatcgcttc taacaacaac gccgagaacc tcaagaaggt caagaaggaa   1380
   gacggaagca tgcacatcgt tgagcagtac ttgactcaaa acgtcgataa cgttaagggt   1440
   cacaacttca tcgtgttgtc cttcaagaac gaggaaaggc tgaccatcgc taagaagaac   1500
   aaggagttct actggatctc tggcgaaatc aaggacgttg atgtgagcca ggtcatccaa   1560
   aagtacaaca gattcaagca ccacatgttc gtgatcggca aggtcaaccg tcgcgagtca   1620
   actacactgc acaacaactt gctgaagctc ttggccttga tcctgcaggg actggtgcca   1680
   ctctccgacg ccatcacatt cgccgagcaa aagctcaact gcaagtacaa gaagttcgag   1740
   ttcaactaa                                                           1749
<210> 3
   <211> 1911
   <212> DNA
   <213> Autographa californica nucleopolyhedrovirus
<400> 3
   atgatccgta catccagcca cgtcctgaac gtccaagaaa acatcatgac ttccaactgt 60
   gcttccagcc cctactcctg tgaggccact tcagcctgcg ctgaggccca gcaactgcag 120
   gtggacacag gtggcgataa gatcgtgaac aaccaggtca ccatgactca aatcaacttc 180
   aacgcttcct acacctctgc cagcactccc tctcgtgcta gcttcgacaa ctcatactcg 240
   gagttctgcg acaagcaacc taacgattac ttgtcttact acaaccaccc aaccccggac 300
   ggagctgata ctgtcatctc cgactctgaa accgctgccg ctagcaactt cctcgcctca 360
   gttaactcgc tcactgacaa cgatttggtg gagtgtctgc tcaagaccac tgacaacctg 420
   gaggaagctg tgtcctctgc ctactacagc gagtcactcg aacagccagt ggtcgaacaa 480
   ccctctccta gctcagctta ccacgccgag tccttcgaac actctgctgg tgtcaaccag 540
   ccgtcggcca caggcaccaa gaggaagttg gacgagtacc tggataactc ccagggagtt 600
   gtgggtcaat tcaacaagat caagttgaga cctaagtaca agaagagcac catccagtca 660
   tgcgctacac tggaacaaac catcaaccac aacactaaca tctgtacagt ggcttccacc 720
   caggagatca ctcactactt cacaaacgac ttcgccccct acctgatgag gttcgacgat 780
   aacgactaca actcgaacag attctccgat cacatgtctg aaaccggtta ctacatgttc 840
   gtcgttaaga agtccgaggt gaagcctttc gaaatcatct tcgccaagta cgtctctaac 900
   gtggtctacg agtacacaaa caactactac atggttgaca accgtgtgtt cgttgtgacc 960
   ttcgataaga tccgcttcat gatcagctac aacctggtta aggagactgg catcgaaatc 1020
   ccacactcac aggacgtctg caacgatgag accgccgctc aaaactgcaa gaagtgtcac 1080
   ttcgtggacg tccaccacac attcaaggcc gctctgacct cctacttcaa cctcgatatg 1140
   tactacgctc agacaacctt cgtgaccttg ctgcaatcac tcggcgagcg taagtgtgga 1200
   ttcctcttgt cgaagttgta cgagatgtac caggacaaga acctcttcac tttgcccatc 1260
   atgctgagcc gcaaggaatc aaacgagatc gaaaccgcct ctaacaactt cttcgtctcg 1320
   ccatacgttt cccagatcct caagtactcg gagtccgtcc aattcccgga caaccctccc 1380
   aacaagtacg tcgttgataa cctgaacctc atcgtgaaca agaagagcac tctgacatac 1440
   aagtactcgt ccgtcgctaa cctgctcttc aacaactaca agtaccacga caacatcgct 1500
   tctaacaaca acgccgagaa cctcaagaag gtcaagaagg aagacggaag catgcacatc 1560
   gttgagcagt acttgactca aaacgtcgat aacgttaagg gtcacaactt catcgtgttg 1620
   tccttcaaga acgaggaaag gctgaccatc gctaagaaga acaaggagtt ctactggatc 1680
   tctggcgaaa tcaaggacgt tgatgtgagc caggtcatcc aaaagtacaa cagattcaag 1740
   caccacatgt tcgtgatcgg caaggtcaac cgtcgcgagt caactacact gcacaacaac 1800
   ttgctgaagc tcttggcctt gatcctgcag ggactggtgc cactctccga cgccatcaca 1860
   ttcgccgagc aaaagctcaa ctgcaagtac aagaagttcg agttcaacta a 1911
<210> 4
   <211> 666
   <212> DNA
   <213> Autographa californica nucleopolyhedrovirus
<400> 4
   atgactcaaa tcaacttcaa cgcttcctac acctctgcca gcactccctc tcgtgctagc 60
   ttcgacaact catactcgga gttctgcgac aagcaaccta acgattactt gtcttactac 120
   aaccacccaa ccccggacgg agctgatact gtcatctccg actctgaaac cgctgccgct 180
   agcaacttcc tcgcctcagt taactcgctc actgacaacg atttggtgga gtgtctgctc 240
   aagaccactg acaacctgga ggaagctgtg tcctctgcct actacagcga gtcactcgaa 300
   cagccagtgg tcgaacaacc ctctcctagc tcagcttacc acgccgagtc cttcgaacac 360
   tctgctggtg tcaaccagcc gtcggccaca ggcaccaaga ggaagttgga cgagtacctg 420
   gataactccc agggagttgt gggtcaattc aacaagatca agttgagacc taagtacaag 480
   aagagcacca tccagtcatg cgctacactg gaacaaacca tcaaccacaa cactaacatc 540
   tgtacagtgg cttccaccca ggagatcact cactacttca caaacgactt cgccccctac 600
   ctgatgaggt tcgacgataa cgactacaac tcgaacagat tctccgatca catgtctgaa 660
   accggt 666
<210> 5
   <211> 828
   <212> DNA
   <213> Autographa californica nucleopolyhedrovirus
<400> 5
   atgatccgta catccagcca cgtcctgaac gtccaagaaa acatcatgac ttccaactgt 60
   gcttccagcc cctactcctg tgaggccact tcagcctgcg ctgaggccca gcaactgcag 120
   gtggacacag gtggcgataa gatcgtgaac aaccaggtca ccatgactca aatcaacttc 180
   aacgcttcct acacctctgc cagcactccc tctcgtgcta gcttcgacaa ctcatactcg 240
   gagttctgcg acaagcaacc taacgattac ttgtcttact acaaccaccc aaccccggac 300
   ggagctgata ctgtcatctc cgactctgaa accgctgccg ctagcaactt cctcgcctca 360
   gttaactcgc tcactgacaa cgatttggtg gagtgtctgc tcaagaccac tgacaacctg 420
   gaggaagctg tgtcctctgc ctactacagc gagtcactcg aacagccagt ggtcgaacaa 480
   ccctctccta gctcagctta ccacgccgag tccttcgaac actctgctgg tgtcaaccag 540
   ccgtcggcca caggcaccaa gaggaagttg gacgagtacc tggataactc ccagggagtt 600
   gtgggtcaat tcaacaagat caagttgaga cctaagtaca agaagagcac catccagtca 660
   tgcgctacac tggaacaaac catcaaccac aacactaaca tctgtacagt ggcttccacc 720
   caggagatca ctcactactt cacaaacgac ttcgccccct acctgatgag gttcgacgat 780
   aacgactaca actcgaacag attctccgat cacatgtctg aaaccggt 828
<210> 6
   <211> 582
   <212> PRT
   <213> Autographa californica nucleopolyhedrovirus
<400> 6
   Met Thr Gln Ile Asn Phe Asn Ala Ser Tyr Thr Ser Ala Ser Thr Pro
   1               5                   10                  15
   Ser Arg Ala Ser Phe Asp Asn Ser Tyr Ser Glu Phe Cys Asp Lys Gln
               20                  25                  30
   Pro Asn Asp Tyr Leu Ser Tyr Tyr Asn His Pro Thr Pro Asp Gly Ala
           35                  40                  45
   Asp Thr Val Ile Ser Asp Ser Glu Thr Ala Ala Ala Ser Asn Phe Leu
       50                  55                  60
   Ala Ser Val Asn Ser Leu Thr Asp Asn Asp Leu Val Glu Cys Leu Leu
   65                  70                  75                  80
   Lys Thr Thr Asp Asn Leu Glu Glu Ala Val Ser Ser Ala Tyr Tyr Ser
                   85                  90                  95
   Glu Ser Leu Glu Gln Pro Val Val Glu Gln Pro Ser Pro Ser Ser Ala
               100                 105                 110
   Tyr His Ala Glu Ser Phe Glu His Ser Ala Gly Val Asn Gln Pro Ser
           115                 120                 125
   Ala Thr Gly Thr Lys Arg Lys Leu Asp Glu Tyr Leu Asp Asn Ser Gln
       130                 135                 140
   Gly Val Val Gly Gln Phe Asn Lys Ile Lys Leu Arg Pro Lys Tyr Lys
   145                 150                 155                 160
   Lys Ser Thr Ile Gln Ser Cys Ala Thr Leu Glu Gln Thr Ile Asn His
                   165                 170                 175
   Asn Thr Asn Ile Cys Thr Val Ala Ser Thr Gln Glu Ile Thr His Tyr
               180                 185                 190
   Phe Thr Asn Asp Phe Ala Pro Tyr Leu Met Arg Phe Asp Asp Asn Asp
           195                 200                 205
   Tyr Asn Ser Asn Arg Phe Ser Asp His Met Ser Glu Thr Gly Tyr Tyr
       210                 215                 220
   Met Phe Val Val Lys Lys Ser Glu Val Lys Pro Phe Glu Ile Ile Phe
   225                 230                 235                 240
   Ala Lys Tyr Val Ser Asn Val Val Tyr Glu Tyr Thr Asn Asn Tyr Tyr
                   245                 250                 255
   Met Val Asp Asn Arg Val Phe Val Val Thr Phe Asp Lys Ile Arg Phe
               260                 265                 270
   Met Ile Ser Tyr Asn Leu Val Lys Glu Thr Gly Ile Glu Ile Pro His
           275                 280                 285
   Ser Gln Asp Val Cys Asn Asp Glu Thr Ala Ala Gln Asn Cys Lys Lys
       290                 295                 300
   Cys His Phe Val Asp Val His His Thr Phe Lys Ala Ala Leu Thr Ser
   305                 310                 315                 320
   Tyr Phe Asn Leu Asp Met Tyr Tyr Ala Gln Thr Thr Phe Val Thr Leu
                   325                 330                 335
   Leu Gln Ser Leu Gly Glu Arg Lys Cys Gly Phe Leu Leu Ser Lys Leu
               340                 345                 350
   Tyr Glu Met Tyr Gln Asp Lys Asn Leu Phe Thr Leu Pro Ile Met Leu
           355                 360                 365
   Ser Arg Lys Glu Ser Asn Glu Ile Glu Thr Ala Ser Asn Asn Phe Phe
       370                 375                 380
   Val Ser Pro Tyr Val Ser Gln Ile Leu Lys Tyr Ser Glu Ser Val Gln
   385                 390                 395                 400
   Phe Pro Asp Asn Pro Pro Asn Lys Tyr Val Val Asp Asn Leu Asn Leu
                   405                 410                 415
   Ile Val Asn Lys Lys Ser Thr Leu Thr Tyr Lys Tyr Ser Ser Val Ala
               420                 425                 430
   Asn Leu Leu Phe Asn Asn Tyr Lys Tyr His Asp Asn Ile Ala Ser Asn
           435                 440                 445
   Asn Asn Ala Glu Asn Leu Lys Lys Val Lys Lys Glu Asp Gly Ser Met
       450                 455                 460
   His Ile Val Glu Gln Tyr Leu Thr Gln Asn Val Asp Asn Val Lys Gly
   465                 470                 475                 480
   His Asn Phe Ile Val Leu Ser Phe Lys Asn Glu Glu Arg Leu Thr Ile
                   485                 490                 495
   Ala Lys Lys Asn Lys Glu Phe Tyr Trp Ile Ser Gly Glu Ile Lys Asp
               500                 505                 510
   Val Asp Val Ser Gln Val Ile Gln Lys Tyr Asn Arg Phe Lys His His
           515                 520                 525
   Met Phe Val Ile Gly Lys Val Asn Arg Arg Glu Ser Thr Thr Leu His
       530                 535                 540
   Asn Asn Leu Leu Lys Leu Leu Ala Leu Ile Leu Gln Gly Leu Val Pro
   545 550 555 560
   Leu Ser Asp Ala Ile Thr Phe Ala Glu Gln Lys Leu Asn Cys Lys Tyr
   565 570 575
   Lys Lys Phe Glu Phe Asn
   580
<210> 7
   <211> 636
   <212> PRT
   <213> Autographa californica nucleopolyhedrovirus
<400> 7
   Met Ile Arg Thr Ser Ser His Val Leu Asn Val Gln Glu Asn Ile Met
   1               5                   10                  15
   Thr Ser Asn Cys Ala Ser Ser Pro Tyr Ser Cys Glu Ala Thr Ser Ala
               20                  25                  30
   Cys Ala Glu Ala Gln Gln Leu Gln Val Asp Thr Gly Gly Asp Lys Ile
           35                  40                  45
   Val Asn Asn Gln Val Thr Met Thr Gln Ile Asn Phe Asn Ala Ser Tyr
       50                  55                  60
   Thr Ser Ala Ser Thr Pro Ser Arg Ala Ser Phe Asp Asn Ser Tyr Ser
   65                  70                  75                  80
   Glu Phe Cys Asp Lys Gln Pro Asn Asp Tyr Leu Ser Tyr Tyr Asn His
                   85                  90                  95
   Pro Thr Pro Asp Gly Ala Asp Thr Val Ile Ser Asp Ser Glu Thr Ala
               100                 105                 110
   Ala Ala Ser Asn Phe Leu Ala Ser Val Asn Ser Leu Thr Asp Asn Asp
           115                 120                 125
   Leu Val Glu Cys Leu Leu Lys Thr Thr Asp Asn Leu Glu Glu Ala Val
       130                 135                 140
   Ser Ser Ala Tyr Tyr Ser Glu Ser Leu Glu Gln Pro Val Val Glu Gln
   145                 150                 155                 160
   Pro Ser Pro Ser Ser Ala Tyr His Ala Glu Ser Phe Glu His Ser Ala
                   165                 170                 175
   Gly Val Asn Gln Pro Ser Ala Thr Gly Thr Lys Arg Lys Leu Asp Glu
               180                 185                 190
   Tyr Leu Asp Asn Ser Gln Gly Val Val Gly Gln Phe Asn Lys Ile Lys
           195                 200                 205
   Leu Arg Pro Lys Tyr Lys Lys Ser Thr Ile Gln Ser Cys Ala Thr Leu
       210                 215                 220
   Glu Gln Thr Ile Asn His Asn Thr Asn Ile Cys Thr Val Ala Ser Thr
   225                 230                 235                 240
   Gln Glu Ile Thr His Tyr Phe Thr Asn Asp Phe Ala Pro Tyr Leu Met
                   245                 250                 255
   Arg Phe Asp Asp Asn Asp Tyr Asn Ser Asn Arg Phe Ser Asp His Met
               260                 265                 270
   Ser Glu Thr Gly Tyr Tyr Met Phe Val Val Lys Lys Ser Glu Val Lys
           275                 280                 285
   Pro Phe Glu Ile Ile Phe Ala Lys Tyr Val Ser Asn Val Val Tyr Glu
       290                 295                 300
   Tyr Thr Asn Asn Tyr Tyr Met Val Asp Asn Arg Val Phe Val Val Thr
   305                 310                 315                 320
   Phe Asp Lys Ile Arg Phe Met Ile Ser Tyr Asn Leu Val Lys Glu Thr
                   325                 330                 335
   Gly Ile Glu Ile Pro His Ser Gln Asp Val Cys Asn Asp Glu Thr Ala
               340                 345                 350
   Ala Gln Asn Cys Lys Lys Cys His Phe Val Asp Val His His Thr Phe
           355                 360                 365
   Lys Ala Ala Leu Thr Ser Tyr Phe Asn Leu Asp Met Tyr Tyr Ala Gln
       370                 375                 380
   Thr Thr Phe Val Thr Leu Leu Gln Ser Leu Gly Glu Arg Lys Cys Gly
   385                 390                 395                 400
   Phe Leu Leu Ser Lys Leu Tyr Glu Met Tyr Gln Asp Lys Asn Leu Phe
                   405                 410                 415
   Thr Leu Pro Ile Met Leu Ser Arg Lys Glu Ser Asn Glu Ile Glu Thr
               420                 425                 430
   Ala Ser Asn Asn Phe Phe Val Ser Pro Tyr Val Ser Gln Ile Leu Lys
   435 440 445
   Tyr Ser Glu Ser Val Gln Phe Pro Asp Asn Pro Pro Asn Lys Tyr Val
   450 455 460
   Val Asp Asn Leu Asn Leu Ile Val Asn Lys Lys Ser Thr Leu Thr Tyr
   465 470 475 480
   Lys Tyr Ser Ser Val Ala Asn Leu Leu Phe Asn Asn Tyr Lys Tyr His
   485 490 495
   Asp Asn Ile Ala Ser Asn Asn Asn Ala Glu Asn Leu Lys Lys Val Lys
   500 505 510
   Lys Glu Asp Gly Ser Met His Ile Val Glu Gln Tyr Leu Thr Gln Asn
   515 520 525
   Val Asp Asn Val Lys Gly His Asn Phe Ile Val Leu Ser Phe Lys Asn
   530 535 540
   Glu Glu Arg Leu Thr Ile Ala Lys Lys Asn Lys Glu Phe Tyr Trp Ile
   545 550 555 560
   Ser Gly Glu Ile Lys Asp Val Asp Val Ser Gln Val Ile Gln Lys Tyr
   565 570 575
   Asn Arg Phe Lys His His Met Phe Val Ile Gly Lys Val Asn Arg Arg
   580 585 590
   Glu Ser Thr Thr Leu His Asn Asn Leu Leu Lys Leu Leu Ala Leu Ile
   595 600 605
   Leu Gln Gly Leu Val Pro Leu Ser Asp Ala Ile Thr Phe Ala Glu Gln
   610 615 620
   Lys Leu Asn Cys Lys Tyr Lys Lys Phe Glu Phe Asn
   625 630 635
<210> 8
   <211> 222
   <212> PRT
   <213> Autographa californica nucleopolyhedrovirus
<400> 8
   Met Thr Gln Ile Asn Phe Asn Ala Ser Tyr Thr Ser Ala Ser Thr Pro
   1 5 10 15
   Ser Arg Ala Ser Phe Asp Asn Ser Tyr Ser Glu Phe Cys Asp Lys Gln
   20 25 30
   Pro Asn Asp Tyr Leu Ser Tyr Tyr Asn His Pro Thr Pro Asp Gly Ala
   35 40 45
   Asp Thr Val Ile Ser Asp Ser Glu Thr Ala Ala Ala Ser Asn Phe Leu
   50 55 60
   Ala Ser Val Asn Ser Leu Thr Asp Asn Asp Leu Val Glu Cys Leu Leu
   65 70 75 80
   Lys Thr Thr Asp Asn Leu Glu Glu Ala Val Ser Ser Ala Tyr Tyr Ser
   85 90 95
   Glu Ser Leu Glu Gln Pro Val Val Glu Gln Pro Ser Pro Ser Ser Ala
   100 105 110
   Tyr His Ala Glu Ser Phe Glu His Ser Ala Gly Val Asn Gln Pro Ser
   115 120 125
   Ala Thr Gly Thr Lys Arg Lys Leu Asp Glu Tyr Leu Asp Asn Ser Gln
   130 135 140
   Gly Val Val Gly Gln Phe Asn Lys Ile Lys Leu Arg Pro Lys Tyr Lys
   145 150 155 160
   Lys Ser Thr Ile Gln Ser Cys Ala Thr Leu Glu Gln Thr Ile Asn His
   165 170 175
   Asn Thr Asn Ile Cys Thr Val Ala Ser Thr Gln Glu Ile Thr His Tyr
   180 185 190
   Phe Thr Asn Asp Phe Ala Pro Tyr Leu Met Arg Phe Asp Asp Asn Asp
   195 200 205
   Tyr Asn Ser Asn Arg Phe Ser Asp His Met Ser Glu Thr Gly
   210 215 220
<210> 9
   <211> 276
   <212> PRT
   <213> Autographa californica nucleopolyhedrovirus
<400> 9
   Met Ile Arg Thr Ser Ser His Val Leu Asn Val Gln Glu Asn Ile Met
   1 5 10 15
   Thr Ser Asn Cys Ala Ser Ser Pro Tyr Ser Cys Glu Ala Thr Ser Ala
               20                  25                  30
   Cys Ala Glu Ala Gln Gln Leu Gln Val Asp Thr Gly Gly Asp Lys Ile
           35                  40                  45
   Val Asn Asn Gln Val Thr Met Thr Gln Ile Asn Phe Asn Ala Ser Tyr
       50                  55                  60
   Thr Ser Ala Ser Thr Pro Ser Arg Ala Ser Phe Asp Asn Ser Tyr Ser
   65                  70                  75                  80
   Glu Phe Cys Asp Lys Gln Pro Asn Asp Tyr Leu Ser Tyr Tyr Asn His
                   85                  90                  95
   Pro Thr Pro Asp Gly Ala Asp Thr Val Ile Ser Asp Ser Glu Thr Ala
               100                 105                 110
   Ala Ala Ser Asn Phe Leu Ala Ser Val Asn Ser Leu Thr Asp Asn Asp
           115                 120                 125
   Leu Val Glu Cys Leu Leu Lys Thr Thr Asp Asn Leu Glu Glu Ala Val
       130                 135                 140
   Ser Ser Ala Tyr Tyr Ser Glu Ser Leu Glu Gln Pro Val Val Glu Gln
   145                 150                 155                 160
   Pro Ser Pro Ser Ser Ala Tyr His Ala Glu Ser Phe Glu His Ser Ala
                   165                 170                 175
   Gly Val Asn Gln Pro Ser Ala Thr Gly Thr Lys Arg Lys Leu Asp Glu
               180                 185                 190
   Tyr Leu Asp Asn Ser Gln Gly Val Val Gly Gln Phe Asn Lys Ile Lys
           195                 200                 205
   Leu Arg Pro Lys Tyr Lys Lys Ser Thr Ile Gln Ser Cys Ala Thr Leu
       210                 215                 220
   Glu Gln Thr Ile Asn His Asn Thr Asn Ile Cys Thr Val Ala Ser Thr
   225                 230                 235                 240
   Gln Glu Ile Thr His Tyr Phe Thr Asn Asp Phe Ala Pro Tyr Leu Met
                   245                 250                 255
   Arg Phe Asp Asp Asn Asp Tyr Asn Ser Asn Arg Phe Ser Asp His Met
               260                 265                 270
   Ser Glu Thr Gly
   275
<210> 10
   <211> 128
   <212> DNA
   <213> Autographa californica nucleopolyhedrovirus
<400> 10
   atcatggaga taattaaaat gataaccatc tcgcaaataa ataagtattt tactgttttc 60
   gtaacagttt tgtaataaaa aaacctataa atattccgga ttattcatac cgtcccacca 120
   tcgggcgc 128
<210> 11
   <211> 122
   <212> DNA
   <213> Autographa californica nucleopolyhedrovirus
<400> 11
   atacggacct ttaattcaac ccaacacaat atattatagt taaataagaa ttattatcaa 60
   atcatttgta tattaattaa aatactatac tgtaaattac attttattta caatcactcg 120
   ac 122
<210> 12
   <211> 571
   <212> DNA
   <213> Artificial Sequence
<220>
   <223> Recombinant chimeric promoter
<400> 12
   aaaaacatcg attagggtga ctgaaggtta cattggggta ggttatggtt aatacgtaat      60
   ggtttaacac caaaacgata tcatggattt tatataaggt gtaataatat ttttaatgag     120
   tggacgcgtc gggtcaatgt cctgcctatt gacgtcataa catattaggt gattatatta     180
   aaaatagttt aaactcaaat attacttgca agtttaagtt tcatcataat ctgatcataa     240
   gtttcaccca aacagaaacc aaaagcataa ctatcgaata tctttagctt cccatgaaga     300
   aagattaccg taaccatcac taggatttta tacgattgta gaaaataaag tattctcagt     360
   ctcttttcag agcgctataa aaaggggtgc attctcggta agagtacagt tgaactcaca     420
   tcgagttaac tccacgctgc agtctcgaga tacggacctt taattcaacc caacacaata     480
   tattatagtt aaataagaat tattatcaaa tcatttgtat attaattaaa atactatact     540
   gtaaattaca ttttatttac aatcactcga c                                    571
<210> 13
   <211> 465
   <212> DNA
   <213> Artificial Sequence
<220>
   <223> Recombinant chimeric promoter
<400> 13
   ggtaccaaat tccgttttgc gacgatgcag agtttttgaa caggctgctc aaacacatag      60
   atccgtaccc gctcagtcgg atgtattaca atgcagccaa taccatgttt tacacgacta     120
   tggaaaacta tgccgtgtcc aattgcaagt tcaacattga ggattacaat aacatattta     180
   aggtgatgga aaatattagg aaacacagca acaaaaattc aaacgaccaa gacgagttaa     240
   acatatattt gggagttcag tcgtcgaatg caaagcgtaa aaaatattaa taaggtaaaa     300
   attacagcta cataaattac acaatttaaa ctgcagtctg gagatacgga cctttaattc     360
   aacccaacac aatatattat agttaaataa gaattattat caaatcattt gtatattaat     420
   taaaatacta tactgtaaat tacattttat ttacaatcac tcgac                     465
<210> 14
   <211> 436
   <212> DNA
   <213> Autographa californica nucleopolyhedrovirus
<400> 14
   aaaaacatcg attagggtga ctgaaggtta cattggggta ggttatggtt aatacgtaat 60
   ggtttaacac caaaacgata tcatggattt tatataaggt gtaataatat ttttaatgag 120
   tggacgcgtc gggtcaatgt cctgcctatt gacgtcataa catattaggt gattatatta 180
   aaaatagttt aaactcaaat attacttgca agtttaagtt tcatcataat ctgatcataa 240
   gtttcaccca aacagaaacc aaaagcataa ctatcgaata tctttagctt cccatgaaga 300
   aagattaccg taaccatcac taggatttta tacgattgta gaaaataaag tattctcagt 360
   ctcttttcag agcgctataa aaaggggtgc attctcggta agagtacagt tgaactcaca 420
   tcgagttaac tccacg 436
<210> 15
   <211> 1176
   <212> DNA
   <213> Artificial Sequence
<220>
   <223> Recombinant chimeric promoter
<400> 15
   cttgaatgtt agtgaaaccc cctgcgacac aagtattaca ttccttagtg cttgaatcct 60
   ttaggaaaga aaagccaatt ttcaaaatct tagcacttgt taactcgcga aaaagaccaa 120
   cagatttccc atactacaat tcgacattag aaatgtaaac ccattatcat tatttacgcc 180
   tcatttccat ccaataataa gtttaagtac gttgagataa aactggctta cctagaactt 240
   gacatggcga cctcttgcac tctgtatctc aagtcaactt tctctatcca aatatttgat 300
   aacatttgac atgatattga agtaagattg ttactaaggc ttacattgta atattactga 360
   cgcaagttct ttatcaataa aatagctgaa aacaaaaaaa aaaacatcga ttagggtgac 420
   tgaaggttac attggggtag gttatggtta atacgtaatg gtttaacacc aaaacgatat 480
   catggattga ctttataaat tttatataag gtgtaataat atttttaatg agtggacgcg 540
   tcgggtcaat gtcctgccta ttgacgtcat aacatattag gtgattatat taaaaatact 600
   caaatattac ttgcaagttt aagtttcatc ataatctgat cataagtttc acccaaacag 660
   aaaccaaaag cataactatc tgctatttga atatctttag cttcccatga agaaagatta 720
   ccgtaaccat cactaggatt ttatacgatt gtagaaaata aagtattctc agtctctttt 780
   cagtttaaaa tctgctggca tttttacaag tcgctgtatc agtcaatgtt tatacaatat 840
   gtcaatgtac tttcgtatta atcagaaaaa aatattctac tagttttgat aagctatcac 900
   ttttgttaca ttgtactgcc ctttacagtt catcaggtat ttatgaatga catattggag 960
   aaacatcgta atcagtccag tataaaaagg ggtgcattct cggtaagagt acagttgaac 1020
   tcacatcgag ttaactccac gctgcagtct cgagatacgg acctttaatt caacccaaca 1080
   caatatatta tagttaaata agaattatta tcaaatcatt tgtatattaa ttaaaatact 1140
   atactgtaaa ttacatttta tttacaatca ctcgac 1176
<210> 16
   <211> 250
   <212> DNA
   <213> Artificial Sequence
<220>
   <223> Recombinant chimeric promoter
<400> 16
   atcatggaga taattaaaat gataaccatc tcgcaaataa ataagtattt tactgttttc 60
   gtaacagttt tgtaataaaa aaacctataa atattccgga ttattcatac cgtcccacca 120
   tcgggcgcat acggaccttt aattcaaccc aacacaatat attatagtta aataagaatt 180
   attatcaaat catttgtata ttaattaaaa tactatactg taaattacat tttatttaca 240
   atcactcgac 250
<210> 17
   <211> 3163
   <212> DNA
   <213> Artificial Sequence
<220>
   <223> Recombinant expression cassette
<400> 17
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatt 1920
   gggtcatcta gattcgaaag cggccgcgac tagtgagctc gtcgacgtag gcctttgaat 1980
   tccgcgcgct tcggaccggg atccgcgccc gatggtggga cggtatgaat aatccggaat 2040
   atttataggt ttttttatta caaaactgtt acgaaaacag taaaatactt atttatttgc 2100
   gagatggtta tcattttaat tatctccatg atctattaat attccggagt atacatcgat 2160
   gttgacccca acaaaagatt tataattaat cataatcacg aacaacaaca agtcaatgaa 2220
   acaaataaac aagttgtcga taaaacattc ataaatgaca cagcaacata caattcttgc 2280
   ataataaaaa tttaaatgac atcatatttg agaataacaa atgacattat ccctcgattg 2340
   tgttttacaa gtagaattct acccgtaaag cgagtttagt tttgaaaaac aaatgacatc 2400
   atttgtataa tgacatcatc ccctgattgt gttttacaag tagaattcta tccgtaaagc 2460
   gagttcagtt ttgaaaacaa atgagtcata cctaaacacg ttaataatct tctgatatca 2520
   gcttatgact caagttatga gccgtgtgca aaacatgaga taagtttatg acatcatcca 2580
   ctgatcgtgc gttacaagta gaattctact cgtaaagcca gttcggttat gagccgtgtg 2640
   caaaacatga catcagctta tgactcatac ttgattgtgt tttacgcgta gaattctact 2700
   cgtaaagcga gttcggttat gagccgtgtg caaaacatga catcagctta tgagtcataa 2760
   ttaatcgtgc gttacaagta gaattctact cgtaaagcga gttgaaggat catatttagt 2820
   tgcgtttatg agataagatt gaaagcacgt gtaaaatgtt tcccgcgcgt tggcacaact 2880
   atttacaatg cggccaagtt ataaaagatt ctaatctgat atgttttaaa acacctttgc 2940
   ggcccgagtt gtttgcgtac gtgactagcg aagaagatgt gtggaccgca gaacagatag 3000
   taaaacaaaa ccctagtatt ggagcaataa tcgatgagct catacggacc tttaattcaa 3060
   cccaacacaa tatattatag ttaaataaga attattatca aatcatttgt atattaatta 3120
   aaatactata ctgtaaatta cattttattt acaatcactc gac 3163
<210> 18
   <211> 3656
   <212> DNA
   <213> Artificial Sequence
<220>
   <223> Recombinant expression cassette
<400> 18
   ttagttgaac tcgaacttct tgtacttgca gttgagcttt tgctcggcga atgtgatggc      60
   gtcggagagt ggcaccagtc cctgcaggat caaggccaag agcttcagca agttgttgtg     120
   cagtgtagtt gactcgcgac ggttgacctt gccgatcacg aacatgtggt gcttgaatct     180
   gttgtacttt tggatgacct ggctcacatc aacgtccttg atttcgccag agatccagta     240
   gaactccttg ttcttcttag cgatggtcag cctttcctcg ttcttgaagg acaacacgat     300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatt 1920
   gggtcatcta gattcgaaag cggccgcgac tagtgagctc gtcgacgtag gcctttgaat 1980
   tccgcgcgct tcggaccggg atccgcgccc gatggtggga cggtatgaat aatccggaat 2040
   atttataggt ttttttatta caaaactgtt acgaaaacag taaaatactt atttatttgc 2100
   gagatggtta tcattttaat tatctccatg atctattaat attccggagt atacatcgat 2160
   gttgacccca acaaaagatt tataattaat cataatcacg aacaacaaca agtcaatgaa 2220
   acaaataaac aagttgtcga taaaacattc ataaatgaca cagcaacata caattcttgc 2280
   ataataaaaa tttaaatgac atcatatttg agaataacaa atgacattat ccctcgattg 2340
   tgttttacaa gtagaattct acccgtaaag cgagtttagt tttgaaaaac aaatgacatc 2400
   atttgtataa tgacatcatc ccctgattgt gttttacaag tagaattcta tccgtaaagc 2460
   gagttcagtt ttgaaaacaa atgagtcata cctaaacacg ttaataatct tctgatatca 2520
   gcttatgact caagttatga gccgtgtgca aaacatgaga taagtttatg acatcatcca 2580
   ctgatcgtgc gttacaagta gaattctact cgtaaagcca gttcggttat gagccgtgtg 2640
   caaaacatga catcagctta tgactcatac ttgattgtgt tttacgcgta gaattctact 2700
   cgtaaagcga gttcggttat gagccgtgtg caaaacatga catcagctta tgagtcataa 2760
   ttaatcgtgc gttacaagta gaattctact cgtaaagcga gttgaaggat catatttagt 2820
   tgcgtttatg agataagatt gaaagcacgt gtaaaatgtt tcccgcgcgt tggcacaact 2880
   atttacaatg cggccaagtt ataaaagatt ctaatctgat atgttttaaa acacctttgc 2940
   ggcccgagtt gtttgcgtac gtgactagcg aagaagatgt gtggaccgca gaacagatag 3000
   taaaacaaaa ccctagtatt ggagcaataa tcgatgagct cgtcgacgta ggcctttgaa 3060
   ttccgcgcgc ttcggaccgg gatccaaaaa catcgattag ggtgactgaa ggttacattg 3120
   gggtaggtta tggttaatac gtaatggttt aacaccaaaa cgatatcatg gattttatat 3180
   aaggtgtaat aatattttta atgagtggac gcgtcgggtc aatgtcctgc ctattgacgt 3240
   cataacatat taggtgatta tattaaaaat agtttaaact caaatattac ttgcaagttt 3300
   aagtttcatc ataatctgat cataagtttc acccaaacag aaaccaaaag cataactatc 3360
   gaatatcttt agcttcccat gaagaaagat taccgtaacc atcactagga ttttatacga 3420
   ttgtagaaaa taaagtattc tcagtctctt ttcagagcgc tataaaaagg ggtgcattct 3480
   cggtaagagt acagttgaac tcacatcgag ttaactccac gctgcagtct cgagatacgg 3540
   acctttaatt caacccaaca caatatatta tagttaaata agaattatta tcaaatcatt 3600
   tgtatattaa ttaaaatact atactgtaaa ttacatttta tttacaatca ctcgac 3656
<210> 19
   <211> 3541
   <212> DNA
   <213> Artificial Sequence
<220>
   <223> Recombinant expression cassette
<400> 19
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatc 1920
   tagattcgaa agcggccgcg actagtgagc tcgtcgacgt aggcctttga attccgcgcg 1980
   cttcggaccg ggatccgcgc ccgatggtgg gacggtatga ataatccgga atatttatag 2040
   gtttttttat tacaaaactg ttacgaaaac agtaaaatac ttatttattt gcgagatggt 2100
   tatcatttta attatctcca tgatctatta atattccgga gtatacatcg atgttgaccc 2160
   caacaaaaga tttataatta atcataatca cgaacaacaa caagtcaatg aaacaaataa 2220
   acaagttgtc gataaaacat tcataaatga cacagcaaca tacaattctt gcataataaa 2280
   aatttaaatg acatcatatt tgagaataac aaatgacatt atccctcgat tgtgttttac 2340
   aagtagaatt ctacccgtaa agcgagttta gttttgaaaa acaaatgaca tcatttgtat 2400
   aatgacatca tcccctgatt gtgttttaca agtagaattc tatccgtaaa gcgagttcag 2460
   ttttgaaaac aaatgagtca tacctaaaca cgttaataat cttctgatat cagcttatga 2520
   ctcaagttat gagccgtgtg caaaacatga gataagttta tgacatcatc cactgatcgt 2580
   gcgttacaag tagaattcta ctcgtaaagc cagttcggtt atgagccgtg tgcaaaacat 2640
   gacatcagct tatgactcat acttgattgt gttttacgcg tagaattcta ctcgtaaagc 2700
   gagttcggtt atgagccgtg tgcaaaacat gacatcagct tatgagtcat aattaatcgt 2760
   gcgttacaag tagaattcta ctcgtaaagc gagttgaagg atcatattta gttgcgttta 2820
   tgagataaga ttgaaagcac gtgtaaaatg tttcccgcgc gttggcacaa ctatttacaa 2880
   tgcggccaag ttataaaaga ttctaatctg atatgtttta aaacaccttt gcggcccgag 2940
   ttgtttgcgt acgtgactag cgaagaagat gtgtggaccg cagaacagat agtaaaacaa 3000
   aaccctagta ttggagcaat aatcgatgag ctcgtcgacg taggcctttg aattccgcgc 3060
   gcttcggacc gggatcggta ccaaattccg ttttgcgacg atgcagagtt tttgaacagg 3120
   ctgctcaaac acatagatcc gtacccgctc agtcggatgt attacaatgc agccaatacc 3180
   atgttttaca cgactatgga aaactatgcc gtgtccaatt gcaagttcaa cattgaggat 3240
   tacaataaca tatttaaggt gatggaaaat attaggaaac acagcaacaa aaattcaaac 3300
   gaccaagacg agttaaacat atatttggga gttcagtcgt cgaatgcaaa gcgtaaaaaa 3360
   tattaataag gtaaaaatta cagctacata aattacacaa tttaaactgc agtctggaga 3420
   tacggacctt taattcaacc caacacaata tattatagtt aaataagaat tattatcaaa 3480
   tcatttgtat attaattaaa atactatact gtaaattaca ttttatttac aatcactcga 3540
   c 3541
<210> 20
   <211> 3512
   <212> DNA
   <213> Artificial Sequence
<220>
   <223> Recombinant expression cassette
<400> 20
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatc 1920
   tagatgcatt cgcgaggtac cgagctcgaa ttcactggcc gtcgttttac aacgtcgtga 1980
   ctgggaaaac cctggcgtta cccaacttaa tcgccttgca gcacatcccc ctttcgccag 2040
   ctgctagcac catggctcga gcgtggagtt aactcgatgt gagttcaact gtactcttac 2100
   cgagaatgca ccccttttta tagcgctctg aaaagagact gagaatactt tattttctac 2160
   aatcgtataa aatcctagtg atggttacgg taatctttct tcatgggaag ctaaagatat 2220
   tcgatagtta tgcttttggt ttctgtttgg gtgaaactta tgatcagatt atgatgaaac 2280
   ttaaacttgc aagtaatatt tgagtttaaa ctatttttaa tataatcacc taatatgtta 2340
   tgacgtcaat aggcaggaca ttgacccgac gcgtccactc attaaaaata ttattacacc 2400
   ttatataaaa tccatgatat cgttttggtg ttaaaccatt acgtattaac cataacctac 2460
   cccaatgtaa ccttcagtca ccctaatcga tgtttttgta tacatcgatg ttgaccccaa 2520
   caaaagattt ataattaatc ataatcacga acaacaacaa gtcaatgaaa caaataaaca 2580
   agttgtcgat aaaacattca taaatgacac agcaacatac aattcttgca taataaaaat 2640
   ttaaatgaca tcatatttga gaataacaaa tgacattatc cctcgattgt gttttacaag 2700
   tagaattcta cccgtaaagc gagtttagtt ttgaaaaaca aatgacatca tttgtataat 2760
   gacatcatcc cctgattgtg ttttacaagt agaattctat ccgtaaagcg agttcagttt 2820
   tgaaaacaaa tgagtcatac ctaaacacgt taataatctt ctgatatcag cttatgactc 2880
   aagttatgag ccgtgtgcaa aacatgagat aagtttatga catcatccac tgatcgtgcg 2940
   ttacaagtag aattctactc gtaaagccag ttcggttatg agccgtgtgc aaaacatgac 3000
   atcagcttat gactcatact tgattgtgtt ttacgcgtag aattctactc gtaaagcgag 3060
   ttcggttatg agccgtgtgc aaaacatgac atcagcttat gagtcataat taatcgtgcg 3120
   ttacaagtag aattctactc gtaaagcgag ttgaaggatc atatttagtt gcgtttatga 3180
   gataagattg aaagcacgtg taaaatgttt cccgcgcgtt ggcacaacta tttacaatgc 3240
   ggccaagtta taaaagattc taatctgata tgttttaaaa cacctttgcg gcccgagttg 3300
   tttgcgtacg tgactagcga agaagatgtg tggaccgcag aacagatagt aaaacaaaac 3360
   cctagtattg gagcaataat cgatgagctc atacggacct ttaattcaac ccaacacaat 3420
   atattatagt taaataagaa ttattatcaa atcatttgta tattaattaa aatactatac 3480
   tgtaaattac attttattta caatcactcg ac 3512
<210> 21
   <211> 4005
   <212> DNA
   <213> Artificial Sequence
<220>
   <223> Recombinant expression cassette
<400> 21
   ttagttgaac tcgaacttct tgtacttgca gttgagcttt tgctcggcga atgtgatggc     60
   gtcggagagt ggcaccagtc cctgcaggat caaggccaag agcttcagca agttgttgtg    120
   cagtgtagtt gactcgcgac ggttgacctt gccgatcacg aacatgtggt gcttgaatct    180
   gttgtacttt tggatgacct ggctcacatc aacgtccttg atttcgccag agatccagta    240
   gaactccttg ttcttcttag cgatggtcag cctttcctcg ttcttgaagg acaacacgat    300
   gaagttgtga cccttaacgt tatcgacgtt ttgagtcaag tactgctcaa cgatgtgcat    360
   gcttccgtct tccttcttga ccttcttgag gttctcggcg ttgttgttag aagcgatgtt    420
   gtcgtggtac ttgtagttgt tgaagagcag gttagcgacg gacgagtact tgtatgtcag    480
   agtgctcttc ttgttcacga tgaggttcag gttatcaacg acgtacttgt tgggagggtt    540
   gtccgggaat tggacggact ccgagtactt gaggatctgg gaaacgtatg gcgagacgaa    600
   gaagttgtta gaggcggttt cgatctcgtt tgattccttg cggctcagca tgatgggcaa    660
   agtgaagagg ttcttgtcct ggtacatctc gtacaacttc gacaagagga atccacactt    720
   acgctcgccg agtgattgca gcaaggtcac gaaggttgtc tgagcgtagt acatatcgag    780
   gttgaagtag gaggtcagag cggccttgaa tgtgtggtgg acgtccacga agtgacactt    840
   cttgcagttt tgagcggcgg tctcatcgtt gcagacgtcc tgtgagtgtg ggatttcgat    900
   gccagtctcc ttaaccaggt tgtagctgat catgaagcgg atcttatcga aggtcacaac    960
   gaacacacgg ttgtcaacca tgtagtagtt gtttgtgtac tcgtagacca cgttagagac   1020
   gtacttggcg aagatgattt cgaaaggctt cacctcggac ttcttaacga cgaacatgta   1080
   gtaaccggtt tcagacatgt gatcggagaa tctgttcgag ttgtagtcgt tatcgtcgaa   1140
   cctcatcagg tagggggcga agtcgtttgt gaagtagtga gtgatctcct gggtggaagc   1200
   cactgtacag atgttagtgt tgtggttgat ggtttgttcc agtgtagcgc atgactggat   1260
   ggtgctcttc ttgtacttag gtctcaactt gatcttgttg aattgaccca caactccctg   1320
   ggagttatcc aggtactcgt ccaacttcct cttggtgcct gtggccgacg gctggttgac   1380
   accagcagag tgttcgaagg actcggcgtg gtaagctgag ctaggagagg gttgttcgac   1440
   cactggctgt tcgagtgact cgctgtagta ggcagaggac acagcttcct ccaggttgtc   1500
   agtggtcttg agcagacact ccaccaaatc gttgtcagtg agcgagttaa ctgaggcgag   1560
   gaagttgcta gcggcagcgg tttcagagtc ggagatgaca gtatcagctc cgtccggggt   1620
   tgggtggttg tagtaagaca agtaatcgtt aggttgcttg tcgcagaact ccgagtatga   1680
   gttgtcgaag ctagcacgag agggagtgct ggcagaggtg taggaagcgt tgaagttgat   1740
   ttgagtcatg gtgacctggt tgttcacgat cttatcgcca cctgtgtcca cctgcagttg   1800
   ctgggcctca gcgcaggctg aagtggcctc acaggagtag gggctggaag cacagttgga   1860
   agtcatgatg ttttcttgga cgttcaggac gtggctggat gtacggatca tagatctatc 1920
   tagatgcatt cgcgaggtac cgagctcgaa ttcactggcc gtcgttttac aacgtcgtga 1980
   ctgggaaaac cctggcgtta cccaacttaa tcgccttgca gcacatcccc ctttcgccag 2040
   ctgctagcac catggctcga gcgtggagtt aactcgatgt gagttcaact gtactcttac 2100
   cgagaatgca ccccttttta tagcgctctg aaaagagact gagaatactt tattttctac 2160
   aatcgtataa aatcctagtg atggttacgg taatctttct tcatgggaag ctaaagatat 2220
   tcgatagtta tgcttttggt ttctgtttgg gtgaaactta tgatcagatt atgatgaaac 2280
   ttaaacttgc aagtaatatt tgagtttaaa ctatttttaa tataatcacc taatatgtta 2340
   tgacgtcaat aggcaggaca ttgacccgac gcgtccactc attaaaaata ttattacacc 2400
   ttatataaaa tccatgatat cgttttggtg ttaaaccatt acgtattaac cataacctac 2460
   cccaatgtaa ccttcagtca ccctaatcga tgtttttgta tacatcgatg ttgaccccaa 2520
   caaaagattt ataattaatc ataatcacga acaacaacaa gtcaatgaaa caaataaaca 2580
   agttgtcgat aaaacattca taaatgacac agcaacatac aattcttgca taataaaaat 2640
   ttaaatgaca tcatatttga gaataacaaa tgacattatc cctcgattgt gttttacaag 2700
   tagaattcta cccgtaaagc gagtttagtt ttgaaaaaca aatgacatca tttgtataat 2760
   gacatcatcc cctgattgtg ttttacaagt agaattctat ccgtaaagcg agttcagttt 2820
   tgaaaacaaa tgagtcatac ctaaacacgt taataatctt ctgatatcag cttatgactc 2880
   aagttatgag ccgtgtgcaa aacatgagat aagtttatga catcatccac tgatcgtgcg 2940
   ttacaagtag aattctactc gtaaagccag ttcggttatg agccgtgtgc aaaacatgac 3000
   atcagcttat gactcatact tgattgtgtt ttacgcgtag aattctactc gtaaagcgag 3060
   ttcggttatg agccgtgtgc aaaacatgac atcagcttat gagtcataat taatcgtgcg 3120
   ttacaagtag aattctactc gtaaagcgag ttgaaggatc atatttagtt gcgtttatga 3180
   gataagattg aaagcacgtg taaaatgttt cccgcgcgtt ggcacaacta tttacaatgc 3240
   ggccaagtta taaaagattc taatctgata tgttttaaaa cacctttgcg gcccgagttg 3300
   tttgcgtacg tgactagcga agaagatgtg tggaccgcag aacagatagt aaaacaaaac 3360
   cctagtattg gagcaataat cgatgagctc gtcgacgtag gcctttgaat tccgcgcgct 3420
   tcggaccggg atccaaaaac atcgattagg gtgactgaag gttacattgg ggtaggttat 3480
   ggttaatacg taatggttta acaccaaaac gatatcatgg attttatata aggtgtaata 3540
   atatttttaa tgagtggacg cgtcgggtca atgtcctgcc tattgacgtc ataacatatt 3600
   aggtgattat attaaaaata gtttaaactc aaatattact tgcaagttta agtttcatca 3660
   taatctgatc ataagtttca cccaaacaga aaccaaaagc ataactatcg aatatcttta 3720
   gcttcccatg aagaaagatt accgtaacca tcactaggat tttatacgat tgtagaaaat     3780
   aaagtattct cagtctcttt tcagagcgct ataaaaaggg gtgcattctc ggtaagagta     3840
   cagttgaact cacatcgagt taactccacg ctgcagtctc gagatacgga cctttaattc     3900
   aacccaacac aatatattat agttaaataa gaattattat caaatcattt gtatattaat     3960
   taaaatacta tactgtaaat tacattttat ttacaatcac tcgac                     4005
<210> 22
   <211> 3898
   <212> DNA
   <213> Artificial Sequence
<220>
   <223> Recombinant expression cassette
<400> 22
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatc 1920
   tagatgcatt cgcgaggtac cgagctcgaa ttcactggcc gtcgttttac aacgtcgtga 1980
   ctgggaaaac cctggcgtta cccaacttaa tcgccttgca gcacatcccc ctttcgccag 2040
   ctgctagcac catggctcga gcgtggagtt aactcgatgt gagttcaact gtactcttac 2100
   cgagaatgca ccccttttta tagcgctctg aaaagagact gagaatactt tattttctac 2160
   aatcgtataa aatcctagtg atggttacgg taatctttct tcatgggaag ctaaagatat 2220
   tcgatagtta tgcttttggt ttctgtttgg gtgaaactta tgatcagatt atgatgaaac 2280
   ttaaacttgc aagtaatatt tgagtttaaa ctatttttaa tataatcacc taatatgtta 2340
   tgacgtcaat aggcaggaca ttgacccgac gcgtccactc attaaaaata ttattacacc 2400
   ttatataaaa tccatgatat cgttttggtg ttaaaccatt acgtattaac cataacctac 2460
   cccaatgtaa ccttcagtca ccctaatcga tgtttttgta tacatcgatg ttgaccccaa 2520
   caaaagattt ataattaatc ataatcacga acaacaacaa gtcaatgaaa caaataaaca 2580
   agttgtcgat aaaacattca taaatgacac agcaacatac aattcttgca taataaaaat 2640
   ttaaatgaca tcatatttga gaataacaaa tgacattatc cctcgattgt gttttacaag 2700
   tagaattcta cccgtaaagc gagtttagtt ttgaaaaaca aatgacatca tttgtataat 2760
   gacatcatcc cctgattgtg ttttacaagt agaattctat ccgtaaagcg agttcagttt 2820
   tgaaaacaaa tgagtcatac ctaaacacgt taataatctt ctgatatcag cttatgactc 2880
   aagttatgag ccgtgtgcaa aacatgagat aagtttatga catcatccac tgatcgtgcg 2940
   ttacaagtag aattctactc gtaaagccag ttcggttatg agccgtgtgc aaaacatgac 3000
   atcagcttat gactcatact tgattgtgtt ttacgcgtag aattctactc gtaaagcgag 3060
   ttcggttatg agccgtgtgc aaaacatgac atcagcttat gagtcataat taatcgtgcg 3120
   ttacaagtag aattctactc gtaaagcgag ttgaaggatc atatttagtt gcgtttatga 3180
   gataagattg aaagcacgtg taaaatgttt cccgcgcgtt ggcacaacta tttacaatgc 3240
   ggccaagtta taaaagattc taatctgata tgttttaaaa cacctttgcg gcccgagttg 3300
   tttgcgtacg tgactagcga agaagatgtg tggaccgcag aacagatagt aaaacaaaac 3360
   cctagtattg gagcaataat cgatgagctc gtcgacgtag gcctttgaat tccgcgcgct 3420
   tcggaccggg atcggtacca aattccgttt tgcgacgatg cagagttttt gaacaggctg 3480
   ctcaaacaca tagatccgta cccgctcagt cggatgtatt acaatgcagc caataccatg 3540
   ttttacacga ctatggaaaa ctatgccgtg tccaattgca agttcaacat tgaggattac 3600
   aataacatat ttaaggtgat ggaaaatatt aggaaacaca gcaacaaaaa ttcaaacgac 3660
   caagacgagt taaacatata tttgggagtt cagtcgtcga atgcaaagcg taaaaaatat 3720
   taataaggta aaaattacag ctacataaat tacacaattt aaactgcagt ctggagatac 3780
   ggacctttaa ttcaacccaa cacaatatat tatagttaaa taagaattat tatcaaatca 3840
   tttgtatatt aattaaaata ctatactgta aattacattt tatttacaat cactcgac 3898
<210> 23
   <211> 3179
   <212> DNA
   <213> Artificial Sequence
<220>
   <223> Recombinant expression cassette
<400> 23
   ttagttgaac tcgaacttct tgtacttgca gttgagcttt tgctcggcga atgtgatggc      60
   gtcggagagt ggcaccagtc cctgcaggat caaggccaag agcttcagca agttgttgtg     120
   cagtgtagtt gactcgcgac ggttgacctt gccgatcacg aacatgtggt gcttgaatct     180
   gttgtacttt tggatgacct ggctcacatc aacgtccttg atttcgccag agatccagta     240
   gaactccttg ttcttcttag cgatggtcag cctttcctcg ttcttgaagg acaacacgat     300
   gaagttgtga cccttaacgt tatcgacgtt ttgagtcaag tactgctcaa cgatgtgcat    360
   gcttccgtct tccttcttga ccttcttgag gttctcggcg ttgttgttag aagcgatgtt     420
   gtcgtggtac ttgtagttgt tgaagagcag gttagcgacg gacgagtact tgtatgtcag     480
   agtgctcttc ttgttcacga tgaggttcag gttatcaacg acgtacttgt tgggagggtt     540
   gtccgggaat tggacggact ccgagtactt gaggatctgg gaaacgtatg gcgagacgaa     600
   gaagttgtta gaggcggttt cgatctcgtt tgattccttg cggctcagca tgatgggcaa     660
   agtgaagagg ttcttgtcct ggtacatctc gtacaacttc gacaagagga atccacactt     720
   acgctcgccg agtgattgca gcaaggtcac gaaggttgtc tgagcgtagt acatatcgag     780
   gttgaagtag gaggtcagag cggccttgaa tgtgtggtgg acgtccacga agtgacactt     840
   cttgcagttt tgagcggcgg tctcatcgtt gcagacgtcc tgtgagtgtg ggatttcgat     900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatt 1920
   gggtcatcta gattcgaaag cggccgcgac tagtgagctc gtcgacgtag gcctttgaat 1980
   tccgcgcgct tcggaccggg atccgcgccc gatggtggga cggtatgaat aatccggaat 2040
   atttataggt ttttttatta caaaactgtt acgaaaacag taaaatactt atttatttgc 2100
   gagatggtta tcattttaat tatctccatg atctattaat attccggagt atacatcgat 2160
   gttgacccca acaaaagatt tataattaat cataatcacg aacaacaaca agtcaatgaa 2220
   acaaataaac aagttgtcga taaaacattc ataaatgaca cagcaacata caattcttgc 2280
   ataataaaaa tttaaatgac atcatatttg agaataacaa atgacattat ccctcgattg 2340
   tgttttacaa gtagaattct acccgtaaag cgagtttagt tttgaaaaac aaatgacatc 2400
   atttgtataa tgacatcatc ccctgattgt gttttacaag tagaattcta tccgtaaagc 2460
   gagttcagtt ttgaaaacaa atgagtcata cctaaacacg ttaataatct tctgatatca 2520
   gcttatgact caagttatga gccgtgtgca aaacatgaga taagtttatg acatcatcca 2580
   ctgatcgtgc gttacaagta gaattctact cgtaaagcca gttcggttat gagccgtgtg 2640
   caaaacatga catcagctta tgactcatac ttgattgtgt tttacgcgta gaattctact 2700
   cgtaaagcga gttcggttat gagccgtgtg caaaacatga catcagctta tgagtcataa 2760
   ttaatcgtgc gttacaagta gaattctact cgtaaagcga gttgaaggat catatttagt 2820
   tgcgtttatg agataagatt gaaagcacgt gtaaaatgtt tcccgcgcgt tggcacaact 2880
   atttacaatg cggccaagtt ataaaagatt ctaatctgat atgttttaaa acacctttgc 2940
   ggcccgagtt gtttgcgtac gtgactagcg aagaagatgt gtggaccgca gaacagatag 3000
   taaaacaaaa ccctagtatt ggagcaataa tcgattccgg aatattaata gatcatggag 3060
   ataattaaaa tgataaccat ctcgcaaata aataagtatt ttactgtttt cgtaacagtt 3120
   ttgtaataaa aaaacctata aatattccgg attattcata ccgtcccacc atcgggcgc 3179
<210> 24
   <211> 3528
   <212> DNA
   <213> Artificial Sequence
<220>
   <223> Recombinant expression cassette
<400> 24
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatc 1920
   tagatgcatt cgcgaggtac cgagctcgaa ttcactggcc gtcgttttac aacgtcgtga 1980
   ctgggaaaac cctggcgtta cccaacttaa tcgccttgca gcacatcccc ctttcgccag 2040
   ctgctagcac catggctcga gcgtggagtt aactcgatgt gagttcaact gtactcttac 2100
   cgagaatgca ccccttttta tagcgctctg aaaagagact gagaatactt tattttctac 2160
   aatcgtataa aatcctagtg atggttacgg taatctttct tcatgggaag ctaaagatat 2220
   tcgatagtta tgcttttggt ttctgtttgg gtgaaactta tgatcagatt atgatgaaac 2280
   ttaaacttgc aagtaatatt tgagtttaaa ctatttttaa tataatcacc taatatgtta 2340
   tgacgtcaat aggcaggaca ttgacccgac gcgtccactc attaaaaata ttattacacc 2400
   ttatataaaa tccatgatat cgttttggtg ttaaaccatt acgtattaac cataacctac 2460
   cccaatgtaa ccttcagtca ccctaatcga tgtttttgta tacatcgatg ttgaccccaa 2520
   caaaagattt ataattaatc ataatcacga acaacaacaa gtcaatgaaa caaataaaca 2580
   agttgtcgat aaaacattca taaatgacac agcaacatac aattcttgca taataaaaat 2640
   ttaaatgaca tcatatttga gaataacaaa tgacattatc cctcgattgt gttttacaag 2700
   tagaattcta cccgtaaagc gagtttagtt ttgaaaaaca aatgacatca tttgtataat 2760
   gacatcatcc cctgattgtg ttttacaagt agaattctat ccgtaaagcg agttcagttt 2820
   tgaaaacaaa tgagtcatac ctaaacacgt taataatctt ctgatatcag cttatgactc 2880
   aagttatgag ccgtgtgcaa aacatgagat aagtttatga catcatccac tgatcgtgcg 2940
   ttacaagtag aattctactc gtaaagccag ttcggttatg agccgtgtgc aaaacatgac 3000
   atcagcttat gactcatact tgattgtgtt ttacgcgtag aattctactc gtaaagcgag 3060
   ttcggttatg agccgtgtgc aaaacatgac atcagcttat gagtcataat taatcgtgcg 3120
   ttacaagtag aattctactc gtaaagcgag ttgaaggatc atatttagtt gcgtttatga 3180
   gataagattg aaagcacgtg taaaatgttt cccgcgcgtt ggcacaacta tttacaatgc 3240
   ggccaagtta taaaagattc taatctgata tgttttaaaa cacctttgcg gcccgagttg 3300
   tttgcgtacg tgactagcga agaagatgtg tggaccgcag aacagatagt aaaacaaaac 3360
   cctagtattg gagcaataat cgattccgga atattaatag atcatggaga taattaaaat 3420
   gataaccatc tcgcaaataa ataagtattt tactgttttc gtaacagttt tgtaataaaa 3480
   aaacctataa atattccgga ttattcatac cgtcccacca tcgggcgc 3528
<210> 25
   <211> 3291
   <212> DNA
   <213> Artificial Sequence
<220>
   <223> Recombinant expression cassette
<400> 25
   ttagttgaac tcgaacttct tgtacttgca gttgagcttt tgctcggcga atgtgatggc      60
   gtcggagagt ggcaccagtc cctgcaggat caaggccaag agcttcagca agttgttgtg     120
   cagtgtagtt gactcgcgac ggttgacctt gccgatcacg aacatgtggt gcttgaatct     180
   gttgtacttt tggatgacct ggctcacatc aacgtccttg atttcgccag agatccagta     240
   gaactccttg ttcttcttag cgatggtcag cctttcctcg ttcttgaagg acaacacgat     300
   gaagttgtga cccttaacgt tatcgacgtt ttgagtcaag tactgctcaa cgatgtgcat     360
   gcttccgtct tccttcttga ccttcttgag gttctcggcg ttgttgttag aagcgatgtt     420
   gtcgtggtac ttgtagttgt tgaagagcag gttagcgacg gacgagtact tgtatgtcag     480
   agtgctcttc ttgttcacga tgaggttcag gttatcaacg acgtacttgt tgggagggtt     540
   gtccgggaat tggacggact ccgagtactt gaggatctgg gaaacgtatg gcgagacgaa     600
   gaagttgtta gaggcggttt cgatctcgtt tgattccttg cggctcagca tgatgggcaa     660
   agtgaagagg ttcttgtcct ggtacatctc gtacaacttc gacaagagga atccacactt     720
   acgctcgccg agtgattgca gcaaggtcac gaaggttgtc tgagcgtagt acatatcgag     780
   gttgaagtag gaggtcagag cggccttgaa tgtgtggtgg acgtccacga agtgacactt     840
   cttgcagttt tgagcggcgg tctcatcgtt gcagacgtcc tgtgagtgtg ggatttcgat     900
   gccagtctcc ttaaccaggt tgtagctgat catgaagcgg atcttatcga aggtcacaac     960
   gaacacacgg ttgtcaacca tgtagtagtt gtttgtgtac tcgtagacca cgttagagac    1020
   gtacttggcg aagatgattt cgaaaggctt cacctcggac ttcttaacga cgaacatgta    1080
   gtaaccggtt tcagacatgt gatcggagaa tctgttcgag ttgtagtcgt tatcgtcgaa    1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatt 1920
   gggtcatcta gattcgaaag cggccgcgac tagtgagctc gtcgacgtag gcctttgaat 1980
   tccgcgcgct tcggaccggg atccgcgccc gatggtggga cggtatgaat aatccggaat 2040
   atttataggt ttttttatta caaaactgtt acgaaaacag taaaatactt atttatttgc 2100
   gagatggtta tcattttaat tatctccatg atctattaat attccggagt atacatcgat 2160
   gttgacccca acaaaagatt tataattaat cataatcacg aacaacaaca agtcaatgaa 2220
   acaaataaac aagttgtcga taaaacattc ataaatgaca cagcaacata caattcttgc 2280
   ataataaaaa tttaaatgac atcatatttg agaataacaa atgacattat ccctcgattg 2340
   tgttttacaa gtagaattct acccgtaaag cgagtttagt tttgaaaaac aaatgacatc 2400
   atttgtataa tgacatcatc ccctgattgt gttttacaag tagaattcta tccgtaaagc 2460
   gagttcagtt ttgaaaacaa atgagtcata cctaaacacg ttaataatct tctgatatca 2520
   gcttatgact caagttatga gccgtgtgca aaacatgaga taagtttatg acatcatcca 2580
   ctgatcgtgc gttacaagta gaattctact cgtaaagcca gttcggttat gagccgtgtg 2640
   caaaacatga catcagctta tgactcatac ttgattgtgt tttacgcgta gaattctact 2700
   cgtaaagcga gttcggttat gagccgtgtg caaaacatga catcagctta tgagtcataa 2760
   ttaatcgtgc gttacaagta gaattctact cgtaaagcga gttgaaggat catatttagt 2820
   tgcgtttatg agataagatt gaaagcacgt gtaaaatgtt tcccgcgcgt tggcacaact 2880
   atttacaatg cggccaagtt ataaaagatt ctaatctgat atgttttaaa acacctttgc 2940
   ggcccgagtt gtttgcgtac gtgactagcg aagaagatgt gtggaccgca gaacagatag 3000
   taaaacaaaa ccctagtatt ggagcaataa tcgatgagct catcatggag ataattaaaa      3060
   tgataaccat ctcgcaaata aataagtatt ttactgtttt cgtaacagtt ttgtaataaa      3120
   aaaacctata aatattccgg attattcata ccgtcccacc atcgggcgca tacggacctt      3180
   taattcaacc caacacaata tattatagtt aaataagaat tattatcaaa tcatttgtat      3240
   attaattaaa atactatact gtaaattaca ttttatttac aatcactcga c               3291
<210> 26
   <211> 3640
   <212> DNA
   <213> Artificial Sequence
<220>
   <223> Recombinant expression cassette
<400> 26
   ttagttgaac tcgaacttct tgtacttgca gttgagcttt tgctcggcga atgtgatggc 60
   gtcggagagt ggcaccagtc cctgcaggat caaggccaag agcttcagca agttgttgtg 120
   cagtgtagtt gactcgcgac ggttgacctt gccgatcacg aacatgtggt gcttgaatct 180
   gttgtacttt tggatgacct ggctcacatc aacgtccttg atttcgccag agatccagta 240
   gaactccttg ttcttcttag cgatggtcag cctttcctcg ttcttgaagg acaacacgat 300
   gaagttgtga cccttaacgt tatcgacgtt ttgagtcaag tactgctcaa cgatgtgcat 360
   gcttccgtct tccttcttga ccttcttgag gttctcggcg ttgttgttag aagcgatgtt 420
   gtcgtggtac ttgtagttgt tgaagagcag gttagcgacg gacgagtact tgtatgtcag 480
   agtgctcttc ttgttcacga tgaggttcag gttatcaacg acgtacttgt tgggagggtt 540
   gtccgggaat tggacggact ccgagtactt gaggatctgg gaaacgtatg gcgagacgaa 600
   gaagttgtta gaggcggttt cgatctcgtt tgattccttg cggctcagca tgatgggcaa 660
   agtgaagagg ttcttgtcct ggtacatctc gtacaacttc gacaagagga atccacactt 720
   acgctcgccg agtgattgca gcaaggtcac gaaggttgtc tgagcgtagt acatatcgag 780
   gttgaagtag gaggtcagag cggccttgaa tgtgtggtgg acgtccacga agtgacactt 840
   cttgcagttt tgagcggcgg tctcatcgtt gcagacgtcc tgtgagtgtg ggatttcgat 900
   gccagtctcc ttaaccaggt tgtagctgat catgaagcgg atcttatcga aggtcacaac 960
   gaacacacgg ttgtcaacca tgtagtagtt gtttgtgtac tcgtagacca cgttagagac 1020
   gtacttggcg aagatgattt cgaaaggctt cacctcggac ttcttaacga cgaacatgta 1080
   gtaaccggtt tcagacatgt gatcggagaa tctgttcgag ttgtagtcgt tatcgtcgaa 1140
   cctcatcagg tagggggcga agtcgtttgt gaagtagtga gtgatctcct gggtggaagc 1200
   cactgtacag atgttagtgt tgtggttgat ggtttgttcc agtgtagcgc atgactggat 1260
   ggtgctcttc ttgtacttag gtctcaactt gatcttgttg aattgaccca caactccctg 1320
   ggagttatcc aggtactcgt ccaacttcct cttggtgcct gtggccgacg gctggttgac 1380
   accagcagag tgttcgaagg actcggcgtg gtaagctgag ctaggagagg gttgttcgac 1440
   cactggctgt tcgagtgact cgctgtagta ggcagaggac acagcttcct ccaggttgtc 1500
   agtggtcttg agcagacact ccaccaaatc gttgtcagtg agcgagttaa ctgaggcgag 1560
   gaagttgcta gcggcagcgg tttcagagtc ggagatgaca gtatcagctc cgtccggggt 1620
   tgggtggttg tagtaagaca agtaatcgtt aggttgcttg tcgcagaact ccgagtatga 1680
   gttgtcgaag ctagcacgag agggagtgct ggcagaggtg taggaagcgt tgaagttgat 1740
   ttgagtcatg gtgacctggt tgttcacgat cttatcgcca cctgtgtcca cctgcagttg 1800
   ctgggcctca gcgcaggctg aagtggcctc acaggagtag gggctggaag cacagttgga 1860
   agtcatgatg ttttcttgga cgttcaggac gtggctggat gtacggatca tagatctatc 1920
   tagatgcatt cgcgaggtac cgagctcgaa ttcactggcc gtcgttttac aacgtcgtga 1980
   ctgggaaaac cctggcgtta cccaacttaa tcgccttgca gcacatcccc ctttcgccag 2040
   ctgctagcac catggctcga gcgtggagtt aactcgatgt gagttcaact gtactcttac 2100
   cgagaatgca ccccttttta tagcgctctg aaaagagact gagaatactt tattttctac 2160
   aatcgtataa aatcctagtg atggttacgg taatctttct tcatgggaag ctaaagatat 2220
   tcgatagtta tgcttttggt ttctgtttgg gtgaaactta tgatcagatt atgatgaaac 2280
   ttaaacttgc aagtaatatt tgagtttaaa ctatttttaa tataatcacc taatatgtta 2340
   tgacgtcaat aggcaggaca ttgacccgac gcgtccactc attaaaaata ttattacacc 2400
   ttatataaaa tccatgatat cgttttggtg ttaaaccatt acgtattaac cataacctac 2460
   cccaatgtaa ccttcagtca ccctaatcga tgtttttgta tacatcgatg ttgaccccaa 2520
   caaaagattt ataattaatc ataatcacga acaacaacaa gtcaatgaaa caaataaaca 2580
   agttgtcgat aaaacattca taaatgacac agcaacatac aattcttgca taataaaaat 2640
   ttaaatgaca tcatatttga gaataacaaa tgacattatc cctcgattgt gttttacaag 2700
   tagaattcta cccgtaaagc gagtttagtt ttgaaaaaca aatgacatca tttgtataat 2760
   gacatcatcc cctgattgtg ttttacaagt agaattctat ccgtaaagcg agttcagttt 2820
   tgaaaacaaa tgagtcatac ctaaacacgt taataatctt ctgatatcag cttatgactc 2880
   aagttatgag ccgtgtgcaa aacatgagat aagtttatga catcatccac tgatcgtgcg 2940
   ttacaagtag aattctactc gtaaagccag ttcggttatg agccgtgtgc aaaacatgac 3000
   atcagcttat gactcatact tgattgtgtt ttacgcgtag aattctactc gtaaagcgag 3060
   ttcggttatg agccgtgtgc aaaacatgac atcagcttat gagtcataat taatcgtgcg 3120
   ttacaagtag aattctactc gtaaagcgag ttgaaggatc atatttagtt gcgtttatga 3180
   gataagattg aaagcacgtg taaaatgttt cccgcgcgtt ggcacaacta tttacaatgc 3240
   ggccaagtta taaaagattc taatctgata tgttttaaaa cacctttgcg gcccgagttg 3300
   tttgcgtacg tgactagcga agaagatgtg tggaccgcag aacagatagt aaaacaaaac 3360
   cctagtattg gagcaataat cgatgagctc atcatggaga taattaaaat gataaccatc 3420
   tcgcaaataa ataagtattt tactgttttc gtaacagttt tgtaataaaa aaacctataa 3480
   atattccgga ttattcatac cgtcccacca tcgggcgcat acggaccttt aattcaaccc 3540
   aacacaatat attatagtta aataagaatt attatcaaat catttgtata ttaattaaaa 3600
   tactatactg taaattacat tttatttaca atcactcgac 3640
<210> 27
   <211> 881
   <212> DNA
   <213> Autographa californica nucleopolyhedrovirus
<400> 27
   atcgatgttg accccaacaa aagatttata attaatcata atcacgaaca acaacaagtc 60
   aatgaaacaa ataaacaagt tgtcgataaa acattcataa atgacacagc aacatacaat 120
   tcttgcataa taaaaattta aatgacatca tatttgagaa taacaaatga cattatccct 180
   cgattgtgtt ttacaagtag aattctaccc gtaaagcgag tttagttttg aaaaacaaat 240
   gacatcattt gtataatgac atcatcccct gattgtgttt tacaagtaga attctatccg 300
   taaagcgagt tcagttttga aaacaaatga gtcataccta aacacgttaa taatcttctg 360
   atatcagctt atgactcaag ttatgagccg tgtgcaaaac atgagataag tttatgacat 420
   catccactga tcgtgcgtta caagtagaat tctactcgta aagccagttc ggttatgagc 480
   cgtgtgcaaa acatgacatc agcttatgac tcatacttga ttgtgtttta cgcgtagaat 540
   tctactcgta aagcgagttc ggttatgagc cgtgtgcaaa acatgacatc agcttatgag 600
   tcataattaa tcgtgcgtta caagtagaat tctactcgta aagcgagttg aaggatcata 660
   tttagttgcg tttatgagat aagattgaaa gcacgtgtaa aatgtttccc gcgcgttggc 720
   acaactattt acaatgcggc caagttataa aagattctaa tctgatatgt tttaaaacac 780
   ctttgcggcc cgagttgttt gcgtacgtga ctagcgaaga agatgtgtgg accgcagaac 840
   agatagtaaa acaaaaccct agtattggag caataatcga t 881
<210> 28
   <211> 2124
   <212> DNA
   <213> Artificial Sequence
<220>
   <223> Recombinant DNA construct fusing the Ac-ie-01 cDNA to the polh promoter
<400> 28
   atcatggaga taattaaaat gataaccatc tcgcaaataa ataagtattt tactgttttc      60
   gtaacagttt tgtaataaaa aaacctataa atattccgga ttattcatac cgtcccacca     120
   tcgggcgcgg atcccggtcc gaagcgcgcg gaattcaaag gcctacgtcg acgagctcac     180
   tagtcgcggc cgctttcgaa tctagataga tctatgatcc gtacatccag ccacgtcctg     240
   aacgtccaag aaaacatcat gacttccaac tgtgcttcca gcccctactc ctgtgaggcc     300
   acttcagcct gcgctgaggc ccagcaactg caggtggaca caggtggcga taagatcgtg     360
   aacaaccagg tcaccatgac tcaaatcaac ttcaacgctt cctacacctc tgccagcact     420
   ccctctcgtg ctagcttcga caactcatac tcggagttct gcgacaagca acctaacgat     480
   tacttgtctt actacaacca cccaaccccg gacggagctg atactgtcat ctccgactct     540
   gaaaccgctg ccgctagcaa cttcctcgcc tcagttaact cgctcactga caacgatttg     600
   gtggagtgtc tgctcaagac cactgacaac ctggaggaag ctgtgtcctc tgcctactac     660
   agcgagtcac tcgaacagcc agtggtcgaa caaccctctc ctagctcagc ttaccacgcc     720
   gagtccttcg aacactctgc tggtgtcaac cagccgtcgg ccacaggcac caagaggaag     780
   ttggacgagt acctggataa ctcccaggga gttgtgggtc aattcaacaa gatcaagttg     840
   agacctaagt acaagaagag caccatccag tcatgcgcta cactggaaca aaccatcaac     900
   cacaacacta acatctgtac agtggcttcc acccaggaga tcactcacta cttcacaaac     960
   gacttcgccc cctacctgat gaggttcgac gataacgact acaactcgaa cagattctcc    1020
   gatcacatgt ctgaaaccgg ttactacatg ttcgtcgtta agaagtccga ggtgaagcct    1080
   ttcgaaatca tcttcgccaa gtacgtctct aacgtggtct acgagtacac aaacaactac    1140
   tacatggttg acaaccgtgt gttcgttgtg accttcgata agatccgctt catgatcagc    1200
   tacaacctgg ttaaggagac tggcatcgaa atcccacact cacaggacgt ctgcaacgat    1260
   gagaccgccg ctcaaaactg caagaagtgt cacttcgtgg acgtccacca cacattcaag    1320
   gccgctctga cctcctactt caacctcgat atgtactacg ctcagacaac cttcgtgacc    1380
   ttgctgcaat cactcggcga gcgtaagtgt ggattcctct tgtcgaagtt gtacgagatg    1440
   taccaggaca agaacctctt cactttgccc atcatgctga gccgcaagga atcaaacgag    1500
   atcgaaaccg cctctaacaa cttcttcgtc tcgccatacg tttcccagat cctcaagtac    1560
   tcggagtccg tccaattccc ggacaaccct cccaacaagt acgtcgttga taacctgaac   1620
   ctcatcgtga acaagaagag cactctgaca tacaagtact cgtccgtcgc taacctgctc    1680
   ttcaacaact acaagtacca cgacaacatc gcttctaaca acaacgccga gaacctcaag    1740
   aaggtcaaga aggaagacgg aagcatgcac atcgttgagc agtacttgac tcaaaacgtc    1800
   gataacgtta agggtcacaa cttcatcgtg ttgtccttca agaacgagga aaggctgacc    1860
   atcgctaaga agaacaagga gttctactgg atctctggcg aaatcaagga cgttgatgtg 1920
   agccaggtca tccaaaagta caacagattc aagcaccaca tgttcgtgat cggcaaggtc 1980
   aaccgtcgcg agtcaactac actgcacaac aacttgctga agctcttggc cttgatcctg 2040
   cagggactgg tgccactctc cgacgccatc acattcgccg agcaaaagct caactgcaag 2100
   tacaagaagt tcgagttcaa ctaa 2124
<210> 29
   <211> 911
   <212> DNA
   <213> Artificial Sequence
<220>
   <223> Recombinant DNA construct fusing the GFP cDNA to the polh promoter
<400> 29
   atcatggaga taattaaaat gataaccatc tcgcaaataa ataagtattt tactgttttc 60
   gtaacagttt tgtaataaaa aaacctataa atattccgga ttattcatac cgtcccacca 120
   tcgggcgcgg atccaaggcc actagtgcgg ccgctctgca gtctcgagca tgcggtacca 180
   agcttgaatt catggtgagc aagggcgagg agctgttcac cggggtggtg cccatcctgg 240
   tcgagctgga cggcgacgta aacggccaca agttcagcgt gtccggcgag ggcgagggcg 300
   atgccaccta cggcaagctg accctgaagt tcatctgcac caccggcaag ctgcccgtgc 360
   cctggcccac cctcgtgacc accctgacct acggcgtgca gtgcttcagc cgctaccccg 420
   accacatgaa gcagcacgac ttcttcaagt ccgccatgcc cgaaggctac gtccaggagc 480
   gcaccatctt cttcaaggac gacggcaact acaagacccg cgccgaggtg aagttcgagg 540
   gcgacaccct ggtgaaccgc atcgagctga agggcatcga cttcaaggag gacggcaaca 600
   tcctggggca caagctggag tacaactaca acagccacaa cgtctatatc atggccgaca 660
   agcagaagaa cggcatcatg gtgaacttca agatccgcca caacatcgag gacggcagcg 720
   tgcagctcgc cgaccactac cagcagaaca cccccatcgg cgacggcccc gtgctgctgc 780
   ccgacaacca ctacctgagc acccagtccg ccctgagcaa agaccccaac gagaagcgcg 840
   atcacatggt cctgctggag ttcgtgaccg ccgccgggat cactctcggc atggacgagc 900
   tgtacaagta a 911
<210> 30
   <211> 1201
   <212> DNA
   <213> Artificial Sequence
<220>
   <223> Recombinant chimeric promoter
<400> 30
   atacggacct ttaattcaac ccaacacaat atattatagt taaataagaa ttattatcaa      60
   atcatttgta tattaattaa aatactatac tgtaaattac attttattta caatcactcg     120
   acggatccaa ggccactagt gcggccgcgg gaattcgatt cttgaatgtt agtgaaaccc     180
   cctgcgacac aagtattaca ttccttagtg cttgaatcct ttaggaaaga aaagccaatt     240
   ttcaaaatct tagcacttgt taactcgcga aaaagaccaa cagatttccc atactacaat     300
   tcgacattag aaatgtaaac ccattatcat tatttacgcc tcatttccat ccaataataa     360
   gtttaagtac gttgagataa aactggctta cctagaactt gacatggcga cctcttgcac     420
   tctgtatctc aagtcaactt tctctatcca aatatttgat aacatttgac atgatattga     480
   agtaagattg ttactaaggc ttacattgta atattactga cgcaagttct ttatcaataa     540
   aatagctgaa aacaaaaaaa aaaacatcga ttagggtgac tgaaggttac attggggtag     600
   gttatggtta atacgtaatg gtttaacacc aaaacgatat catggattga ctttataaat     660
   tttatataag gtgtaataat atttttaatg agtggacgcg tcgggtcaat gtcctgccta     720
   ttgacgtcat aacatattag gtgattatat taaaaatact caaatattac ttgcaagttt     780
   aagtttcatc ataatctgat cataagtttc acccaaacag aaaccaaaag cataactatc     840
   tgctatttga atatctttag cttcccatga agaaagatta ccgtaaccat cactaggatt     900
   ttatacgatt gtagaaaata aagtattctc agtctctttt cagtttaaaa tctgctggca     960
   tttttacaag tcgctgtatc agtcaatgtt tatacaatat gtcaatgtac tttcgtatta    1020
   atcagaaaaa aatattctac tagttttgat aagctatcac ttttgttaca ttgtactgcc    1080
   ctttacagtt catcaggtat ttatgaatga catattggag aaacatcgta atcagtccag    1140
   tataaaaagg ggtgcattct cggtaagagt acagttgaac tcacatcgag ttaactccac    1200
   g                                                                    1201
<210> 31
   <211> 1207
   <212> DNA
   <213> Artificial Sequence
<220>
   <223> Recombinant chimeric promoter
<400> 31
   atcatggaga taattaaaat gataaccatc tcgcaaataa ataagtattt tactgttttc 60
   gtaacagttt tgtaataaaa aaacctataa atattccgga ttattcatac cgtcccacca 120
   tcgggcgcgg atccaaggcc actagtgcgg ccgcgggaat tcgattcttg aatgttagtg 180
   aaaccccctg cgacacaagt attacattcc ttagtgcttg aatcctttag gaaagaaaag 240
   ccaattttca aaatcttagc acttgttaac tcgcgaaaaa gaccaacaga tttcccatac 300
   tacaattcga cattagaaat gtaaacccat tatcattatt tacgcctcat ttccatccaa 360
   taataagttt aagtacgttg agataaaact ggcttaccta gaacttgaca tggcgacctc 420
   ttgcactctg tatctcaagt caactttctc tatccaaata tttgataaca tttgacatga 480
   tattgaagta agattgttac taaggcttac attgtaatat tactgacgca agttctttat 540
   caataaaata gctgaaaaca aaaaaaaaaa catcgattag ggtgactgaa ggttacattg 600
   gggtaggtta tggttaatac gtaatggttt aacaccaaaa cgatatcatg gattgacttt 660
   ataaatttta tataaggtgt aataatattt ttaatgagtg gacgcgtcgg gtcaatgtcc 720
   tgcctattga cgtcataaca tattaggtga ttatattaaa aatactcaaa tattacttgc 780
   aagtttaagt ttcatcataa tctgatcata agtttcaccc aaacagaaac caaaagcata 840
   actatctgct atttgaatat ctttagcttc ccatgaagaa agattaccgt aaccatcact 900
   aggattttat acgattgtag aaaataaagt attctcagtc tcttttcagt ttaaaatctg 960
   ctggcatttt tacaagtcgc tgtatcagtc aatgtttata caatatgtca atgtactttc 1020
   gtattaatca gaaaaaaata ttctactagt tttgataagc tatcactttt gttacattgt 1080
   actgcccttt acagttcatc aggtatttat gaatgacata ttggagaaac atcgtaatca 1140
   gtccagtata aaaaggggtg cattctcggt aagagtacag ttgaactcac atcgagttaa 1200
   ctccacg 1207
<210> 32
   <211> 1205
   <212> DNA
   <213> Artificial Sequence
<220>
   <223> Recombinant chimeric promoter
<400> 32
   cttgaatgtt agtgaaaccc cctgcgacac aagtattaca ttccttagtg cttgaatcct 60
   ttaggaaaga aaagccaatt ttcaaaatct tagcacttgt taactcgcga aaaagaccaa 120
   cagatttccc atactacaat tcgacattag aaatgtaaac ccattatcat tatttacgcc 180
   tcatttccat ccaataataa gtttaagtac gttgagataa aactggctta cctagaactt 240
   gacatggcga cctcttgcac tctgtatctc aagtcaactt tctctatcca aatatttgat 300
   aacatttgac atgatattga agtaagattg ttactaaggc ttacattgta atattactga 360
   cgcaagttct ttatcaataa aatagctgaa aacaaaaaaa aaaacatcga ttagggtgac 420
   tgaaggttac attggggtag gttatggtta atacgtaatg gtttaacacc aaaacgatat 480
   catggattga ctttataaat tttatataag gtgtaataat atttttaatg agtggacgcg 540
   tcgggtcaat gtcctgccta ttgacgtcat aacatattag gtgattatat taaaaatact 600
   caaatattac ttgcaagttt aagtttcatc ataatctgat cataagtttc acccaaacag 660
   aaaccaaaag cataactatc tgctatttga atatctttag cttcccatga agaaagatta 720
   ccgtaaccat cactaggatt ttatacgatt gtagaaaata aagtattctc agtctctttt 780
   cagtttaaaa tctgctggca tttttacaag tcgctgtatc agtcaatgtt tatacaatat 840
   gtcaatgtac tttcgtatta atcagaaaaa aatattctac tagttttgat aagctatcac 900
   ttttgttaca ttgtactgcc ctttacagtt catcaggtat ttatgaatga catattggag 960
   aaacatcgta atcagtccag tataaaaagg ggtgcattct cggtaagagt acagttgaac 1020
   tcacatcgag ttaactccac gaatcactag tgaattcgcg gccgctgcag tctcgagatc 1080
   atggagataa ttaaaatgat aaccatctcg caaataaata agtattttac tgttttcgta 1140
   acagttttgt aataaaaaaa cctataaata ttccggatta ttcataccgt cccaccatcg 1200
   ggcgc 1205
<210> 33
   <211> 564
   <212> DNA
   <213> Artificial Sequence
<220>
   <223> Recombinant chimeric promoter
<400> 33
   aaaaacatcg attagggtga ctgaaggtta cattggggta ggttatggtt aatacgtaat 60
   ggtttaacac caaaacgata tcatggattt tatataaggt gtaataatat ttttaatgag 120
   tggacgcgtc gggtcaatgt cctgcctatt gacgtcataa catattaggt gattatatta 180
   aaaatagttt aaactcaaat attacttgca agtttaagtt tcatcataat ctgatcataa 240
   gtttcaccca aacagaaacc aaaagcataa ctatcgaata tctttagctt cccatgaaga 300
   aagattaccg taaccatcac taggatttta tacgattgta gaaaataaag tattctcagt 360
   ctcttttcag agcgctataa aaaggggtgc attctcggta agagtacagt tgaactcaca 420
   tcgagttaac tccacgcatc atggagataa ttaaaatgat aaccatctcg caaataaata 480
   agtattttac tgttttcgta acagttttgt aataaaaaaa cctataaata ttccggatta 540
   ttcataccgt cccaccatcg ggcg 564
<210> 34
   <211> 1041
   <212> DNA
   <213> Autographa californica nucleopolyhedrovirus
<400> 34
   cttgaatgtt agtgaaaccc cctgcgacac aagtattaca ttccttagtg cttgaatcct 60
   ttaggaaaga aaagccaatt ttcaaaatct tagcacttgt taactcgcga aaaagaccaa 120
   cagatttccc atactacaat tcgacattag aaatgtaaac ccattatcat tatttacgcc 180
   tcatttccat ccaataataa gtttaagtac gttgagataa aactggctta cctagaactt     240
   gacatggcga cctcttgcac tctgtatctc aagtcaactt tctctatcca aatatttgat     300
   aacatttgac atgatattga agtaagattg ttactaaggc ttacattgta atattactga     360
   cgcaagttct ttatcaataa aatagctgaa aacaaaaaaa aaaacatcga ttagggtgac     420
   tgaaggttac attggggtag gttatggtta atacgtaatg gtttaacacc aaaacgatat     480
   catggattga ctttataaat tttatataag gtgtaataat atttttaatg agtggacgcg     540
   tcgggtcaat gtcctgccta ttgacgtcat aacatattag gtgattatat taaaaatact     600
   caaatattac ttgcaagttt aagtttcatc ataatctgat cataagtttc acccaaacag     660
   aaaccaaaag cataactatc tgctatttga atatctttag cttcccatga agaaagatta     720
   ccgtaaccat cactaggatt ttatacgatt gtagaaaata aagtattctc agtctctttt     780
   cagtttaaaa tctgctggca tttttacaag tcgctgtatc agtcaatgtt tatacaatat     840
   gtcaatgtac tttcgtatta atcagaaaaa aatattctac tagttttgat aagctatcac     900
   ttttgttaca ttgtactgcc ctttacagtt catcaggtat ttatgaatga catattggag     960
   aaacatcgta atcagtccag tataaaaagg ggtgcattct cggtaagagt acagttgaac    1020
   tcacatcgag ttaactccac g                                              1041
<210> 35
   <211> 2110
   <212> DNA
   <213> Artificial Sequence
<220>
   <223> Recombinant expression cassette
<400> 35
   atcgatgttg accccaacaa aagatttata attaatcata atcacgaaca acaacaagtc 60
   aatgaaacaa ataaacaagt tgtcgataaa acattcataa atgacacagc aacatacaat 120
   tcttgcataa taaaaattta aatgacatca tatttgagaa taacaaatga cattatccct 180
   cgattgtgtt ttacaagtag aattctaccc gtaaagcgag tttagttttg aaaaacaaat 240
   gacatcattt gtataatgac atcatcccct gattgtgttt tacaagtaga attctatccg 300
   taaagcgagt tcagttttga aaacaaatga gtcataccta aacacgttaa taatcttctg 360
   atatcagctt atgactcaag ttatgagccg tgtgcaaaac atgagataag tttatgacat 420
   catccactga tcgtgcgtta caagtagaat tctactcgta aagccagttc ggttatgagc 480
   cgtgtgcaaa acatgacatc agcttatgac tcatacttga ttgtgtttta cgcgtagaat 540
   tctactcgta aagcgagttc ggttatgagc cgtgtgcaaa acatgacatc agcttatgag 600
   tcataattaa tcgtgcgtta caagtagaat tctactcgta aagcgagttg aaggatcata 660
   tttagttgcg tttatgagat aagattgaaa gcacgtgtaa aatgtttccc gcgcgttggc 720
   acaactattt acaatgcggc caagttataa aagattctaa tctgatatgt tttaaaacac 780
   ctttgcggcc cgagttgttt gcgtacgtga ctagcgaaga agatgtgtgg accgcagaac 840
   agatagtaaa acaaaaccct agtattggag caataatcga tgtatactcc ggaatattaa 900
   tagatcatgg agataattaa aatgataacc atctcgcaaa taaataagta ttttactgtt 960
   ttcgtaacag ttttgtaata aaaaaaccta taaatattcc ggattattca taccgtccca 1020
   ccatcgggcg cggatccaag gccactagtg cggccgcggg aattcgattc ttgaatgtta 1080
   gtgaaacccc ctgcgacaca agtattacat tccttagtgc ttgaatcctt taggaaagaa 1140
   aagccaattt tcaaaatctt agcacttgtt aactcgcgaa aaagaccaac agatttccca 1200
   tactacaatt cgacattaga aatgtaaacc cattatcatt atttacgcct catttccatc 1260
   caataataag tttaagtacg ttgagataaa actggcttac ctagaacttg acatggcgac 1320
   ctcttgcact ctgtatctca agtcaacttt ctctatccaa atatttgata acatttgaca 1380
   tgatattgaa gtaagattgt tactaaggct tacattgtaa tattactgac gcaagttctt 1440
   tatcaataaa atagctgaaa acaaaaaaaa aaacatcgat tagggtgact gaaggttaca 1500
   ttggggtagg ttatggttaa tacgtaatgg tttaacacca aaacgatatc atggattgac 1560
   tttataaatt ttatataagg tgtaataata tttttaatga gtggacgcgt cgggtcaatg 1620
   tcctgcctat tgacgtcata acatattagg tgattatatt aaaaatactc aaatattact 1680
   tgcaagttta agtttcatca taatctgatc ataagtttca cccaaacaga aaccaaaagc 1740
   ataactatct gctatttgaa tatctttagc ttcccatgaa gaaagattac cgtaaccatc 1800
   actaggattt tatacgattg tagaaaataa agtattctca gtctcttttc agtttaaaat 1860
   ctgctggcat ttttacaagt cgctgtatca gtcaatgttt atacaatatg tcaatgtact 1920
   ttcgtattaa tcagaaaaaa atattctact agttttgata agctatcact tttgttacat 1980
   tgtactgccc tttacagttc atcaggtatt tatgaatgac atattggaga aacatcgtaa 2040
   tcagtccagt ataaaaaggg gtgcattctc ggtaagagta cagttgaact cacatcgagt 2100
   taactccacg 2110
<210> 36
   <211> 2168
   <212> DNA
   <213> Artificial Sequence
<220>
   <223> Recombinant expression cassette
<400> 36
   atcgatgttg accccaacaa aagatttata attaatcata atcacgaaca acaacaagtc 60
   aatgaaacaa ataaacaagt tgtcgataaa acattcataa atgacacagc aacatacaat 120
   tcttgcataa taaaaattta aatgacatca tatttgagaa taacaaatga cattatccct 180
   cgattgtgtt ttacaagtag aattctaccc gtaaagcgag tttagttttg aaaaacaaat 240
   gacatcattt gtataatgac atcatcccct gattgtgttt tacaagtaga attctatccg 300
   taaagcgagt tcagttttga aaacaaatga gtcataccta aacacgttaa taatcttctg 360
   atatcagctt atgactcaag ttatgagccg tgtgcaaaac atgagataag tttatgacat 420
   catccactga tcgtgcgtta caagtagaat tctactcgta aagccagttc ggttatgagc 480
   cgtgtgcaaa acatgacatc agcttatgac tcatacttga ttgtgtttta cgcgtagaat 540
   tctactcgta aagcgagttc ggttatgagc cgtgtgcaaa acatgacatc agcttatgag 600
   tcataattaa tcgtgcgtta caagtagaat tctactcgta aagcgagttg aaggatcata 660
   tttagttgcg tttatgagat aagattgaaa gcacgtgtaa aatgtttccc gcgcgttggc 720
   acaactattt acaatgcggc caagttataa aagattctaa tctgatatgt tttaaaacac 780
   ctttgcggcc cgagttgttt gcgtacgtga ctagcgaaga agatgtgtgg accgcagaac 840
   agatagtaaa acaaaaccct agtattggag caataatcga tgagctcgtc gacgtaggcc 900
   tttgaattcc gcgcgcttcg gaccgggatc caaggccact agtgcggccg cgggaattcg 960
   attcttgaat gttagtgaaa ccccctgcga cacaagtatt acattcctta gtgcttgaat 1020
   cctttaggaa agaaaagcca attttcaaaa tcttagcact tgttaactcg cgaaaaagac 1080
   caacagattt cccatactac aattcgacat tagaaatgta aacccattat cattatttac 1140
   gcctcatttc catccaataa taagtttaag tacgttgaga taaaactggc ttacctagaa 1200
   cttgacatgg cgacctcttg cactctgtat ctcaagtcaa ctttctctat ccaaatattt 1260
   gataacattt gacatgatat tgaagtaaga ttgttactaa ggcttacatt gtaatattac 1320
   tgacgcaagt tctttatcaa taaaatagct gaaaacaaaa aaaaaaacat cgattagggt 1380
   gactgaaggt tacattgggg taggttatgg ttaatacgta atggtttaac accaaaacga 1440
   tatcatggat tgactttata aattttatat aaggtgtaat aatattttta atgagtggac 1500
   gcgtcgggtc aatgtcctgc ctattgacgt cataacatat taggtgatta tattaaaaat 1560
   actcaaatat tacttgcaag tttaagtttc atcataatct gatcataagt ttcacccaaa 1620
   cagaaaccaa aagcataact atctgctatt tgaatatctt tagcttccca tgaagaaaga 1680
   ttaccgtaac catcactagg attttatacg attgtagaaa ataaagtatt ctcagtctct 1740
   tttcagttta aaatctgctg gcatttttac aagtcgctgt atcagtcaat gtttatacaa 1800
   tatgtcaatg tactttcgta ttaatcagaa aaaaatattc tactagtttt gataagctat 1860
   cacttttgtt acattgtact gccctttaca gttcatcagg tatttatgaa tgacatattg 1920
   gagaaacatc gtaatcagtc cagtataaaa aggggtgcat tctcggtaag agtacagttg 1980
   aactcacatc gagttaactc cacgaatcac tagtgaattc gcggccgctg cagtctcgag 2040
   atcatggaga taattaaaat gataaccatc tcgcaaataa ataagtattt tactgttttc 2100
   gtaacagttt tgtaataaaa aaacctataa atattccgga ttattcatac cgtcccacca 2160
   tcgggcgc 2168
<210> 37
   <211> 2088
   <212> DNA
   <213> Artificial Sequence
<220>
   <223> Recombinant expression cassette
<400> 37
   atcgatgttg accccaacaa aagatttata attaatcata atcacgaaca acaacaagtc 60
   aatgaaacaa ataaacaagt tgtcgataaa acattcataa atgacacagc aacatacaat 120
   tcttgcataa taaaaattta aatgacatca tatttgagaa taacaaatga cattatccct 180
   cgattgtgtt ttacaagtag aattctaccc gtaaagcgag tttagttttg aaaaacaaat 240
   gacatcattt gtataatgac atcatcccct gattgtgttt tacaagtaga attctatccg 300
   taaagcgagt tcagttttga aaacaaatga gtcataccta aacacgttaa taatcttctg 360
   atatcagctt atgactcaag ttatgagccg tgtgcaaaac atgagataag tttatgacat 420
   catccactga tcgtgcgtta caagtagaat tctactcgta aagccagttc ggttatgagc 480
   cgtgtgcaaa acatgacatc agcttatgac tcatacttga ttgtgtttta cgcgtagaat 540
   tctactcgta aagcgagttc ggttatgagc cgtgtgcaaa acatgacatc agcttatgag 600
   tcataattaa tcgtgcgtta caagtagaat tctactcgta aagcgagttg aaggatcata 660
   tttagttgcg tttatgagat aagattgaaa gcacgtgtaa aatgtttccc gcgcgttggc 720
   acaactattt acaatgcggc caagttataa aagattctaa tctgatatgt tttaaaacac 780
   ctttgcggcc cgagttgttt gcgtacgtga ctagcgaaga agatgtgtgg accgcagaac 840
   agatagtaaa acaaaaccct agtattggag caataatcga tgagctcata cggaccttta 900
   attcaaccca acacaatata ttatagttaa ataagaatta ttatcaaatc atttgtatat 960
   taattaaaat actatactgt aaattacatt ttatttacaa tcactcgacg gatccaaggc 1020
   cactagtgcg gccgcgggaa ttcgattctt gaatgttagt gaaaccccct gcgacacaag 1080
   tattacattc cttagtgctt gaatccttta ggaaagaaaa gccaattttc aaaatcttag 1140
   cacttgttaa ctcgcgaaaa agaccaacag atttcccata ctacaattcg acattagaaa 1200
   tgtaaaccca ttatcattat ttacgcctca tttccatcca ataataagtt taagtacgtt 1260
   gagataaaac tggcttacct agaacttgac atggcgacct cttgcactct gtatctcaag 1320
   tcaactttct ctatccaaat atttgataac atttgacatg atattgaagt aagattgtta 1380
   ctaaggctta cattgtaata ttactgacgc aagttcttta tcaataaaat agctgaaaac 1440
   aaaaaaaaaa acatcgatta gggtgactga aggttacatt ggggtaggtt atggttaata 1500
   cgtaatggtt taacaccaaa acgatatcat ggattgactt tataaatttt atataaggtg 1560
   taataatatt tttaatgagt ggacgcgtcg ggtcaatgtc ctgcctattg acgtcataac 1620
   atattaggtg attatattaa aaatactcaa atattacttg caagtttaag tttcatcata 1680
   atctgatcat aagtttcacc caaacagaaa ccaaaagcat aactatctgc tatttgaata 1740
   tctttagctt cccatgaaga aagattaccg taaccatcac taggatttta tacgattgta 1800
   gaaaataaag tattctcagt ctcttttcag tttaaaatct gctggcattt ttacaagtcg 1860
   ctgtatcagt caatgtttat acaatatgtc aatgtacttt cgtattaatc agaaaaaaat 1920
   attctactag ttttgataag ctatcacttt tgttacattg tactgccctt tacagttcat 1980
   caggtattta tgaatgacat attggagaaa catcgtaatc agtccagtat aaaaaggggt 2040
   gcattctcgg taagagtaca gttgaactca catcgagtta actccacg 2088
<210> 38
   <211> 2162
   <212> DNA
   <213> Artificial Sequence
<220>
   <223> Recombinant expression cassette
<900> 38
<210> 39
   <211> 1508
   <212> DNA
   <213> Artificial Sequence
<220>
   <223> Recombinant expression cassette
<400> 39
   atcgatgttg accccaacaa aagatttata attaatcata atcacgaaca acaacaagtc      60
   aatgaaacaa ataaacaagt tgtcgataaa acattcataa atgacacagc aacatacaat     120
   tcttgcataa taaaaattta aatgacatca tatttgagaa taacaaatga cattatccct     180
   cgattgtgtt ttacaagtag aattctaccc gtaaagcgag tttagttttg aaaaacaaat     240
   gacatcattt gtataatgac atcatcccct gattgtgttt tacaagtaga attctatccg 300
   taaagcgagt tcagttttga aaacaaatga gtcataccta aacacgttaa taatcttctg 360
   atatcagctt atgactcaag ttatgagccg tgtgcaaaac atgagataag tttatgacat 420
   catccactga tcgtgcgtta caagtagaat tctactcgta aagccagttc ggttatgagc 480
   cgtgtgcaaa acatgacatc agcttatgac tcatacttga ttgtgtttta cgcgtagaat 540
   tctactcgta aagcgagttc ggttatgagc cgtgtgcaaa acatgacatc agcttatgag 600
   tcataattaa tcgtgcgtta caagtagaat tctactcgta aagcgagttg aaggatcata 660
   tttagttgcg tttatgagat aagattgaaa gcacgtgtaa aatgtttccc gcgcgttggc 720
   acaactattt acaatgcggc caagttataa aagattctaa tctgatatgt tttaaaacac 780
   ctttgcggcc cgagttgttt gcgtacgtga ctagcgaaga agatgtgtgg accgcagaac 840
   agatagtaaa acaaaaccct agtattggag caataatcga tgagctcgtc gacgtaggcc 900
   tttgaattcc gcgcgcttcg gaccgggatc caaaaacatc gattagggtg actgaaggtt 960
   acattggggt aggttatggt taatacgtaa tggtttaaca ccaaaacgat atcatggatt 1020
   ttatataagg tgtaataata tttttaatga gtggacgcgt cgggtcaatg tcctgcctat 1080
   tgacgtcata acatattagg tgattatatt aaaaatagtt taaactcaaa tattacttgc 1140
   aagtttaagt ttcatcataa tctgatcata agtttcaccc aaacagaaac caaaagcata 1200
   actatcgaat atctttagct tcccatgaag aaagattacc gtaaccatca ctaggatttt 1260
   atacgattgt agaaaataaa gtattctcag tctcttttca gagcgctata aaaaggggtg 1320
   cattctcggt aagagtacag ttgaactcac atcgagttaa ctccacgctg cagtctcgag 1380
   atcatggaga taattaaaat gataaccatc tcgcaaataa ataagtattt tactgttttc 1440
   gtaacagttt tgtaataaaa aaacctataa atattccgga ttattcatac cgtcccacca 1500
   tcgggcgc 1508
<210> 40
   <211> 1502
   <212> DNA
   <213> Artificial Sequence
<220>
   <223> Recombinant expression cassette
<400> 40
   atcgatgttg accccaacaa aagatttata attaatcata atcacgaaca acaacaagtc      60
   aatgaaacaa ataaacaagt tgtcgataaa acattcataa atgacacagc aacatacaat     120
   tcttgcataa taaaaattta aatgacatca tatttgagaa taacaaatga cattatccct     180
   cgattgtgtt ttacaagtag aattctaccc gtaaagcgag tttagttttg aaaaacaaat     240
   gacatcattt gtataatgac atcatcccct gattgtgttt tacaagtaga attctatccg     300
   taaagcgagt tcagttttga aaacaaatga gtcataccta aacacgttaa taatcttctg     360
   atatcagctt atgactcaag ttatgagccg tgtgcaaaac atgagataag tttatgacat     420
   catccactga tcgtgcgtta caagtagaat tctactcgta aagccagttc ggttatgagc     480
   cgtgtgcaaa acatgacatc agcttatgac tcatacttga ttgtgtttta cgcgtagaat     540
   tctactcgta aagcgagttc ggttatgagc cgtgtgcaaa acatgacatc agcttatgag     600
   tcataattaa tcgtgcgtta caagtagaat tctactcgta aagcgagttg aaggatcata     660
   tttagttgcg tttatgagat aagattgaaa gcacgtgtaa aatgtttccc gcgcgttggc     720
   acaactattt acaatgcggc caagttataa aagattctaa tctgatatgt tttaaaacac     780
   ctttgcggcc cgagttgttt gcgtacgtga ctagcgaaga agatgtgtgg accgcagaac     840
   agatagtaaa acaaaaccct agtattggag caataatcga tgagctcgtc gacgtaggcc     900
   tttgaattcc gcgcgcttcg gaccgggatc caaaaacatc gattagggtg actgaaggtt     960
   acattggggt aggttatggt taatacgtaa tggtttaaca ccaaaacgat atcatggatt    1020
   ttatataagg tgtaataata tttttaatga gtggacgcgt cgggtcaatg tcctgcctat    1080
   tgacgtcata acatattagg tgattatatt aaaaatagtt taaactcaaa tattacttgc    1140
   aagtttaagt ttcatcataa tctgatcata agtttcaccc aaacagaaac caaaagcata    1200
   actatcgaat atctttagct tcccatgaag aaagattacc gtaaccatca ctaggatttt    1260
   atacgattgt agaaaataaa gtattctcag tctcttttca gagcgctata aaaaggggtg    1320
   cattctcggt aagagtacag ttgaactcac atcgagttaa ctccacgctg cagtctcgag    1380
   atacggacct ttaattcaac ccaacacaat atattatagt taaataagaa ttattatcaa    1440
   atcatttgta tattaattaa aatactatac tgtaaattac attttattta caatcactcg    1500
   ac                                                                   1502

## Description

### FIELD OF THE INVENTION

The present invention may be included in the field of biotechnology and it covers nucleic acid sequences comprising such as promoters, homologous regions (*hr*) as enhancers, and sequences encoding transcriptional regulators, for example, the baculovirus *Ac-ie-01* cDNA, or any combination thereof, which are able to increase the quality and efficiency of recombinant protein production. Moreover, the present invention is also directed to the vectors themselves comprising the above mentioned nucleic acid sequences of the invention, cells infected, transformed or transfected with those sequences or vectors, and methods for producing proteins by using the aforesaid sequences, vectors or cells.

### STATE OF THE ART

The baculovirus expression vector system (BEVS) is a well-established method for the production of recombinant proteins to be used as vaccines, therapeutic molecules or diagnostic reagents. With its potential for over-expression and rapid speed of development, BEVS is one of the most attractive choices for producing recombinant proteins for any purpose. The most employed baculovirus used in industry for recombinant protein expression is based on *Autographa californica* multinuclear polyhedrosis virus (*Ac*MNPV) with *Spodoptera frugiperda* 9 *(Sf9)* or 21 (*Sf*21) insect cells as suitable expression hosts (1), as well as *Trichoplusia ni (T. ni)* insect larvae as living biofactories (2). Since the BEVS was developed in the 80's (3), hundreds of recombinant proteins, ranging from cytosolic enzymes to membrane-bound proteins, have been successfully produced in baculovirus-infected insect cells.

Efforts have been made to increase BEVS productivity (4). A variety of transfer vectors are available for the construction of recombinant baculoviruses, encoding resident fusion proteins, which have been reported to improve protein expression, including maltose binding protein, glutathione S transferase, SUMO and KDEL retention signal. Other attempts related to improve the stability of expressed proteins have been investigated focusing on two genes in the baculovirus genome, which are not essential for growth of the virus in cell culture, namely *chiA* (chitinase) and *cath* (cathepsin). *ChiA* deletion appears to improve the production of secreted proteins by accumulating the protein in the endoplasmic reticulum and processing the proteins through the secretory pathway of the cells. Additionally, the prevention of the formation of cathepsin protease may also contribute to improved product stability from *chiA*-viruses. Novel insect cell lines, such as High-Five™ (Hi-5) or BTI-TnAo38 cell lines from *T. ni*, have recently been developed to increase the baculovirus productivity with significant improvements in the final amount of heterologous protein recovery (5,6).

Accelerating recombinant protein expression, so that protein expression takes place before the machinery of insect cells is severely impaired by the baculovirus infection, would be an important improvement of the BEVS. Late expression, driven by the conventional strong virus promoters of *polyhedrin (polh)* or *p10* genes, has serious disadvantages in the foreign protein post-translational modifications. Baculovirus promoters that allow for earlier expression than the -conventionally used *polh* or *p10* promoters have been characterized and been used for heterologous protein production, but showed a reduced productivity (7).

Another possibility for improving the BEVS would be to increase preservation of cell integrity at late times post-infection by reducing the virus-induced cell death. Reduction in the severe impairment of the insect cell machinery at late times post-infection caused by BEVS should not only increase the time frame for producing and accumulating recombinant proteins (secreted or not), but also allow more time for the folding of complex proteins or any post-translational modification of the produced proteins.

Some baculovirus DNA elements have been determined to be involved in the activation of late expression factor genes, which are necessary for virus propagation. One of them is the immediate early (ie) protein IE-1 and its splice variant IE-0 from *Ac*MNPV *(Autographa californica* multinuclear polyhedrosis virus). Translation of the *Ac*MNPV mRNAs encoded by *Ac-ie-01* gene results in both IE-0 and IE-1 due to internal translation initiation. Both are thought to be critical mediators of baculovirus gene expression due to their potency as transcriptional regulators (8). Synthesized very early during infection, *Ac*MNPV IE-1 is a 67-kDa dimeric DNA-binding protein that stimulates transcription in plasmid transfection assays through the activity of its N-terminal acidic domain (9, 10). IE-1 accumulates within the nucleus, where it is maintained through late times (11). Transactivation by IE-1 is enhanced by its binding as a homodimer to the baculovirus homologous region (*hr*) sequences, which function as transcriptional enhancers and origins of viral DNA replication. *Ac*MNPV IE-0 is a 72.6-kDa 636 amino acid protein composed of 38 amino acids encoded by *orf141 (exon0),* 16 amino acids encoded by the upstream nontranslated leader of *ie1,* and the entire 582 amino acid IE-1 protein. The final product is therefore identical to IE-1 except for the additional 54 amino acids fused to the N-terminus. Presumably due to their common sequences, IE-0 and IE-1 share biochemical activities, including *hr* enhancer binding and transcriptional regulation.

V. J. VALDES et.al: J. BIOL. CHEM. 16.5.2003 278,(21) 9317-24 discloses the use of combination of baculoviral regulatory polynucleotide sequences which drive the expression of major insect proteins (hexamerins) at specific evolution stages of larva from *Trichoplusia ni* for making regulatory regions (tandem promoters) for stronger expression of foreign proteins in insect cells or larvae. pB1 and pB2 drive stronger foreign gene expression in BEVS than the conventional polh promoter and may be combined in tandem with any other regulatory sequence (e.g. pol or p10) resulting in an increased expression levels in Sf21 cells and insect larvae with respect to conventional baculoviruses. pB2 also drives gene expression at earlier times than pol (a great advantage for correct protein folding and post translational modification)

There is a need of novel alternative BEVSs that allow a) stronger expression than the commercial BEVS that use the *polh* or *p10* promoters and b) long-term expression in the baculovirus system by reducing virus-induced cell damage.

### SUMMARY OF THE INVENTION

The present invention provides products and methods for the improved expression of recombinant proteins using the BEVS, as also defined in the claims

The present disclosure provides :
1. Recombinant baculovirus containing an endogenous *Ac-ie-01* gene, comprising
   [i] a further copy of baculoviral *Ac-ie-01* cDNA as transgene under the control of a suitable promoter that allows for the expression above endogenous levels of the proteins IE-1, IE-0 and/or fragments thereof functioning as transcriptional regulators, wherein the nucleic acid is selected from the group consisting of:
      a) nucleic acid containing the nucleotide sequence indicated in any of SEQ ID NO: 1-5;
      b) a nucleic acid sequence having a sequence identity of at least 70%, preferably at least 75%, more preferably at least 80%, more preferably at least 85%, more preferably at least 90% and most preferably at least 95% with the nucleotide sequence indicated in any of SEQ ID NO: 1-5 and encoding a protein able to function as a transcriptional regulator in a recombinant baculovirus;
      c) a nucleic acid sequence encoding an amino acid containing the amino acid sequence indicated in any of SEQ ID NO: 6-9; and
      d) a nucleic acid sequence encoding an amino acid sequence having a sequence similarity of at least 70%, preferably at least 75%, more preferably at least 80%, more preferably at least 85%, more preferably at least 90% and most preferably at least 95% with the amino acid sequence indicated in any of SEQ ID NO: 6-9 and able to function as a transcriptional regulator in a recombinant baculovirus;
   [ii] wherein the recombinant baculovirus further comprises at least one recombinant homologous region (*hr*) as enhancer region, operably linked to any promoter that is suitable for driving the expression of a recombinant protein,
   [iii] wherein the promoter operably linked to the homologous region *(hr)* is selected from the group of nucleic acids comprising:
      a) a nucleic acid containing the nucleotide sequence indicated in any of SEQ ID NO: 10-16; and
      b) a nucleic acid sequence able to function as a promoter in a recombinant baculovirus and having a sequence identity of at least 70%, preferably at least 75%, more preferably at least 80%, more preferably at least 85%, more preferably at least 90% and most preferably at least 95% with the nucleotide sequence indicated in any of SEQ ID NO: 10-16.
2. Recombinant baculovirus according to item 1, wherein the recombinant homologous region (*hr*) is the sequence indicated in SEQ ID NO: 27 (*hr1*).
3. Recombinant baculovirus according to any of the items 1-2, comprising a nucleic acid sequence that comprises combinations of recombinant promoters, sequences encoding transcriptional regulators and enhancer regions selected from the group comprising SEQ ID NO: 17-26.
4. Recombinant baculovirus according to any of the items 1-3, further comprising a nucleic acid sequence encoding a recombinant protein, wherein said nucleic acid sequence is operably linked to a nucleic acid sequence selected from the group consisting of the nucleic acid sequences of items 1-3.
5. Transfer vector suitable for producing a recombinant baculovirus according to any of the items 1-4, comprising said further copy of baculoviral Ac-ie-01 cDNA under the control of a suitable promoter for expression above endogenous levels of the proteins IE-0, IE-1 and/or fragments thereof functioning as transcriptional regulators, further comprising a nucleic acid sequence suitable for integration or transposition in a baculovirus.
6. Transfer vector according to item 5, wherein the recombinant homologous region *(hr)* is the sequence indicated in SEQ ID NO: 27 (*hr1*).
7. Transfer vector according to any of the items 5-6, comprising a nucleic acid sequence that comprises combinations of recombinant promoters, sequences encoding transcriptional regulators and enhancer regions selected from the group comprising SEQ ID NO: 17-26.
8. Transfer vector according to any of the items 5-7, further comprising a nucleic acid sequence encoding a recombinant protein, wherein said nucleic acid sequence is operably linked to a nucleic acid sequence selected from the group consisting of the nucleic acid sequences of items 7-11.
9. Transfer vector according to any of the items 5-8, lacking a nucleic acid sequence encoding a recombinant protein.
10. Transfer vector according to any of the items 5-9, characterized in that the transfer vector is a bacmid.
11. Transfer vector according to any of the items 5-10, characterized in that the transfer vector is derived from any of the baculovirus expression systems "Bac-to-Bac®" (invitrogen™), "BacPAK™" (Clontech™), "FlashBAC™" (Oxford Expression Technologies™), "BacuVance™" (GenScript™), "Bac-N-Blue DNA™" (invitrogen™), "BaculoDirect™" (invitrogen™), "BacVector®" 1000, 2000, 3000 (Novagen®), "DiamondBac™" (Sigma-Aldrich®) or "BaculoGold™" (BD biosciences™).
12. Cloning vector suitable for producing a recombinant baculovirus or transfer vector according to any of the items 1-11, comprising said further copy of baculoviral Ac-ie-01 cDNA under the control of a suitable promoter for expression above endogenous levels of the proteins IE-0, IE-1 and/or fragments thereof functioning as transcriptional regulators, which is further suitable for bacterial replication.
13. Cloning vector according to item 12, wherein the recombinant homologous region (*hr*) is the sequence indicated in SEQ ID NO: 27 *(hr1).*
14. Cloning vector according to any of the items 12-13, comprising a nucleic acid sequence that comprises combinations of recombinant promoters, sequences encoding transcriptional regulators and enhancer regions selected from the group comprising SEQ ID NO: 17-26.
15. Cloning vector according to any of the items 12-14, further comprising a nucleic acid sequence encoding a recombinant protein, wherein said nucleic acid sequence is operably linked to a nucleic acid sequence selected from the group consisting of the nucleic acid sequences of items 16-20.
16. Cloning vector according to any of the items 12-14, lacking a nucleic acid sequence encoding a recombinant protein.
17. Nucleic acid sequence suitable for producing a recombinant baculovirus, transfer vector or cloning vector according to any of the items 1-16, comprising said further copy of baculoviral Ac-ie-01 cDNA under the control of a suitable promoter for expression above endogenous levels of the proteins IE-0, IE-1 and/or fragments thereof functioning as transcriptional regulators.
18. Nucleic acid sequence according to item 17, wherein the recombinant homologous region (*hr*) is the sequence indicated in SEQ ID NO: 27 *(hr1).*
19. Nucleic acid sequence according to any of the items 17-18, comprising a nucleic acid sequence that comprises combinations of recombinant promoters, sequences encoding transcriptional regulators and enhancer regions selected from the group comprising SEQ ID NO: 17-26.
20. Nucleic acid sequence according to any of the items 17-19, further comprising a nucleic acid sequence encoding a recombinant protein, wherein said nucleic acid sequence is operably linked to a nucleic acid sequence selected from the group consisting of the nucleic acid sequences of items 23-27.
21. Nucleic acid sequence according to any of the items 17-19, lacking a nucleic acid sequence encoding a recombinant protein.
22. A host cell infected, transfected, transduced or transformed with the recombinant baculovirus, transfer vector, cloning vector or nucleic acid sequence of any of the items 1-21.
23. Infected, transfected, transduced or transformed host cell according to item 22, characterized in that it is an insect cell line.
24. Infected, transfected, transduced or transformed host cell according to item 22 or 23, characterized in that it is derived from an insect belonging to the Lepidoptera or Diptera genus.
25. Infected, transfected, transduced or transformed host cell according to any of the items 22-24, characterized in that it is derived from *Trichoplusia ni, Spodoptera frugiperda, Ascalapha odorata, Bombyx mori, Drosophila melanogaster* or *Aedes aegypti.*
26. Infected, transfected, transduced or transformed host cell according to any of the items 22-25, characterized in that it is a cell line selected from the group consisting of Hi-5™, *Sf9, Sf*21*,* BTI-Tn5B-1, Tn368, ExpresSf+®, BTI-TnAo38, ATC-10 and Schneider's *Drosophila* Line 2.
27. Culture medium comprising the recombinant baculovirus, transfer vector, cloning vector or nucleic acid sequence of any of the items 1-21.
28. Method for producing a recombinant protein that comprises the infected, transfected, transduced or transformed host cell according to items 22-26 and the extraction and purification of the recombinant protein by conventional means.
29. Method for producing a recombinant protein according to item 28, wherein the recombinant protein is selected from the group comprising subunit monomeric vaccine, subunit multimeric vaccine, virus like particle, therapeutic protein, antibody, enzyme, cytokine, blood clotting factor, anticoagulant, receptor, hormone and diagnostic protein reagent.
30. Use of the transfer vector according to any of the items 5-11 for producing a recombinant baculovirus according to any of the items 1-4.
31. Use of the cloning vector according to any of the items 12-16 for producing a recombinant baculovirus or transfer vector according to any of the items 1-11.
32. Use of the nucleic acid sequence according to any of the items 17-21 for producing a recombinant baculovirus, transfer vector or cloning vector according to any of the items 1-16.

### BRIEF DESCRIPTION OF THE FIGURES

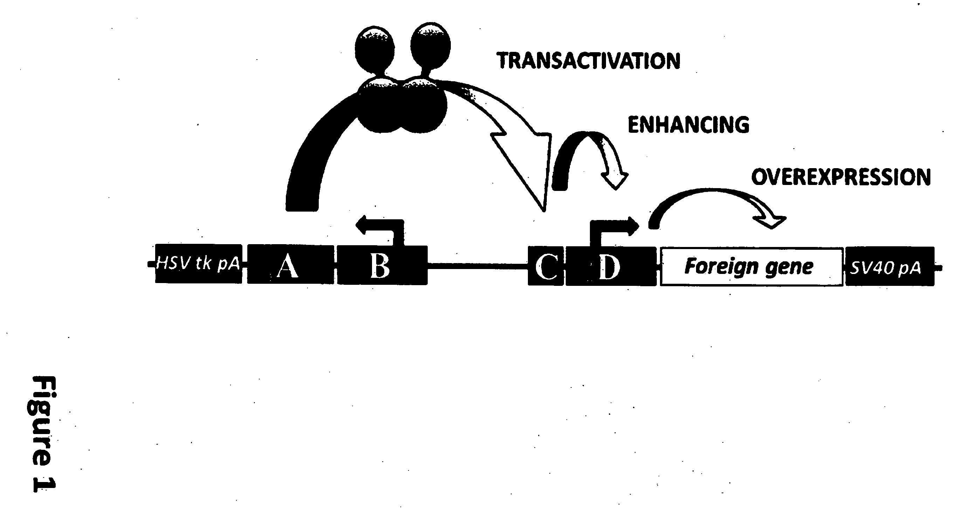

**Figure 1****:** Schematic representation of the baculovirus recombinant DNA elements of the invention, comprising four principal elements: a sequence encoding for transcriptional regulators (**A**; e.g. IE0 and IE1), which expression is driven by a promoter (**B**; e.g. *polh or pB2₉);* an enhancer homologous region (*hr*) sequence (**C**; e.g. *hr1*), upstream of the promoters (**D**; e.g. *p10, polh, pB2₉p10* or *p6.9p10)* driving the expression of the foreign gene coding for the recombinant protein. The scheme shows the theoretical mechanism of interaction between the recombinant DNA elements of the present invention that results in the unprecedented overexpression of the recombinant protein.

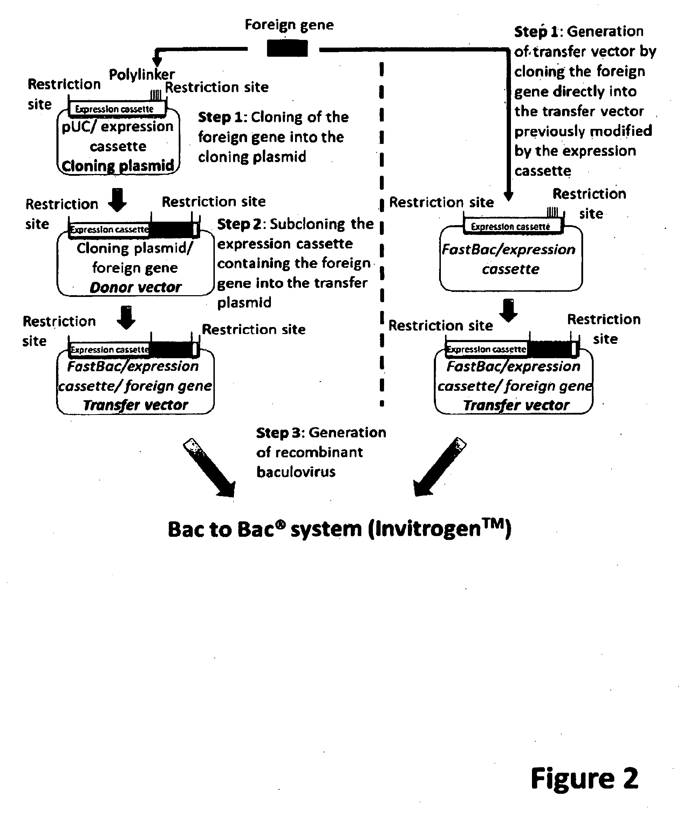

**Figure 2****:** Different strategies that result in the generation of recombinant baculoviruses by the "Bac-to-Bac^{®}" cloning system (invitrogen™).

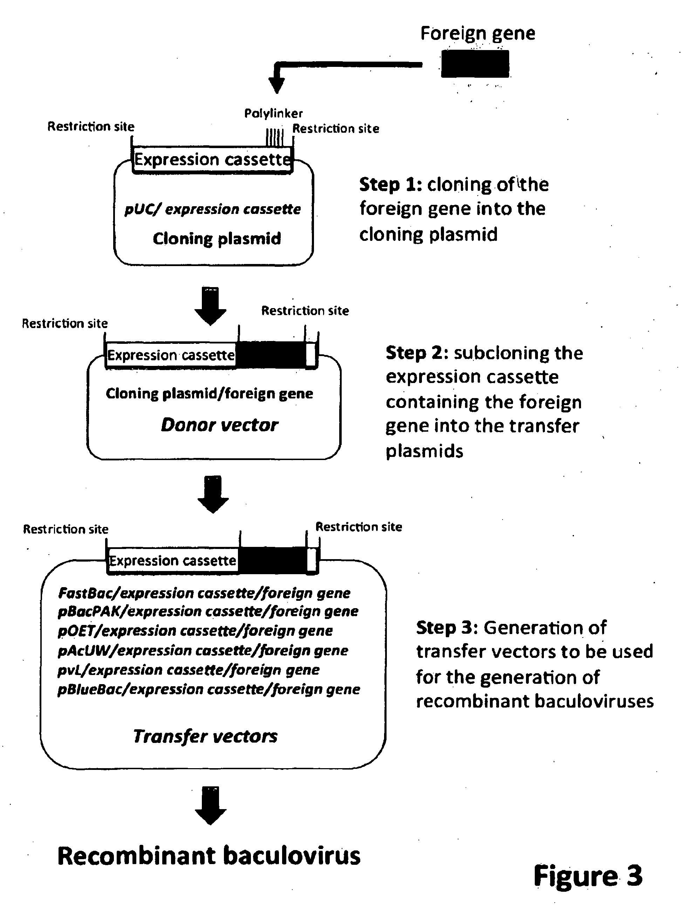

**Figure 3****:** General scheme for the generation of cloning, donor and transfer vectors compatible with other commercial technologies used to generate recombinant baculoviruses.

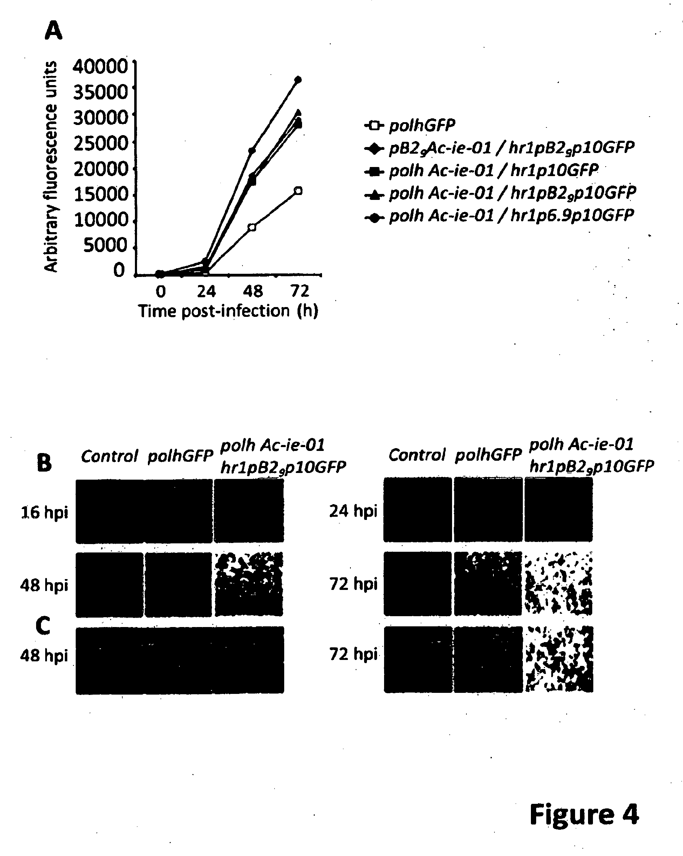

**Figure 4: A)** Fluorimetric assays for the measurement of the increment in GFP protein accumulated in infected *Sf*21 cells at different times post-infection when expressed in the baculovirus expression cassettes of the invention, containing *hr1p10, hr1pB2₉p10* or *hr1p6.9p10* and the *Ac-ie-01* cDNA expressed under the control of *pB2₉* or *polh* promoters. All GFP expression levels were compared to that obtained with the *polh* promoter in a conventional baculovirus vector. The graph represents the mean values of three independent expression experiments for each baculovirus with standard deviations lower than 5 % in each case. This figure also shows representative fluorescence micrographs showing *Sf*21 cells at different times post-infection with a wild-type baculovirus (control), with a conventional baculovirus expressing the GFP under the control of *polh* promoter or with a baculovirus expressing the GFP by the expression cassette of the invention *polhAc-ie-01*/*hrZpB2₉p10GFP.* Cells were infected in **A)** and **B)** at a multiplicity of infection (MOI) of 5 or in **C)** at a MOI of 0.1.

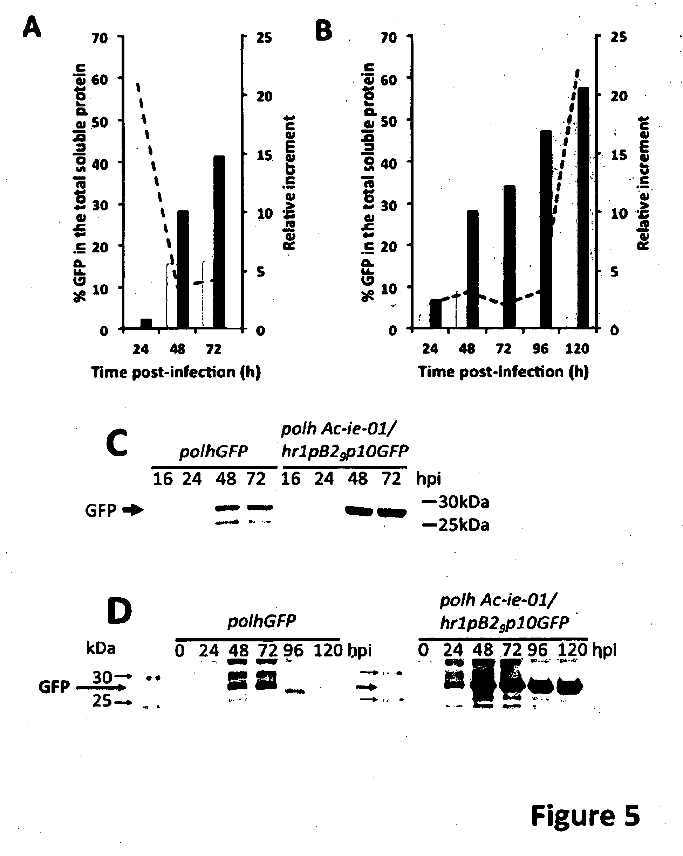

**Figure 5: A)** Comparison of the amounts of recombinant GFP protein in *Sf*21 insect cells grown in monolayer at different times post-infection expressed by a conventional baculovirus vector under the control of a *polh* promoter (light grey) or by the baculovirus vector engineered with an expression cassette of the invention, composed of the elements *polhAc-ie-01*/*hr1pB2₉p10GFP* (dark grey) and measured by microfluidic protein analysis (Experion™; BioRad™, USA). Cells were infected at a MOI of 5 with both viruses; **B)** Comparison of recombinant GFP protein accumulated in *Sf*9 insect cells grown in suspension at different times post-infection expressed by a conventional baculovirus vector under the control of a *polh* promoter (light grey) or by the baculovirus vector engineered with an expression cassette of the invention, composed of the elements *polhAc-ie-01*/*hr1p6.p10GFP* (dark grey) and measured by microfluidic protein analysis (Experion™; BioRad™, USA). Cells were infected at a MOI of 0.1 with both viruses; Discontinued lines indicate the percentage of increment of recombinant GFP produced by the baculoviruses containing the expression cassette of the invention in comparison to that obtained with the conventional baculovirus expressing the GFP under the control of the *polh* promoter; **C)** Coomassie blue staining of SDS-PAGE gels resolving the infected cell extracts of the experiment described in panel A; **D)** Coomassie blue staining of SDS-PAGE gels resolving the infected cell extracts of the experiment described in panel B.

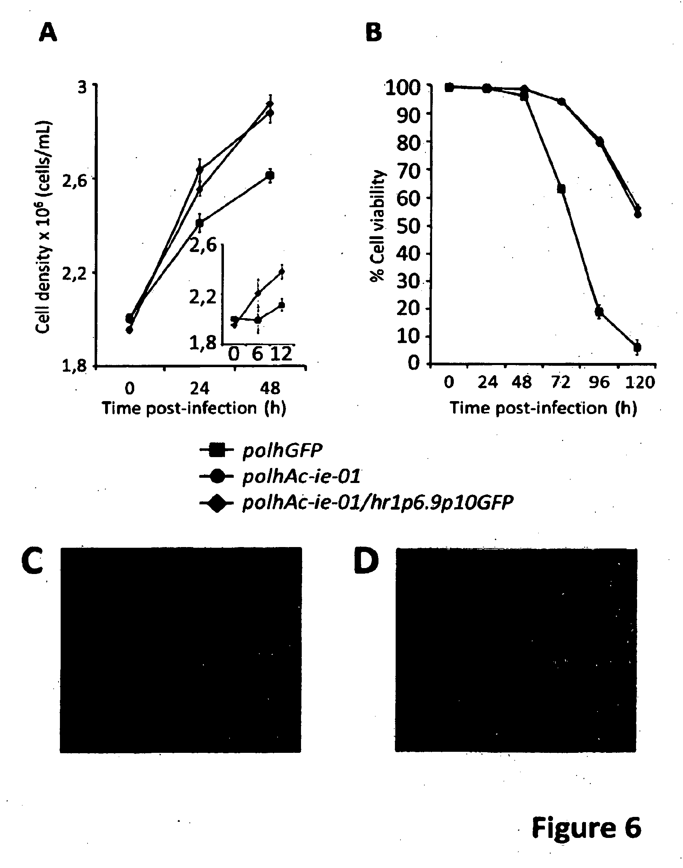

**Figure 6****.** *Sf*9 insect cells were cultured in suspension and infected by a baculovirus overexpressing the *Ac-ie-01* cDNA under the control of *polh* or by a baculovirus expressing a reporter protein GFP in the context of the baculovirus expression cassette *polhAc-ie-01*/*hr1p6.9p10GFP* of the present invention to assess the cell density **(A)** and viability **(B)** of these cells. As a control, a conventional baculovirus expressing the GFP protein under the control of *polh* was used. Cells were infected in suspension at a MOI of 0.1. **(A)** The cells were counted at different times post-infection (0, 24 and 48 hours) to calculate the cell density. A more detailed analysis of the precise moment in which cell proliferation is produced by the overexpression of the *Ac-ie-01* cDNA is shown in the insert for cells infected with *polhGFP* or *polhAc-ie-01*/*hr1p6.9p10GFP.* **(B)** Cell viability was assessed by Trypan blue staining (dilution 1:1 of suspended cells and colorant at 0.4 % in PBS buffer). This staining allows the differentiation between live and death cells. Cell viability was calculated by the percentage of living cells with respect to the total number of cells at different times post-infection (from 0 to 120 hours). Micrographs of Hi-5™ insect cell monolayers infected at a MOI of 5 with a control conventional baculovirus overexpressing the reporter protein GFP under the *polh* promoter **(C)** or with a baculovirus overexpressing the *Ac-ie-01* cDNA under the control of the *polh* promoter **(D).** Micrographs were obtained at 96 hours post-infection at a 20X magnification in an inverted microscope Leica™ DMIL™.

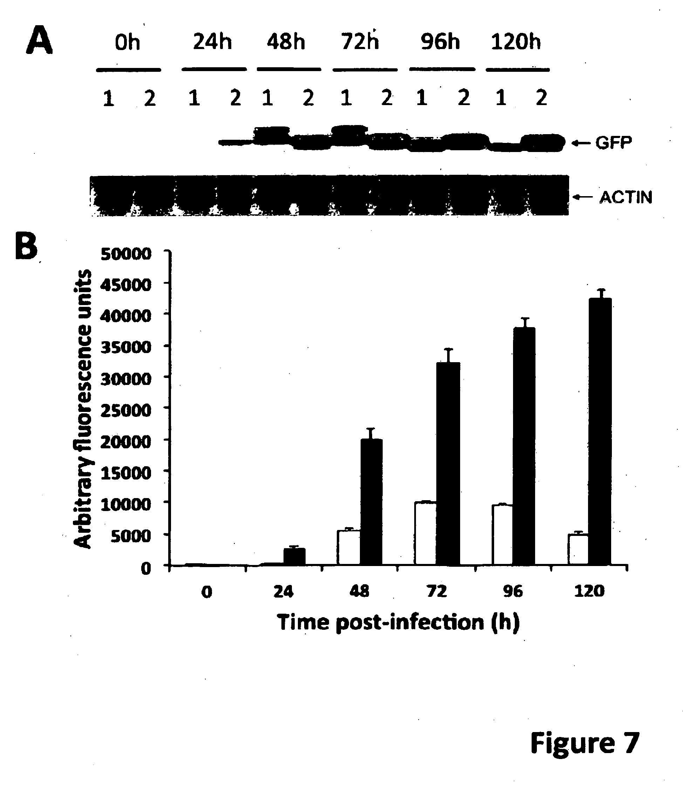

**Figure 7: A)** *Sf*9 insect cells were infected by a conventional baculovirus expressing the GFP protein under the control of the *polh* promoter (1) or by a baculovirus vector engineered with the expression cassette of the invention containing the elements *polhAc-ie-01*/*hr1p6.9p10GFP,* overexpressing the transcriptional regulators encoded by *Ac-ie-01* cDNA (2). Cells were sampled at different times post-infection (0 to 120 hours) and cell extracts analysed by SDS-PAGE and Western blot with an antiserum against GFP or against the cellular actin protein. **B)** The functionality of the GFP protein expressed in *Sf*9 insect cells as analyzed in panel **A** was measured by fluorimetry. The fluorescence values obtained at different hours post-infection with the GFP protein produced by a conventional recombinant baculovirus (grey bars) was compared to that produced by the recombinant baculovirus with the baculovirus cassette of the present invention (black bars).

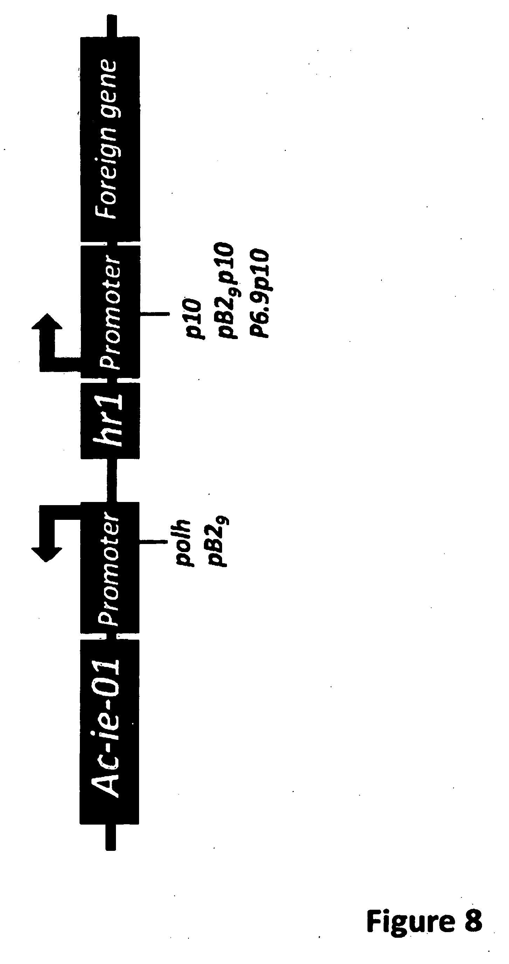

**Figure 8****:** Schematic representation of the preferred elements contained in the baculovirus expression cassettes of the invention, comprising encoding sequences for transcriptional regulators, homologous regions *(hr)* enhancing the transcription from promoter(s) of a foreign gene encoding a recombinant protein.

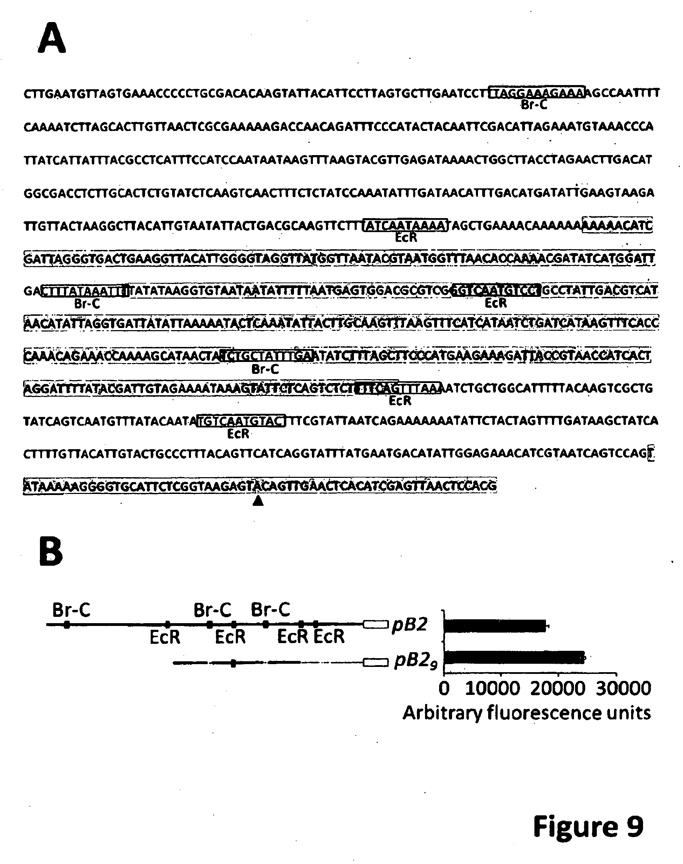

**Figure 9****:** Sequence analysis of the *pB2* promoter isolated from *T. ni* and determination of the transcriptional regulatory element *pB*2₉ sequence. **A)** Nucleotide sequence of the *pB2* promoter region of BJHSP2 (Genbank accession no **U41640)** isolated from *T. ni.* The previously described transcription start site is indicated by a triangle, whereas the TATA box is underlined and the potential cis-acting elements are enclosed in boxes in the *pB2* sequence. Shaded nucleotide residues indicate the sequence incorporated into the *pB2₉* promoter. The predicted Br-C and EcR putative binding sites are also indicated in the figure by transparent boxes **B)** Schematic illustration of the *pB2* DNA fragment and its derivative promoter *pB2₉.* Their promoter activity was analyzed by fluorimetric analysis using the GFP protein as the reporter gene. The GFP expression was quantified 72 hours post- infection in all experiments and is indicated as the arithmetic media with standard deviations of three independent experiments.

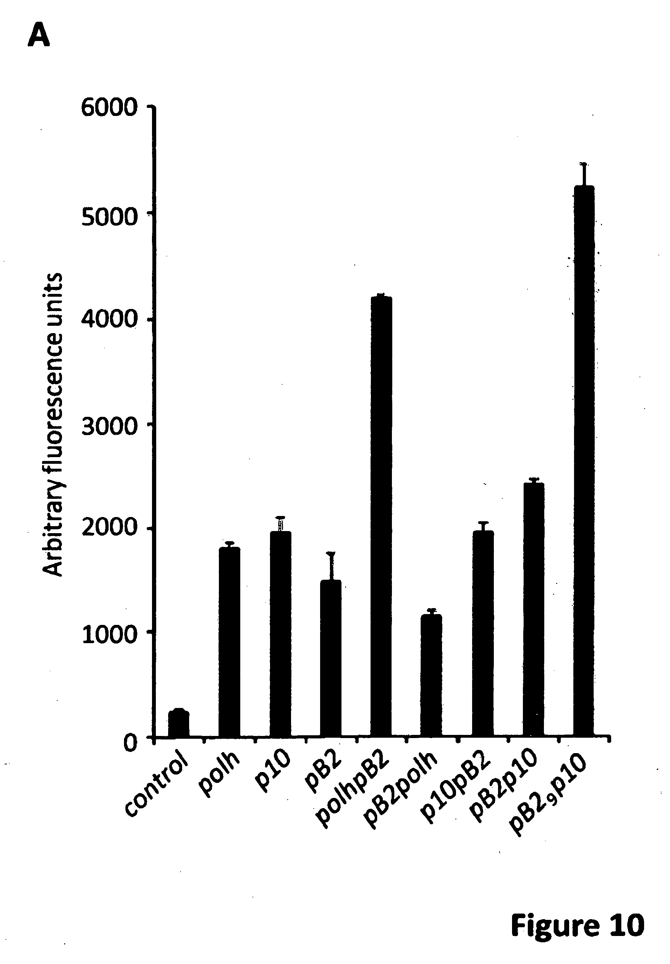

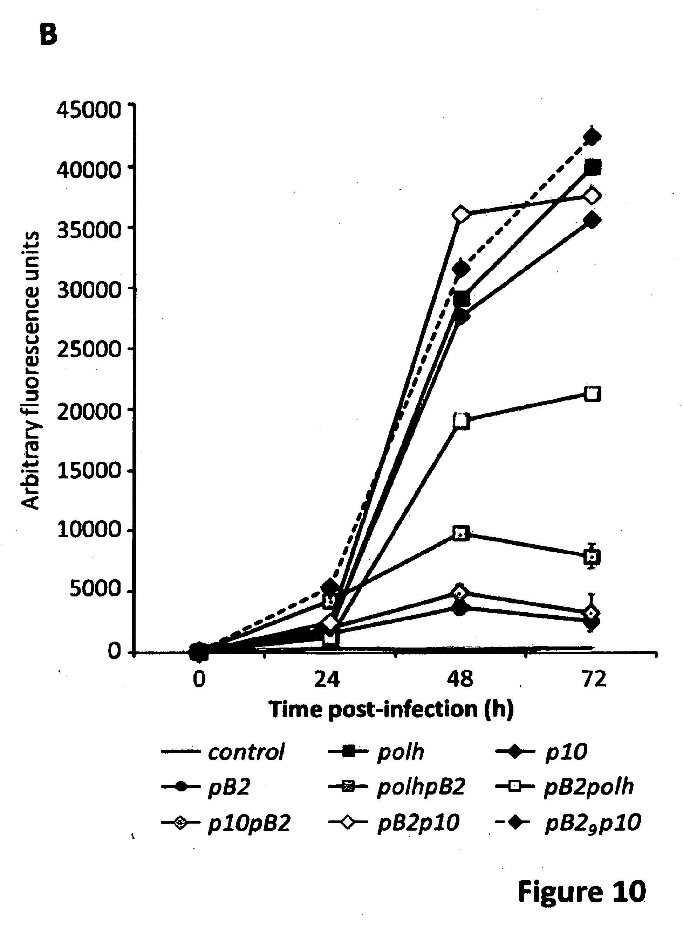

**Figure 10****:** GFP expression levels mediated by the use of different promoters or combination of promoters. **A)** Fluorimetric analysis at 24 hours post-infection of *Sf*21 cells infected with different recombinant baculoviruses expressing the GFP under the control of different individual or chimeric promoters. **B)** Time course study of the GFP expression in *Sf*21 cells infected with the same recombinant baculoviruses as in panel A, measured by a fluorimetric assay. All the experiments were done at a MOI of 5 and the figure shows the arithmetic media of three independent experiments with the corresponding standard deviations.

### DETAILED DESCRIPTION OF THE INVENTION

The present invention improves the expression of recombinant proteins in the BEVS by means of the introduction of recombinant DNA elements into baculoviruses.

The recombinant DNA elements of the present disclosure are sequences that cause the expression of baculovirus transcriptional regulators above endogenous levels and optionally enhancer homologous regions (*hr*) and promoters operably linked to these aforementioned elements.

Furthermore, the recombinant DNA elements may form part of an expression cassette.

"Expression cassette" refers to a nucleic acid sequence that contains recombinant DNA elements, which control (e.g. the promoter) and/or are required (e.g. the gene itself) for gene expression. The expression cassette can be introduced in a recombinant vector or baculovirus.

The recombinant DNA elements may be incorporated in a single nucleic acid sequence, cloning vector, transfer vector, recombinant baculovirus or cell. However, they can also be present in different nucleic acid sequences, cloning vectors, transfer vectors or recombinant baculoviruses and be introduced into the same cell.

The present invention surprisingly shows that introduction of sequences that cause the expression of baculovirus transcriptional regulators above endogenous levels and the introduction of an enhancer homologous region (*hr*) sequence, a promoter or a combination of promoters is able to increase the production of a recombinant protein to unprecedented levels from early (6 to 8 hours post-infection) to late (48 to 96-120 hours post-infection) times post-infection.

Additionally, the introduction of these recombinant DNA elements also increases the proliferation of baculovirus-infected cells (particularly at early times post-infection), the viability at late times post-infection and the integrity of the molecular cell machinery and morphology of said baculovirus-infected cells. An improvement in the integrity of cell functions during baculovirus infections also contributes to the correct post-translational processing of the recombinant protein.

Introduction of these recombinant DNA elements also increases the recombinant protein production in host cells compared to the conventional *polh* or *p10* promoters.

Thus, one aspect of the invention relates to a recombinant baculovirus that contains a further copy of baculoviral Ac-ie-01 cDNA under the control of a suitable promoter that allows for the expression above endogenous levels of transcriptional regulators. The transcriptional regulators are IE-1, IE-0 and/or fragments thereof.

"Baculovirus" refers to a family of infectious viruses for invertebrates, mainly infecting insects and arthropods. A "recombinant baculovirus" has further introduced recombinant DNA through, for example, homologous recombination or transposition. The recombinant baculovirus preferably originates from *Ac*MNPV.

"Recombinant DNA" refers to a form of artificial DNA that is engineered through the combination or insertion of one or more DNA strands, thereby combining DNA that would normally not occur together.

"Recombinant DNA element" refers to a functional element within recombinant DNA, such as a promoter, enhancer or a gene. As mentioned above, the recombinant DNA elements of the present invention are sequences that cause the expression of baculovirus transcriptional regulators above endogenous levels, enhancer homologous regions (*hr*) and promoters operably linked to these aforementioned elements.

"Transcriptional regulator" refers to a regulatory protein that has the ability to modulate the transcription of specific genes by, for example, binding to enhancer or repressor regions and/or recruiting further proteins that are involved in transcription.

IE-1 and its splice variant IE-0 are transcriptional regulators that are endogenously expressed during baculovirus infection. According to the present invention, IE-1, IE-0 and/or fragments thereof are recombinantly expressed to increase the total level of these proteins above endogenous levels. This is achieved through introducing further copies of the endogenous gene. Further, copies of the endogenous genes can be introduced as transgenes under the control of a suitable promoter such as *polh* or *pB2₉.*

The expression level of the proteins IE-1, IE-0 and/or fragments thereof can be determined at both the mRNA and at the protein level with methods conventionally known to the person skilled in the art, such as quantitative PCR and Western Blot analysis.

According to the invention, IE-1, IE-0 and fragments thereof are encoded by the nucleic acids of SEQ ID NO: 1 (also referred to as *Ac-ie-01)* to SEQ ID NO: 5. SEQ ID NO: 1 is the *Ac-ie-01* cDNA that encodes both IE-1 and IE-0, SEQ ID NO: 2 is the coding sequence (CDS) of IE-1 and SEQ ID NO: 3 is the CDS of IE-0. SEQ ID NO: 4 and 5 are the CDSs of the N-terminal domains of IE-1 and IE-0 respectively that retain the catalytic transcriptional regulator activity. The proteins that are encoded by SEQ ID NO: 2-5 are represented by SEQ ID NO: 6-9 respectively.

The present disclosure furthermore provides variants of SEQ ID NO: 1-9 that are or encode amino acids that are able to function as a transcriptional regulator. These variants are nucleic or amino acids whose nucleotide or amino acid sequence differs in one or more positions from these parental nucleic or amino acids, whereby differences might be additions, deletions and/or substitutions of nucleotides or amino acid residues.

Nucleic and amino acid sequences of the present disclosure shall be distinguished from other nucleic and amino acid sequences by their degree of sequence identity or similarity respectively as determined using EMBOSS Needle with the default parameters (http://www.ebi.ac.uk/Tools/psa/emboss_needle/). Methods for the generation of such variants include random or site directed mutagenesis, site-saturation mutagenesis, PCR-based fragment assembly, DNA shuffling, homologous recombination *in vitro* or *in vivo,* and methods of gene-synthesis.

The sequence of the variants of SEQ ID NO: 1-5 is at least 70%, preferably at least 75%, more preferably at least 80%, more preferably at least 85%, more preferably at least 90% and most preferably at least 95% identical to the sequences of SEQ ID NO: 1-5.

The sequence of the variants of SEQ ID NO: 6-9 is at least 70%, preferably at least 75%, more preferably at least 80%, more preferably at least 85%, more preferably at least 90% and most preferably at least 95% similar to the sequences of SEQ ID NO: 6-9.

The recombinant baculovirus of the present invention further contains, in addition to the nucleic acid sequence that allows for the expression above endogenous levels of the proteins IE-1, IE-0 and/or fragments thereof, a recombinant homologous region *(hr)* that can enhance the expression of a recombinant protein by being operably linked to the respective promoter.

Homologous regions, *hr*, are comprised of repeated units of about 70-bp with an imperfect 30-bp palindrome near their center. Homologous regions are repeated at eight locations in the *Ac*MNPV genome with 2 to 8 repeats at each side. Homologous regions have been implicated as both transcriptional enhancers and origins of baculovirus DNA replication.

"Enhancer region" refers to a control sequence, whose binding by transcriptional regulators increases the level of transcription of associated genes.

"Recombinant protein" refers to a protein that originates from recombinant DNA. Such proteins can be used for the benefit of humans and animals and may have industrial, commercial or therapeutic application.

"Being operably linked" refers to two nucleic acid sequences that are connected in a way that one influences the other in terms of, for example, transcriptional regulation.

"Promoter" refers to a DNA sequence to which RNA polymerase can bind to initiate transcription. The sequence may further contain binding sites for various proteins that regulate transcription, such as transcription factors. The promoter sequence may be composed of different promoter fragments (either different or the same fragments) that are localized closely in the DNA sequence and may be separated by linkers or spacer. Such promoters are referred to as chimeric promoters.

The enhancer homologous region sequence hr upstream of the promoter/s is preferably *hr1* (SEQ ID NO: 27). The promoters that drive the expression of the recombinant protein are selected from the group comprising SEQ ID NO: 10-16 or a sequence that is able to function as a promoter and has at least 70%, preferably at least 75%, more preferably at least 80%, more preferably at least 85%, more preferably at least 90% and most preferably at least 95% identity with the nucleotide sequence indicated in any of SEQ ID NO: 10-16.

In a preferred embodiment, the nucleic acid sequence comprises combinations of recombinant promoters, sequences encoding transcriptional regulators and enhancer regions selected from the group comprising SEQ ID NO: 17-26.

As indicated above, the recombinant promoters, sequences encoding transcriptional regulators and enhancer regions of the present invention do not need to form part of a single molecule, instead these sequences may form part of distinct molecules as long as they are operably linked, i.e. contained within the same cells.

The recombinant baculovirus of the present invention preferably comprises a nucleic acid sequence encoding a recombinant protein. This nucleic acid sequence is operably linked to the nucleic acid sequence that allows for the expression above endogenous levels of the proteins IE-1, IE-0 and/or fragments thereof and to a homologous region *(hr).*

In one embodiment, the present invention discloses a host cell that is infected, transfected, transduced or transformed with the recombinant baculovirus, transfer vector, cloning vector or nucleic acid sequence of the present invention. Preferably, the host cell is kept in cell culture. The host cell is preferably an insect cell line, more preferably a cell line derived from an insect belonging to the Lepidoptera or Diptera genus, more preferably the host cell is derived from the group consisting of *Trichoplusia ni, Spodoptera frugiperda, Ascalapha odorata, Bombyx mori, Drosophila melanogaster* and *Aedes aegypti* and most preferably it is selected from the group of insect cell lines consisting of Hi-5™, *Sf*9, *Sf*21, BTI-Tn5B-1, Tn368, ExpresSf+®, BTI-TnAo38, ATC-10 and Schneider's *Drosophila* Line 2. The host cell may be cultured in monolayer or in suspension.

In a further aspect the invention discloses methods for producing a recombinant protein using the host cell of the invention. After expression of the recombinant protein, extraction and purification of the recombinant protein is done by conventional means.

In a preferred aspect the host cells are cultured in suspension (bioreactors), at densities between 2x10⁶ to 8x10⁶ cells per ml, depending on the cell line and the fermentation procedure used. Furthermore, cells are infected at a MOI of 0.1 to 1 for protein production.

The recombinant protein that is preferably produced by the methods of the present invention is a protein selected from the group comprising subunit monomeric vaccine, subunit multimeric vaccine, virus like particle, therapeutic protein, antibody, enzyme, cytokine, blood clotting factor, anticoagulant, receptor, hormone and diagnostic protein reagent.

One aspect of the disclosure relates to the use of the recombinant baculovirus, transfer vector, cloning vector or nucleic acid sequence of the present invention in a culture medium. In a preferred aspect the culture medium comprises a baculovirus of the present invention.

The present invention discloses a transfer vector that can be used to produce the recombinant baculovirus of the present invention and comprises said sequence for expression above endogenous levels of the proteins IE-0, IE-1 and/or fragments thereof, in addition to a sequence suitable for integration or transposition in a baculovirus.

Transfer vectors generally permit the insertion of genetic information into a baculovirus.

The transfer vector contains in addition to (i) the further copy of baculoviral Ac-ie-01 cDNA under the control of a suitable promoter for expression above endogenous levels of the proteins IE-0, IE-1 and/or fragments thereof, (ii) a recombinant homologous region (*hr*) linked to (iii) a suitable promoter for driving the expression of a recombinant protein. The preferred combinations of these recombinant DNA elements are as described above for the recombinant baculovirus.

In one preferred aspect, the transfer vector comprises a nucleic acid sequence encoding said recombinant protein, whereas in another preferred aspect the transfer vector lacks such sequence.

In a preferred aspect the transfer vector is a bacmid.

*"Bacmid"* refers to a plasmid construct which contains the DNA sequence sufficient for generating a baculovirus when transfected into a cell.

In a further preferred aspect, the transfer vector is derived from any of the commercially available baculovirus expression systems "Bac-to-Bac^{®}" (invitrogen™), "BacPAK™" (Clontech™), "FlashBAC™" (Oxford Expression Technologies™), "BacuVance™" (GenScript™), "Bac-N-Blue DNA™" (invitrogen™), "BaculoDirect™" (invitrogen™), "BacVector^{®}" 1000, 2000, 3000 (Novagen^{®}), "DiamondBac™" (Sigma-Aldrich^{®}) or "BaculoGold™" (BD biosciences™).

The present invention discloses a cloning vector that can be used to produce the recombinant baculovirus and/or transfer vector of the present invention and comprises said further copy of baculoviral Ac-ie-01 cDNA under the control of a suitable promoter for expression above endogenous levels of the proteins IE-0, IE-1 and/or fragments thereof, which is further suitable for bacterial replication.

"Cloning vector" refers to any vector that is suitable for cloning, which generally involves the presence of restriction sites, an origin of replication for bacterial propagation and a selectable marker.

The cloning vector contains in addition to (i) the further copy of baculoviral Ac-ie-01 cDNA under the control of a suitable promoter for expression above endogenous levels of the proteins IE-0, IE-1 and/or fragments thereof, (ii) a recombinant homologous region (*hr*) linked to (iii) a suitable promoter for driving the expression of a recombinant protein. The preferred combinations of these recombinant DNA elements are as described above for the recombinant baculovirus.

In one preferred aspect, the cloning vector comprises a nucleic acid sequence encoding said recombinant protein (also referred to as the "donor vector"), whereas in another preferred aspect the cloning vector lacks such sequence.

The present invention discloses a nucleic acid sequence that can be used to produce the recombinant baculovirus, transfer vector and/or cloning vector of the present invention and comprises said further copy of baculoviral Ac-ie-01 cDNA under the control of a suitable promoter for expression above endogenous levels of the proteins IE-0, IE-1 and/or fragments thereof.

The nucleic acid sequence contains in addition to (i) the further copy of baculoviral Ac-ie-01 cDNA under the control of a suitable promoter for expression above endogenous levels of the proteins IE-0, IE-1 and/or fragments thereof, (ii) a recombinant homologous region *(hr)* linked to (iii) a suitable promoter for driving the expression of a recombinant protein. The preferred combinations of these recombinant DNA elements are as described above for the recombinant baculovirus.

In one preferred aspect, the nucleic acid sequence comprises a nucleic acid sequence encoding said recombinant protein, whereas in another preferred aspect the nucleic add sequence lacks such sequence.

The transient expression of the *Ac-ie-01* cDNA by a baculovirus system surprisingly confers unique properties of virus resistance and cell proliferation to the cells. This suggests that the overexpression of the transcriptional regulators encoded by this gene activates other virus or cellular genes which may be responsible for such interesting biotechnology applications related to the productivity increase and resistance to different cellular stresses. A potential application derived from this study could be the generation of insect, avian or mammalian transgenic cells which may represent highly productive cell lines to be used for recombinant protein production or virus propagation with potential use in the production of conventional vaccines based on attenuated or inactivated viruses.Hence, one aspect of the disclosure relates to a transgenic cell line that carries as a transgene the sequences of SEQ ID NO: 1-5 or variants thereof or sequences that encode the proteins of SEQ ID NO: 6-9 or variants thereof as defined above. The transgenic cell line is preferably of mammalian, avian or insect origin. In a further embodiment, the transgenic cell line of the invention can be used for the production of a recombinant protein, which is extracted and purified by conventional means. "Transgenic cell line" refers to a cell line that contains a gene that was transferred into the cell.

Based on the surprising finding that the recombinant baculovirus promoter *pB2₉* (SEQ ID NO: 14) and the chimeric recombinant baculovirus promoters *pB2₉p10* (SEQ ID NO: 12) and *pB2p10* (SEQ ID NO: 15) allow an improved expression as compared to conventional promoters, such as *pB2,* a further aspect of the present disclosure relates to nucleic acid sequences comprising such sequence or sequence variants of SEQ ID NO: 12, 14 or 15 that are at least 70%, preferably at least 75%, more preferably at least 80%, more preferably at least 85%, more preferably at least 90% and most preferably at least 95% identical to the sequences of SEQ ID NO: 12, 14 or 15.

**SUMMARY OF SEQUENCES**

| **SEQ ID NO:** | **Name:** |
|---|---|
| 1 | Complete *Ac-ie-01* cDNA |
| 2 | Protein coding sequence (CDS) of IE-1 |
| 3 | CDS of IE-0 |
| 4 | CDS of the IE-1 N-terminal domain |
| 5 | CDS of the IE-0 N-terminal domain |
| 6 | IE-1 protein |
| 7 | IE-0 protein |
| 8 | IE-1 N-terminal domain protein |
| 9 | IE-0 N-terminal domain protein |
| 10 | *polh* (promoter) |
| 11 | *p10* (promoter) |
| 12 | *pB2₉p10* (promoter) |
| 13 | *p6.9p10* (promoter) |
| 14 | *pB2₉* (promoter) |
| 15 | *pB2p10* (promoter) |
| 16 | *polhp10* (promoter) |
| 17 | *polhAc-ie-01*/*hr1p10* |
| 18 | *polhAc-ie-01*/*hr1pB2₉p10* |
| 19 | *polhAc-ie-01*/*hr2p6.9p10* |
| 20 | *pB2₉Ac-ie-01*/*hr1p10* |
| 21 | *pB2₉Ac-ie-01*/*hr1pB2₉p10* |
| 22 | *pB2₉Ac-ie-01*/*hr1p6.9p10* |
| 23 | *polhAc-ie-01*/*hr1polh* |
| 24 | *pB2₉Ac-ie-01*/*hr1polh* |
| 25 | *polhAc-ie-01*/*hrI polhp10* |
| 26 | *pB2₉Ac-ie-01*/*hr1polhp10* |
| 27 | *Homologous region enhancer hr1* |
| 28 | *polhAc-ie-01* |
| 29 | *polhGFP* |
| 30 | *p10pB2* (promoter) |
| 31 | *polhpB2* (promoter) |
| 32 | *pB2polh* (promoter) |
| 33 | *pB2₉polh* (promoter) |
| 34 | *pB2* (promoter) |
| 35 | *hr1polhpB2* |
| 36 | *hr1pB2polh* |
| 37 | *hr1p10pB2* |
| 38 | *hr1pB2p10* |
| 39 | *hr1pB2₉polh* |
| 40 | *hr1pB2₉p10* |

### DEPOSITION OF MICROORGANISMS ACCORDING TO THE BUDAPEST TREATY

Plasmids were deposited in the Spanish Type Culture Collection (CECT) (www.cect.org); University of Valencia, Parc Científjc Universitat de València; Catedrático Agustín Escardino, 9; 46980 Paterna (Valencia), Spain, with the accession number CECT 8031, on the date October 4, 2011.

### EXAMPLES

### Example 1. Overexpression of baculovirus transcriptional regulators potentiates the enhancer function of a homologous region hr functionally linked to a promoter increasing the expression of a recombinant protein in a baculovirus vector expression system (BEVS).

Immediate early viral proteins encoded by the *Ac-ie-01* cDNA, i.e. IE-1 and IE-0, from *Ac*MNPV are potent transcriptional regulators in the baculovirus. Transactivation mediated by these proteins is enhanced by their binding as a homodimer to the baculovirus homologous region (*hr*) sequences, which act as transcriptional enhancers. *Ac*MNPV IE-1/IE-0 are 67-72 kDa dimeric DNA-binding proteins that stimulate transcription in plasmid transfection assays through the activity of its N-terminal acidic domain (7, 8). Synthesized very early during infection, IE-1 and IE-0 accumulate within the nucleus, where they are maintained through late times. Using the dual plasmid pFastBac™ (invitrogen™), the *Ac-ie-01* cDNA was cloned under the control of the *polh* promoter. In the same plasmid, but in another locus, the GFP encoding gene was cloned downstream of the *hr1pB2₉p10* chimeric promoter that was previously synthesized and contains the homologous region *hr1* fused to the promoters *pB2₉* and *p10*. Promoter *pB2₉* is a DNA fragment derived from the promoter *pB2* which drives the expression of the Basic juvenile hormone-suppressible protein 2 (BJHSP-2) in *T. ni* lepidopter. Fragment *pB2₉,* comprising a sequence consisting of 436 nucleotides derived from the *pB2* promoter, showing higher expression levels than the full-length insect-derived promoter when incorporated into a baculovirus expression vector. A schematic representation of the resulting baculovirus expression cassette of the present invention and the putative function of the recombinant DNA elements is shown in **Figure 1****.** The resulting plasmid was used to generate a recombinant baculovirus by the "Bac-to-Bac^{®}" system (invitrogen™). In parallel, a conventional baculovirus expressing the GFP protein under the control of *polh* promoter was generated by the same system.

The expression of GFP protein mediated by the different baculoviruses was studied by fluorimetry at different times post-infection in *Sf*21 cells cultured in monolayer. Compared to a conventional baculovirus expressing the protein under the control of the *polh* promoter, the expression level of GFP was about 2 times higher with the baculovirus expression cassette containing the transcriptional regulators and enhancer sequence when used at a MOI of 5 **(****Figure 4A)** and 4 times higher when used at a MOI of 0.1 (data not shown). These differences in protein accumulation were also observed in Hi-5™ cells (data not shown), suggesting that the baculovirus expression cassette of the invention could be used to produce recombinant proteins in different insect cell lines used in research and industry.

Importantly, in infected cells observed by fluorescence microscopy, the GFP expression mediated by the baculovirus expression cassette of the present invention was detected earlier than when the conventional promoter *polh* was used for driving GFP expression and, moreover, the fluorescence intensity of infected cells was significantly higher **(****Figure 4B****).** Fluorescent cells were detected as early as 16 hours post-infection when infected at a MOI of 5 with the recombinant baculovirus containing the expression cassette of the invention, and said GFP expression was increasing along the time of infection **(****Figure 4B****).** These marked differences among novel and control recombinant baculoviruses were also observed at a low MOI of 0.1 **(****Figure 4C****).**

To analyze the influence of the promoter used for the overexpression of the transcriptional regulators, the *Ac-ie-01* cDNA was also cloned in the expression cassette described above under the control of *pB2₉* promoter in substitution of the *polh* promoter. Independently of the promoter used to drive the *Ac-ie-01* cDNA expression, the GFP accumulation was higher than the one observed when using a conventional baculovirus in which the reporter protein was expressed under the control of the *polh* promoter without *Ac-ie-01* and *hr1* elements in the expression cassette **(****Figure 4A****).** In a similar way, the absence of *Ac-ie-01* cDNA in an expression cassette containing the *hr1* enhancer linked to *pB2₉p10* chimeric promoter also resulted in lower expression levels of the GFP with respect to that containing the *Ac-ie-01* cDNA (data not shown).

### Example 2. Transcriptional regulators encoded by Ac-ie-01 cDNA potentiate other hr1 cis-linked baculovirus promoters.

To analyze if transcriptional regulators encoded by *Ac-ie-01* in combination with an enhancer sequence increase the expression mediated by other baculovirus promoters, chimeric or not *(p6.9p10* or *p10), cis*-linked to the transcription enhancer homologous region 1 *(hr1),* we generated a new set of baculovirus expression cassettes and their corresponding AcMNPV recombinant baculoviruses. These expression cassettes contained the *Ac-ie-01* cDNA cloned under the control of the *polh* promoter and the GFP encoding gene downstream of the *hr1p10* or *hr1p6.9p10* promoters. The expression levels were measured by fluorimetric analysis of extracts from insect cells infected with the different baculoviruses. As a control, the conventional recombinant baculovirus expressing the GFP protein under the control of *polh* promoter was used. The results observed demonstrated that transcriptional regulators encoded by *Ac-ie-01* cDNA in combination with a homologous sequence *hr1* were also able to promote unprecedented expression levels of the reporter protein GFP expressed under the control of other baculovirus promoters or combination of promoters (chimeric) **(****Figure 4A****).** In the case of the expression cassette using the chimeric promoter composed of *p6*.9 and *p10,* the recombinant protein production was the highest among different recombinant baculoviruses assayed (about 2.5 times higher compared to the control at 72 hours post-infection).

A further quantification of the GFP production in both *Sf*21 insect cells in monolayer and *Sf*9 insect cells in suspension mediated by a conventional baculovirus or by a baculovirus incorporating the expression cassette of the present invention containing the elements *polhAc-ie-01*/*hr1pB2₉p10GFP,* was carried out by microfluidic protein analysis (Experion™; BioRad™, USA). Insect cells in monolayer were infected at a high MOI of 5 and in suspension at a low MOI of 0.1. **Figure 5A** shows the percentages of recombinant GFP produced with respect to the total soluble cell proteins at different times post-infection (24 to 72 hours) and the relative increases of productivity with respect to the conventional baculovirus. At the latest time analyzed, the baculovirus containing the expression cassette of the invention reached levels of the recombinant GFP of more than 40 % of the total cellular protein. Significant differences in the GFP band intensities between the assayed baculoviruses were visible in a Coomassie blue stained SDS-PAGE gel in which cellular extracts from infected cells were resolved **(****Figure 5C****).** When insect cells were cultured in suspension and infected with each baculovirus, differences in recombinant protein productivity were even higher than those detected in insect cells cultured in monolayer and infected at a high MOI of 5. **Figure 5B** shows such differences found in GFP production between 24 to 120 hours post-infection. Interestingly, when the baculovirus incorporating the expression cassette of the invention was used, recombinant protein productivity increased along time, reaching maximum levels at 120 hours post-infection. In contrast, the recombinant protein produced by a conventional baculovirus reached maximum levels at 72 hours post-infection decreasing at later times post-infection **(****Figure 5B****).** An increase of productivity of more than 20 times was observed with the baculovirus expression cassette of the present invention at very late times post-infection. Similar results were observed in Hi-5™ cells (data not shown), demonstrating that the expression cassette of the invention can be employed to produce recombinant proteins in different insect cell lines used in research and industry. Significant differences in the GFP band intensities between the assayed baculoviruses from cells cultured in suspension were also visible in a Coomassie blue stained SDS-PAGE gel in which cellular extracts from infected cells were resolved **(****Figure 5D****).**

### Example 3. The baculovirus expression cassettes of the invention induce cell proliferation and increase cell viability through the transcriptional regulators encoded by the Ac-ie-01 cDNA

We observed by microscopy that recombinant baculoviruses incorporating the baculovirus expression cassettes of the invention have interesting properties related to a decrease in the virus-induced cytopathic effects and an increase of the cell density in cultures. To quantify these phenomena and to determine the DNA element/s responsible for such interesting properties, we generated a recombinant baculovirus expressing the transcriptional regulators encoded by the *Ac-ie-01* cDNA under the control of *polh* promoter. This baculovirus jointly with a baculovirus incorporating the elements of expression cassette of the present invention used in example 2 *(polhAc-ie-01*/*hr1p6.9p10GFP)* and a conventional baculovirus expressing the GFP protein, were used to infect *Sf*9 cells in suspension at a low MOI of 0.1. The increase in cell number and cell viability was studied between 24 to 120 hours post-infection. At 24 hours post-infection, insect cells infected by any of the baculoviruses overexpressing the above mentioned transcriptional regulators presented an increase in cell number higher than 10 % with respect to cultures infected by the control recombinant baculovirus **(****Figure 6A****).** A more detailed analysis by flow cytometry of the time required for these factors to induce the observed differences in cell proliferation revealed an increase of insect cells in S phase at 3 hours post-infection and then at 6 hours post-infection, an increase in the number of insect cells in G1 was observed. These data imply a very early increment of the mitosis in those cultures infected by the baculovirus overexpressing the *Ac-ie-01* cDNA encoding proteins (data not shown).

Fluorescence measurement was performed on a FACSCalibur™ (BD Biosciences™) flow cytometer. Cells were fixed in 70% EtOH, resuspended and incubated in the staining solution (50 µg/ml propidium iodide in PBS, 5 ug /ml RNAse). The data were gated to eliminate particles with a distinct size from cells and analyzed by plotting the cell number vs the red fluorescence from propidium iodide. 50,000 cells were counted per assay. Data analysis of the total number of cells per cell cycle phase (G1, S and G2) was made using Modfit software.

Infected cell cultures were also analyzed by Trypan blue staining to determine cell viability at different times post-infection. Interestingly, at very late times post-infection (96-120 hours), insect cells infected by the viruses overexpressing the transcriptional regulators showed an increase (50-60 % increase) of cell viability and integrity **(****Figure 6B****).** This suggests that the overexpression of the transcriptional regulators of the present invention protects the cells from the baculovirus-induced cytopathic effect, allowing long-term expression. Both cell proliferation and increased cell viability after infection have important consequences in the recombinant protein productivity of the BEVS. Similar results were obtained when the overexpression of the transcriptional regulators was driven by both the *pB2₉* or *polh* promoters (data not shown). Results observed in *Sf*9 insect cells infected in suspension were confirmed in *Sf*21 cells cultured in monolayer (data not shown) and also in Hi-5™ cells cultured in monolayer **(Figure 6C and D).** These figures demonstrate how the overexpression of the transcriptional regulators improves the cell integrity at late times post-infection (96 hours).

### Example 4. Overexpression in a baculovirus expression system of transcriptional regulators encoded by the Ac-ie-01 cDNA facilitates the post-translational processing of recombinant proteins.

Cellular integrity during baculovirus infection is of great importance to ascertain the correct folding or any other post-translational modification of foreign proteins expressed by this system. The baculovirus strong promoters commonly used for research and production, such as *polh* and *p10,* only express the foreign genes at late times post-infection when infected cells already show severe cytopathic effects and the cellular viability decreases. As described above, the overexpression of the transcriptional regulators used in the baculovirus expression cassette of the present invention protects cells from pathogenic effects of the baculovirus infection by a still unknown mechanism, allowing a wide temporal window for recombinant protein production in cells remaining fully viable. We studied the relevance of the elements incorporated into the expression cassette of the invention in relation to post-translational modifications of recombinant proteins. For this purpose, a conventional baculovirus expressing the reporter protein GFP under the control of the *polh* promoter and a baculovirus incorporating the baculovirus cassette of the present invention and also expressing the GFP protein *(polhAc-ie-01*/*hr1p6.9p10GFP)* were used to infect *Sf*9 insect cells in suspension at a MOI of 0.1. Infected cells were analysed at different times after infection by Western blot using an anti-GFP monoclonal antibody (mab2515; Millipore™) as shown in **Figure 7**. Interestingly, GFP protein expressed by a conventional baculovirus showed several reactive bands at 48 and 72 hours post-infection (suggesting non-proper expression and/or folding of the protein) and at later times a band with a reduced molecular weight (lower than predicted) was observed (suggesting degradation) **(****Figure 7A****).** In contrast, when the GFP protein expression was mediated by the baculovirus expression cassette of the invention, only one GFP band was observed at all times post-infection analyzed, showing the expected molecular weight of this protein **(****Figure 7A****).** The expression of GFP by this vector was not significantly reduced at very late times post-infection (120 hours), confirming that the baculovirus expression cassette of the invention confers to baculovirus vectors interesting advantages for long-lasting expression.

In parallel, the integrity of the cell machinery was measured at different times post-infection by Western blot analysis of the cellular actin protein using a specific antiserum **(****Figure 7A****).** Infection with a conventional baculovirus impaired severely the cell integrity at 72 hours post-infection since the actin band detected decreased dramatically after this time point (degradation as a result of a complete loss of cell integrity). Consistent with the cellular protection induced by the recombinant DNA elements contained in the baculovirus expression cassette of the invention, cellular actin was not equally affected in cells infected by the recombinant baculovirus engineered with the expression cassette.

Fluorescence activity of recombinant GFP expressed by the different baculoviruses reflects its correct conformation. As is shown in **Figure 7B****,** the GFP expressed in the context of the baculovirus expression cassettes of the invention keep an increased pattern of functionality along infection times. In contrast, the fluorescence of GFP expressed by a conventional baculovirus peaked at 72 hours post-infection and decreased at later times **(****Figure 7B****),** in parallel to actin degradation **(****Figure 7A****)** and the observed cell viability reduction **(****Figure 6B****).**

### Example 5. Cell culture and viruses.

The *Spodoptera frugiperda Sf*21 or *Sf*9 cell lines were cultured in 6-well tissue culture plates (1x10⁶ cells/well) in TNM-FH insect medium (Pan Biotech™, Germany) containing 10 % heat-inactivated fetal bovine serum (Pan Biotech™, Germany) at 27°C. *Ac*MNPV recombinant baculoviruses were obtained by the "Bac-to-Bac^{®}" Baculovirus Expression System (invitrogen™, USA). Different transfer vectors containing the recombinant DNA elements of the present invention were generated using the pFastBac™-DUAL plasmid (invitrogen™). The promoters and regulatory elements incorporated into pFastBac™-DUAL were synthesized (GenScript™, USA) with the adequate flanking restriction sequences to facilitate the cloning. These transfer vectors were used to transfect *Sf*21 cells with Cellfectin^{®} (invitrogen™, USA). The resulting recombinant baculoviruses from the infection of *Sf*21 cells were then passaged twice in cells and titered by the plaque assay method. The obtained gene constructs of the baculovirus expression cassettes of the present invention are schematically shown in **Figure 8****,** showing different potential combinations of promoters driving the expression of the *Ac-ie-01* cDNA or the foreign gene (e.g. GFP). The different expression cassettes were used to generate the recombinant baculoviruses used in the examples shown in **Figures 4 to 7****.**

### Example 6. Generation of the cloning vector

The cloning vector is a small piece of DNA containing the baculovirus expression cassette of the invention into which a foreign DNA fragment can be inserted by treating the vehicle and the foreign DNA with a restriction enzyme that creates the same overhang, then ligating the fragments together. The essential characteristics of the cloning vector must include a synthetic multiple cloning site (MCS) to facilitate the insertion of foreign genes directed in a chosen orientation, a selectable marker, such as an antibiotic resistance to allow the selection of positively transformed cells and a functional origin of replication (ORI) for propagation in bacteria.

### Example 7. Generation of the donor vector containing the baculovirus expression cassette of the disclosure

A donor vector consists of a cloning vector, for example a pUC57 plasmid, containing the baculovirus expression cassette, into which a foreign gene has been cloned using the appropriate restriction enzymes. The baculovirus expression cassette of the invention was synthesized by ligating the following DNA sequences: (i) the baculovirus transcriptional regulator encoding sequence *Ac-ie-01* downstream of a promoter sequence, such as the *polh* or the *pB2₉* promoter, and upstream of the HSV TK polyadenylation signal and (ii) in another locus an enhancer sequence, for example, the homologous region *hr1,* upstream of (iii) a promoter sequence, for example, *pB2₉p10, p10, p6.9p10* or *polh,* followed by a multiple cloning site (MCS) for cloning the gene of interest and the SV40 polyadenylation signal downstream of the MCS **(****Figure 1****).** The baculovirus expression cassette is flanked by specific restriction sites (for example *Bgl*II and *BstZ17*I at the 5'-terminal end and *Bgl*II and *Sgf*I at the the 3'-terminal end) to facilitate subcloning into a transfer vector of a commercial baculovirus generation system (based on transposition, for example the "Bac-to-Bac^{®}" system (invitrogen™), or based on homologous recombination, for example "flashBAC™" (Oxford Expression Technologies™), "Baculogold™" (BD Biosciences™), "BacPAK6™" (Clontech™), "Bac-N-Blue DNA™" (invitrogen™) **(****Figure 2** **and** **3****).**

The encoding gene of the Green Fluorescence Protein (GFP) was cloned into the MCS of the cloning vector using the *Nco*I and *Spe*I restriction sites, generating the donor plasmid vector **(****Figure 2****).**

### Example 8. Generation of the transfer vector containing the baculovirus expression cassette of the disclosure

The transfer vector was generated by digesting a donor vector with *BstZ17*I of the 5'-flanking site and with *Xba*I and cloning it into the transfer vector pFastBac™1 that was also digested with the same enzymes. In this case, as a result of the subcloning, the SV40 polyadenylation signal of the baculovirus expression cassette is exchanged by the SV40 polyadenlation signal from the transfer vector. Apart from this, all the elements of the expression cassette are included in the pFastBac transfer vector, substituting the *polh* promoter and MCS of the original commercial transfer vector **(****Figure 2****).**

### Example 9. Generation of the baculovirus expression vector containing the baculovirus expression cassette of the disclosure using the "Bac-to-Bac®" system

The modified transfer vector pFastBac™1 and the individual baculovirus expression cassette were used to generate recombinant baculoviruses by using the "Bac-to-Bac^{®}" Baculovirus Expression System. More specifically, the modified transfer vector was used to transform the *E. coli* host strain DH10Bac™ that contains a baculovirus shuttle vector (bacmid) and a helper plasmid, and allows the generation of the recombinant bacmid following transposition of the expression cassette. The DNA of the recombinant bacmid containing the baculovirus expression cassette of the present invention and the GFP encoding gene was then used to transfect insect cells, for example, *Sf*21 cells, using Cellfectin^{®}. 72 hours post-transfection, cells were harvested and the first recombinant baculovirus generation was obtained **(****Figure 2****).** This recombinant baculovirus could then be further amplified and/or titered following conventional protocols. Similar procedures can be used to generate recombinant baculoviruses with other transfer vectors provided by commercial BEVSs **(****Figure 3****).**

### Example 10. Protein sample preparation.

Infected cells from each time point (1x10⁶) were harvested and centrifuged. The supernatants were removed and the cell pellets were resuspended in PBS and subjected to three cycles of freezing (-196 °C) and thawing (37 °C). Cellular debris was removed by centrifugation.

### Example 11. Time-Course Study of Protein Expression.

*Sf*9, *Sf*21 or Hi-5™ cells were infected with the different recombinant baculoviruses expressing GFP under the control of different combinations of regulatory, enhancer and promoter elements, using a MOI of 5 or 0.1. Cell cultures were harvested at various time points (24, 48, 72, 96 and 120 hours post-infection) and the GFP expression was analyzed by fluorescence microscopy, fluorimetric assay, SDS-PAGE followed by Coomassie blue staining or Western blot and by the microfluidic separation and quantification (Experion™ automated electrophoresis system; Bio-Rad™, USA).

For quantification of the recombinant GFP, samples were loaded in Pro260 chips (Bio-Rad™) and analyzed by capillary electrophoresis using the Experion™ system (Bio-Rad™), following the manufacturer's instructions. The electrophoresis of the samples was made through microchannels by controlling the applied voltage and electric power. The microfluidic chip allowed several sequential procedures including separation, staining, destaining, detection and basic data analysis without any need of user's intervention. The Experion™ system resolved and quantified protein samples from 10 to 260 kDa in size, with a high sensitivity, comparable to colloidal Coomassie blue SDS-PAGE gel staining. For quantification, the Experion™ system uses a Pro260 ladder, a modified version of the Precision Plus Protein™ standards, that have been optimized for use in that system.

### Example 12. Fluorescence microscopy analysis.

Infected cells were visualized directly in 6-well cell culture plates using a GFP filter on a Leica™ DMIL™ inverted fluorescence microscope.

### Example 13. Fluorimetric analysis.

About 20 µg of total soluble proteins derived from infected cells, containing different amounts of recombinant GFP protein, were analyzed and quantified by a Tecan™ GENios™ (CA, USA) fluorescence plate reader (excitation [Ex], 485/emission [Em], 535).

### Example 14. Western blot analysis.

Total soluble protein fractions (10 µg) from cells infected with the recombinant baculoviruses were resolved in 15% SDS-PAGE gels. Gels were stained by the Coomassie blue staining method or transferred to nitrocellulose membranes. Western blots were probed with the anti-GFP monoclonal antibody mab2515 (Millipore™, USA) at 1:1000 or actin antiserum (20-33; Sigma-Aldrich™) and the immunocomplexes were revealed with anti-mouse IgG-horseradish peroxidase (HRP)-labeled conjugate (KPL™, UK), diluted 1:2,000 or by an anti-rabbit IgG-horseradish peroxidase (HRP)-labeled conjugate (KPLTM, UK), diluted 1:2,000 respectively as a secondary antibody. Protein bands were detected using an ECL western blotting detection system and analyzed by the ChemiDoc™ XRS Gel Imaging System (Bio-Rad™, USA).

### Example 15. Delimitation of the promoter sequence in the pB2 DNA fragment.

The DNA region upstream of the *BJHSP-2* gene *(pB2)* was PCR amplified from *T. ni* insect DNA based on the previusly reported *BJHSP2* sequence (GenBank accession no U41640). The amplified DNA region differed in several aspects from the annotated sequence, comprising 2 insertions, 8 deletions as well as 17 mutations (SEQ ID NO: 34). Using different bioinformatic analyses, six putative binding sites related to hormone-response elements were found along the *pB2* sequence, four of them corresponding to putative ecdysone-response elements (EcR) and three of them to putative Broad-Complex sites (Br-C) **(Figure 9A and B).**

In order to determine essential regulatory regions for transcriptional activity and the relevance of potential hormone-regulated elements, a *pB2* truncated sequence was analyzed (fragment *pB2₉*) **(****Figure 9B****).** Both *pB2* and *pB2₉* fragments were cloned in a baculovirus vector and tested for their promoter activity by using the GFP reporter protein. **Figure 9B** shows the GFP expression levels obtained with every fragment and quantified by a fluorimetric analysis. Surprisingly, the *pB2₉* fragment showed a stronger promoter activity than the parental full-length *pB2,* even though it lacked two putative Br-C binding sites **(****Figure 9B****).**

### Example 16. Synergistic cooperation between pB2 or pB2₉ and conventional baculovirus promoters.

Different chimeric promoters comprising the *pB2* or *pB2₉* sequence and the conventional baculovirus promoters *polh* or *p10* were constructed, resulting in the combinations *pB2polh, polhpB2, pB2p10, p10pB2* and *pB2₉p10.* All of them were used to generate recombinant baculoviruses and tested for their promoter characteristics. At 24 hours post-infection, the GFP protein expression driven by *polhpB2* and *pB2₉p10* hybrid promoters was higher than by using the conventional promoters *polh* or *p10* **(****Figure 10A****).** Interestingly, the GFP expression under the control of the chimeric promoter *polhpB2* dropped at 48 hours post-infection, while the GFP expressed under the control of the chimeric promoter *pB2₉p10* increased along time at levels even higher than obtained by using the *polh* promoter **(****Figure 10B****).** The chimeric promoter *pB2p10* showed the maximum GFP expression levels at 48 hours post-infection, but the expression increase was not linear at later times **(****Figure 10B****).** In conclusion, the hybrid promoter *pB2₉p10* was earlier and stronger than the *polh* or *p10* promoter alone.

### BIBLIOGRAPHY

1. Nettleship, J.E., Assenberg, R., Diprose, J.M., Rahman-Huq, N., Owens, R.J. Recent advances in the production of proteins in insect and mammalian cells for structural biology. J. Struct. Biol. 2010, 172, 55-65.
2. Gomez-Casado E, Gomez-Sebastian S, Núfiez MC, Lasa-Covarrubias R, Martínez-Pulgarín S, Escribano JM. Insect larvae biofactories as a platform for influenza vaccine production. Protein Expr Purif. 79: 35-43. 2011.
3. Smith, G.E., M.D. Summers, and M.J. Fraser. 1983. Production of human beta interferon in insect cells infected with a baculovirus expression vector. Mol. Cell. Biol. 3: 2156-21 65.
4. Hitchman RB, Possee RD, King LA. Baculovirus expression systems for recombinant protein production in insect cells. Recent Pat Biotechnol. 2009;3(1):46-54.
5. Hashimoto Y, Zhang S, Blissard GW. Ao38, a new cell line from eggs of the black witch moth, Ascalapha odorata (Lepidoptera: Noctuidae), is permissive for AcMNPV infection and produces high levels of recombinant proteins. BMC Biotechnol. 2010,10:50.
6. Taticek RA, Choi C, Phan SE, Palomares LA, Shuler ML. Comparison of growth and recombinant protein expression in two different insect cell lines in attached and suspension culture. Biotechnol. Prog. 2001, 17 (4), 676-684.
7. Hill-Perkins MS, Possee RD. A baculovirus expression vector derived from the basic protein promoter of Autographa californica nuclear polyhedrosis virus. J Gen Virol. 1990, 71 ( Pt 4):971-6.
8. Passarelli, A. L., and L. K. Miller. Three baculovirus genes involved in late and very late gene expression: ie-1, ie-n, and lef-2. J. Virol. 1993, 67:2149-2158
9. Rodems, S. M., S. S. Pullen, and P. D. Friesen. DNA-dependent transregulation by IE1 of Autographa californica nuclear polyhedrosis virus: IE1 domains required for transactivation and DNA binding. J. Virol. 1997, 71: 9270-9277.
10. Lin X, Chen Y, Yi Y, Zhang Z: Baculovirus immediately early 1, a mediator for homologous regions enhancer function in trans. Virol J 2010, 7:32.
11. Okano K, Mikhailov VS, Maeda S: Colocalization of baculovirus IE-1 and two DNA-binding proteins, DBP and LEF-3, to viral replication factories. Journal of virology 1999, 73(1):110-119.

## Claims

1. Recombinant baculovirus containing an endogenous *Ac-ie-01* gene, comprising
[i] a further copy of baculoviral *Ac-ie-01* cDNA as transgene under the control of a suitable promoter that allows for the expression above endogenous levels of the proteins IE-1, IE-0 and/or fragments thereof functioning as transcriptional regulators, wherein the nucleic acid is selected from the group consisting of:
a) a nucleic acid containing the nucleotide sequence indicated in any of SEQ ID NO: 1-5;
b) a nucleic acid sequence having a sequence identity of at least 70%, preferably at least 75%, more preferably at least 80%, more preferably at least 85%, more preferably at least 90% and most preferably at least 95% with the nucleotide sequence indicated in any of SEQ ID NO: 1-5 and encoding a protein able to function as a transcriptional regulator in a recombinant baculovirus;
c) a nucleic acid sequence encoding an amino acid containing the amino acid sequence indicated in any of SEQ ID NO: 6-9; and
d) a nucleic acid sequence encoding an amino acid sequence having a sequence similarity of at least 70%, preferably at least 75%, more preferably at least 80%, more preferably at least 85%, more preferably at least 90% and most preferably at least 95% with the amino acid sequence indicated in any of SEQ ID NO: 6-9 and able to function as a transcriptional regulator in a recombinant baculovirus;
[ii] wherein the recombinant baculovirus further comprises at least one recombinant homologous region (*hr*) as enhancer region, operably linked to any promoter that is suitable for driving the expression of a recombinant protein,
[iii] wherein the promoter operably linked to the homologous region (*hr*) is selected from the group of nucleic acids comprising:
a) a nucleic acid containing the nucleotide sequence indicated in any of SEQ ID NO: 10-16; and
b) a nucleic acid sequence able to function as a promoter in a recombinant baculovirus and having a sequence identity of at least 70%, preferably at least 75%, more preferably at least 80%, more preferably at least 85%, more preferably at least 90% and most preferably at least 95% with the nucleotide sequence indicated in any of SEQ ID NO: 10-16.

2. Recombinant baculovirus according, to claim 1, wherein the recombinant homologous region (*hr*) is the sequence indicated in SEQ ID NO: 27 (*hr1*).

3. Recombinant baculovirus according to any of the claims 1-2, comprising a nucleic acid sequence that comprises combinations of recombinant promoters, sequences encoding transcriptional regulators and enhancer regions selected from the group comprising SEQ ID NO: 17-26.

4. Recombinant baculovirus according to any of the claims 1-3, further comprising a nucleic acid sequence encoding a recombinant protein, wherein said nucleic acid sequence is operably linked to a nucleic acid sequence selected from the group consisting of the nucleic acid sequence of claim 1-3.

5. Transfer vector suitable for producing a recombinant baculovirus according to any of the claims 1-4, comprising said further copy of baculoviral Ac.ie.01 cDNA under the control of a suitable promoter for expression above endogenous levels of the proteins IE-0, IE-1 and/or fragments thereof functioning as transcriptional regulators, further comprising a nucleic acid sequence suitable for integration or transposition in a baculovirus.

6. Cloning vector suitable for producing a recombinant baculovirus or transfer vector according to any of claims 1-4, comprising said further copy of baculoviral Ac.ie.01 cDNA under the control of a suitable promoter for expression above endogenous levels of the proteins IE-0, IE-1 and/or fragments thereof functioning as transcriptional regulators, which is further suitable for bacterial replication.

7. Nucleic acid sequence suitable for producing a recombinant baculovirus, transfer vector or cloning vector according to any of the claims 1-6, comprising said further copy of baculoviral Ac.ie.01 cDNA under the control of a suitable promoter for expression above endogenous levels of the proteins IE-0, IE-1 and/or fragments thereof functioning as transcriptional regulators.

8. A host cell infected, transfected, transduced or transformed with the recombinant baculovirus, transfer vector, cloning vector or nucleic acid sequence of any of the claims 1-7.

9. Infected, transfected, transduced or transformed host cell according to claim 8, **characterized in that** it is an insect cell line.

10. Infected, transfected, transduced or transformed host cell according to claim 8 or 9, **characterized in that** it is derived from an insect belonging to the Lepidoptera or Diptera genus.

11. Infected, transfected, transduced or transformed host cell according to any of the claims 8-10, **characterized in that** it is derived from *Trichoplusia ni, Spodoptera frugiperda, Ascalapha odorata, Bombyx mori, Drosophila melanogaster* or *Aedes aegypti.*

12. Infected, transfected, transduced or transformed host cell according to any of the claims 8-11, **characterized in that** it is a cell line selected from the group consisting of Hi-5™, *Sf9, Sf*21, BTI-Tn5B-1, Tn368, ExpresSf+®, BTI-TnAo38, ATC-10 and Schneider's *Drosophila* Line 2.

13. Method for producing a recombinant protein that comprises the infected, transfected, transduced or transformed host cell according to claim 8-12 and the extraction and purification of the recombinant protein by conventional means.

14. Use of the transfer vector according to claim 5 for producing a recombinant baculovirus according to any of claims 1-4.

15. Use of the cloning vector according to claim 6 for producing a recombinant baculovirus or transfer vector according to any of claims 1-5.

16. Use of the nucleic acid sequence according to claim 7 for producing a recombinant baculovirus, transfer vector or cloning vector according to any of the claims 1-6.

## Patentansprüche

1. Rekombinanter Baculovirus, welcher ein endogenes *Ac-ie-01*-Gen enthält, umfassend
[i] eine weitere Kopie baculoviraler Ac-ie-01-cDNA als Transgen unter der Kontrolle eines geeigneten Promotors, welcher die Expression der Proteine IE-1, IE-0 und/oder Fragmenten davon, die als Transkriptionsregulatoren funktionieren, oberhalb endogener Niveaus erlaubt, wobei die Nukleinsäure aus der Gruppe ausgewählt ist, bestehend aus:
a) einer Nukleinsäure, welche die in irgendeiner der SEQ ID NO: 1-5 angegebene Nukleotidsequenz enthält;
b) einer Nukleinsäuresequenz, die eine Sequenzidentität von wenigstens 70%, bevorzugt wenigstens 75%, bevorzugter wenigstens 80%, bevorzugter wenigstens 85%, bevorzugter wenigstens 90% und am meisten bevorzugt wenigstens 95% mit der in irgendeiner der SEQ ID NO: 1-5 angegebenen Nukleotidsequenz hat und ein Protein codiert, welches dazu fähig ist, in einem rekombinanten Baculovirus als ein Transkriptionsregulator zu funktionieren;
c) einer Nukleinsäuresequenz, die eine Aminosäure codiert, welche die in irgendeiner der SEQ ID NO: 6-9 angegebene Aminosäuresequenz enthält; und
d) einer Nukleinsäuresequenz, die eine Aminosäuresequenz codiert, welche eine Sequenzähnlichkeit von wenigstens 70%, bevorzugt wenigstens 75%, bevorzugter wenigstens 80%, bevorzugter wenigstens 85%, bevorzugter wenigstens 90% und am meisten bevorzugt wenigstens 95% mit der in irgendeiner der SEQ ID NO: 6-9 angegebenen Aminosäuresequenz hat und dazu fähig ist, in einem rekombinanten Baculovirus als ein Transkriptionsregulator zu funktionieren;
[ii] wobei der rekombinante Baculovirus weiterhin wenigstens eine rekombinante homologe Region (hr) als Enhancer-Region umfasst, die funktionsfähig mit irgendeinem Promotor verknüpft ist, welcher geeignet ist, um die Expression eines rekombinanten Proteins zu steuern,
[iii] wobei der funktionsfähig mit der homologen Region (hr) verknüpfte Promotor aus der Gruppe von Nukleinsäuren ausgewählt ist, welche umfasst:
a) eine Nukleinsäure, welche die in irgendeiner der SEQ ID NO: 10-16 angegebene Nukleotidsequenz enthält; und
b) eine Nukleinsäuresequenz, die dazu fähig ist, in einem rekombinanten Baculovirus als ein Promotor zu funktionieren, und eine Sequenzidentität von wenigstens 70%, bevorzugt wenigstens 75%, bevorzugter wenigstens 80%, bevorzugter wenigstens 85%, bevorzugter wenigstens 90% und am meisten bevorzugt wenigstens 95% mit der in irgendeiner der SEQ ID NO: 10-16 angegebenen Nukleotidsequenz hat.

2. Rekombinanter Baculovirus gemäß Anspruch 1, wobei die rekombinante homologe Region (hr) die in SEQ ID NO: 27 angegebene Sequenz ist (*hr1*).

3. Rekombinanter Baculovirus gemäß irgendeinem der Ansprüche 1-2, welcher eine Nukleinsäuresequenz umfasst, die Kombinationen von rekombinanten Promotoren, Transkriptionsregulatoren codierenden Sequenzen und Enhancer-Regionen umfasst, die aus der Gruppe ausgewählt sind, bestehend aus SEQ ID NO: 17-26.

4. Rekombinanter Baculovirus gemäß irgendeinem der Ansprüche 1-3, welcher weiterhin eine Nukleinsäuresequenz umfasst, die ein rekombinantes Protein codiert, wobei die Nukleinsäuresequenz funktionsfähig mit einer Nukleinsäuresequenz verknüpft ist, die aus der Gruppe ausgewählt ist, bestehend aus der Nukleinsäuresequenz gemäß Anspruch 1-3.

5. Transfervektor, der geeignet ist, um einen rekombinanten Baculovirus gemäß irgendeinem der Ansprüche 1-4 zu produzieren, der die weitere Kopie baculoviraler *Ac-ie-01*-cDNA unter der Kontrolle eines geeigneten Promotors für die Expression der Proteine IE-0, IE-1 und/oder Fragmenten davon, die als Transkriptionsregulatoren funktionieren, oberhalb endogener Niveaus umfasst, und weiterhin eine Nukleinsäuresequenz umfasst, die für die Integration oder Transposition in einen Baculovirus geeignet ist.

6. Klonierungsvektor, der geeignet ist, um einen rekombinanten Baculovirus oder einen Transfervektor gemäß irgendeinem der Ansprüche 1-4 zu produzieren, der die weitere Kopie baculoviraler *Ac-ie-01-*cDNA unter der Kontrolle eines geeigneten Promotors für die Expression der Proteine IE-0, IE-1 und/oder Fragmenten davon, die als Transkriptionsregulatoren funktionieren, oberhalb endogener Niveaus umfasst, und weiterhin für die bakterielle Replikation geeignet ist.

7. Nukleinsäuresequenz, die geeignet ist, um einen rekombinanten Baculovirus, einen Transfervektor oder einen Klonierungsvektor gemäß irgendeinem der Ansprüche 1-6 zu produzieren, die die weitere Kopie baculoviraler *Ac-ie-01*-cDNA unter der Kontrolle eines geeigneten Promotors für die Expression der Proteine IE-0, IE-1 und/oder Fragmenten davon, die als Transkriptionsregulatoren funktionieren, oberhalb endogener Niveaus umfasst.

8. Wirtszelle, die mit dem rekombinanten Baculovirus, dem Transfervektor, dem Klonierungsvektor oder der Nukleinsäuresequenz gemäß irgendeinem der Ansprüche 1-7 infiziert, transfiziert, transduziert oder transformiert ist.

9. Infizierte, transfizierte, transduzierte oder transformierte Wirtszelle gemäß Anspruch 8, **dadurch gekennzeichnet, dass** sie eine Insektenzelllinie ist.

10. Infizierte, transfizierte, transduzierte oder transformierte Wirtszelle gemäß Anspruch 8 oder 9, **dadurch gekennzeichnet, dass** sie von einem Insekt abgeleitet ist, welches zur Gattung Lepidoptera oder Diptera gehört.

11. Infizierte, transfizierte, transduzierte oder transformierte Wirtszelle gemäß irgendeinem der Ansprüche 8-10, **dadurch gekennzeichnet, dass** sie von *Trichoplusia ni, Spodoptera frugiperda, Ascalapha odorata, Bombyx mori, Drosophila melanogaster* oder *Aedes aegypti* abgeleitet ist.

12. Infizierte, transfizierte, transduzierte oder transformierte Wirtszelle gemäß irgendeinem der Ansprüche 8-11, **dadurch gekennzeichnet, dass** sie eine Zelllinie ist, die aus der Gruppe ausgewählt ist, bestehend aus Hi-5™, *Sf*9, *Sf*21, BTI-Tn5B-1, Tn368, ExpresSf+^{®}, BTI-TnAo38, ATC-10 und *Schneider-Drosophila-*Linie 2.

13. Verfahren zum Produzieren eines rekombinanten Proteins, welches die infizierte, transfizierte, transduzierte oder transformierte Wirtszelle gemäß Anspruch 8-12 umfasst, und die Extraktion und Reinigung des rekombinanten Proteins durch herkömmliche Mittel.

14. Verwendung des Transfervektors gemäß Anspruch 5 zum Produzieren eines rekombinanten Baculovirus gemäß irgendeinem der Ansprüche 1-4.

15. Verwendung des Klonierungsvektors gemäß Anspruch 6 zum Produzieren eines rekombinanten Baculovirus oder eines Transfervektors gemäß irgendeinem der Ansprüche 1-5.

16. Verwendung der Nukleinsäuresequenz gemäß Anspruch 7 zum Produzieren eines rekombinanten Baculovirus, eines Transfervektors oder eines Klonierungsvektors gemäß irgendeinem der Ansprüche 1-6.

## Revendications

1. Baculovirus recombinant contenant un gène *Ac-ie-01* endogène, comprenant :
(i) une autre copie d'ADNc *Ac-ie-01* baculoviral en tant que transgène sous le contrôle d'un promoteur approprié qui permet l'expression supérieure aux taux endogènes des protéines IE-1, IE-0 et/ou de leurs fragments fonctionnant en tant que régulateurs de la transcription, dans lequel l'acide nucléique est choisi dans le groupe constitué des suivants :
a) un acide nucléique contenant la séquence nucléotidique indiquée dans l'une quelconque des SEQ ID NO : 1 à 5 ;
b) une séquence d'acide nucléique présentant une identité de séquence d'au moins 70 %, de préférence d'au moins 75 %, de façon davantage préférée d'au moins 80 %, de façon davantage préférée d'au moins 85 %, de façon davantage préférée d'au moins 90 % et de façon préférée entre toutes d'au moins 95 % avec la séquence nucléotidique indiquée dans l'une quelconque des SEQ ID NO : 1 à 5 et codant pour une protéine capable de fonctionner en tant que régulateur de la transcription dans un baculovirus recombinant ;
c) une séquence d'acide nucléique codant pour un acide aminé contenant la séquence d'acides aminés indiquée dans l'une quelconque des SEQ ID NO : 6 à 9 ; et
d) une séquence d'acide nucléique codant pour une séquence d'acides aminés présentant une similarité de séquence d'au moins 70 %, de préférence d'au moins 75 %, de façon davantage préférée d'au moins 80 %, de façon davantage préférée d'au moins 85 %, de façon davantage préférée d'au moins 90 % et de façon préférée entre toutes d'au moins 95 % avec la séquence d'acides aminés indiquée dans l'une quelconque des SEQ ID NO : 6 à 9 et capable de fonctionner en tant que régulateur de la transcription dans un baculovirus recombinant ;
ii) dans lequel le baculovirus recombinant comprend en outre au moins une région homologue recombinante (hr) en tant que région d'amplificateur, liée de façon fonctionnelle à tout promoteur qui est approprié pour diriger l'expression d'une protéine recombinante,
iii) dans lequel le promoteur lié de façon fonctionnelle à la région homologue (*hr*) est choisi dans le groupe d'acides nucléiques comprenant :
a) un acide nucléique contenant la séquence nucléotidique indiquée dans l'une quelconque des SEQ ID NO : 10 à 16 ; et
b) une séquence d'acide nucléique capable de fonctionner en tant que promoteur dans un baculovirus recombinant et présentant une identité de séquence d'au moins 70 %, de préférence d'au moins 75 %, de façon davantage préférée d'au moins 80 %, de façon davantage préférée d'au moins 85 %, de façon davantage préférée d'au moins 90 % et de façon préférée entre toutes d'au moins 95 % avec la séquence nucléotidique indiquée dans l'une quelconque des SEQ ID NO : 10 à 16.

2. Baculovirus recombinant selon la revendication 1, dans lequel la région homologue recombinante (hr) est la séquence indiquée dans la SEQ ID NO : 27 (*hr1*).

3. Baculovirus recombinant selon l'une quelconque des revendications 1 à 2, comprenant une séquence d'acide nucléique qui comprend des combinaisons de promoteurs recombinants, des séquences codant pour des régulateurs de la transcription et des régions d'amplificateurs choisies dans le groupe comprenant les SEQ ID NO : 17 à 26.

4. Baculovirus recombinant selon l'une quelconque des revendications 1 à 3, comprenant en outre une séquence d'acide nucléique codant pour une protéine recombinante, dans lequel ladite séquence d'acide nucléique est liée de façon fonctionnelle à une séquence d'acide nucléique choisie dans le groupe constitué de la séquence d'acide nucléique selon les revendications 1 à 3.

5. Vecteur de transfert approprié pour la production d'un baculovirus recombinant selon l'une quelconque des revendications 1 à 4, comprenant ladite autre copie d'ADNc *Ac*-*ie*-*01* baculoviral sous le contrôle d'un promoteur approprié pour l'expression supérieure aux taux endogènes des protéines IE-0, IE-1 et/ou de leurs fragments fonctionnant en tant que régulateurs de la transcription, comprenant en outre une séquence d'acide nucléique appropriée pour l'intégration ou la transposition dans un baculovirus.

6. Vecteur de clonage approprié pour la production d'un baculovirus recombinant ou d'un vecteur de transfert selon l'une quelconque des revendications 1 à 4, comprenant ladite autre copie d'ADNc *Ac-ie-01* baculoviral sous le contrôle d'un promoteur approprié pour l'expression supérieure aux taux endogènes des protéines IE-0, IE-1 et/ou de leurs fragments fonctionnant en tant que régulateurs de la transcription, qui est en outre approprié pour la réplication bactérienne.

7. Séquence d'acide nucléique appropriée pour la production d'un baculovirus recombinant, d'un vecteur de transfert ou d'un vecteur de clonage selon l'une quelconque des revendications 1 à 6, comprenant ladite autre copie d'*ADNc Ac-ie-*01 baculoviral sous le contrôle d'un promoteur appropriépour l'expression supérieure aux taux endogènes des protéines IE-0, IE-1 et/ou de leurs fragments fonctionnant en tant que régulateurs de la transcription.

8. Cellule hôte infectée, transfectée, transduite ou transformée avec le baculovirus recombinant, le vecteur de transfert, le vecteur de clonage ou la séquence d'acide nucléique selon l'une quelconque des revendications 1 à 7.

9. Cellule hôte infectée, transfectée, transduite ou transformée selon la revendication 8, **caractérisée en ce qu'**il s'agit d'une lignée cellulaire d'insecte.

10. Cellule hôte infectée, transfectée, transduite ou transformée selon la revendication 8 ou 9, **caractérisée en ce qu'**elle est dérivée d'un insecte appartenant au genre des Lépidoptères ou des Diptères.

11. Cellule hôte infectée, transfectée, transduite ou transformée selon l'une quelconque des revendications 8 à 10, **caractérisée en ce qu'**elle est dérivée de *Trichoplusia ni, Spodoptera frugiperda, Ascalapha odorata, Bombyx mori, Drosophila melanogaster* ou *Aedes aegypti.*

12. Cellule hôte infectée, transfectée, transduite ou transformée selon l'une quelconque des revendications 8 à 11, **caractérisée en ce qu'**il s'agit d'une lignée cellulaire choisie dans le groupe constitué de Hi-5T™, *Sf9, Sf21,* BTI-Tn5B-1, Tn368, ExpresSf+^{®}, BTI-TnAo38, ATC-10 et la lignée 2 de *Drosophila* de Schneider.

13. Procédé pour la production d'une protéine recombinante qui comprend la cellule hôte infectée, transfectée, transduite ou transformée selon les revendications 8 à 12 et pour l'extraction et la purification de la protéine recombinante par des moyens traditionnels.

14. Utilisation du vecteur de transfert selon la revendication 5 pour la production de d'un baculovirus recombinant selon l'une quelconque des revendications 1 à 4.

15. Utilisation du vecteur de clonage selon la revendication 6 pour la production d'un baculovirus recombinant ou d'un vecteur de transfert selon l'une quelconque des revendications 1 à 5.

16. Utilisation de la séquence d'acide nucléique selon la revendication 7 pour la production d'un baculovirus recombinant, d'un vecteur de transfert ou d'un vecteur de clonage selon l'une quelconque des revendications 1 à 6.
